(19) Europäisches Patentamt
European Patent Office
Office européen des brevets

(11) EP 3 536 308 A1

(12) EUROPEAN PATENT APPLICATION
published in accordance with Art. 153(4) EPC

(43) Date of publication:
**11.09.2019 Bulletin 2019/37**

(21) Application number: **17866637.6**

(22) Date of filing: **01.11.2017**

(51) Int Cl.:
***A61K 8/81*** *(2006.01)* ***A61K 8/02*** *(2006.01)*
***A61K 8/19*** *(2006.01)* ***A61Q 1/02*** *(2006.01)*
***A61Q 1/10*** *(2006.01)* ***A61Q 1/12*** *(2006.01)*
***A61Q 17/04*** *(2006.01)* ***A61Q 19/00*** *(2006.01)*

(86) International application number:
**PCT/JP2017/039523**

(87) International publication number:
**WO 2018/084173 (11.05.2018 Gazette 2018/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **07.11.2016 JP 2016216982**

(71) Applicant: **Nisshinbo Holdings Inc.**
**Tokyo 103-8650 (JP)**

(72) Inventors:
- **HAYAKAWA Kazutoshi**
  **Chiba-city**
  **Chiba 267-0056 (JP)**
- **HASHIBA Toshifumi**
  **Chiba-city**
  **Chiba 267-0056 (JP)**
- **TAKAHASHI Mitsugu**
  **Chiba-city**
  **Chiba 267-0056 (JP)**
- **YAMADA Goki**
  **Chiba-city**
  **Chiba 267-0056 (JP)**
- **HIRAOKA Hiroko**
  **Chiba-city**
  **Chiba 267-0056 (JP)**

(74) Representative: **Mewburn Ellis LLP**
**City Tower**
**40 Basinghall Street**
**London EC2V 5DE (GB)**

# (54) SKIN CARE PREPARATIONS

(57) Provided are skin cosmetics including 0.1-50% by mass of polymer particles including flat, oval, spherical polymer particles A in which: all of the front view, the plan view, and the side view of a projection drawing based on the third angle method are ovals; (1) the average ($L_{AV}$) of the long diameter (L) of the flat portion is $0.13 \leq L_{AV} \leq 500$ μm; (2) the average ($D_{AV}$) of the short diameter (D) of the flat portion is $0.1 \leq D_{AV} \leq 250$ μm; and (3) the average ($P^1_{AV}$) of the aspect ratio (L/D) calculated from the long diameter (L) and the short diameter (D) is $1.3 < P^1_{AV} \leq 50$.

EP 3 536 308 A1

## Description

### TECHNICAL FIELD

The present invention relates to skin cosmetics.

### BACKGROUND ART

Micron-size polymer particles and inorganic particles are used as fillers and specimens in a variety of fields, such as electrical and electronic materials, optical materials, paints, inks, construction materials, biological and pharmaceutical materials, and cosmetics. Of these, particles of unusual, non-spherical shapes, given their ability to exhibit diverse characteristics unlike those of spherical particles in terms of optical properties, tactile qualities and the like, have been the subject of active research in recent years, and new applications are constantly being developed.

The inventors have worked to date on the development of ellipsoidal or needle-shaped polymer particles of high aspect ratio, discovering various particles superior to conventional spherical particles in terms of such characteristics as hiding power, light-diffusing ability and tactile qualities, and promoting the use of such particles in cosmetic applications (Patent Documents 1 and 2). Yet, in this technical field, further improvements in properties are desired and the development of novel polymer particles and their applications continues to move ahead.

In general, spherical polymer particles are often used as extenders or texture improvers in cosmetics applications. They are very useful in terms of being able to impart slip characteristics, a lightweight feel and the like. However, in skin cosmetics such as foundation and point makeup, improvements in adhesion and long-lasting performance are also desired, but spherical particles readily separate and fall from the applied surface with the passage of time and the amount of particle addition is also subject to limitation.

### PRIOR ART DOCUMENTS

### PATENT DOCUMENTS

Patent Document 1: JP-A 2009-235353
Patent Document 2: JP-A 2009-235355

### SUMMARY OF INVENTION

### TECHNICAL PROBLEM

It is therefore an object of the present invention to provide skin cosmetics which have a high adherence, optical properties such as hiding power, light-diffusing ability and UV-cutting properties, and also, for example, excellent tactile qualities and flowability.

### SOLUTION TO PROBLEM

The inventors have conducted extensive investigations aimed at achieving the above object, discovering as a result that by making improvements in the art of synthesizing ellipsoidal polymer particles, oblate ellipsoidal polymer particles, i.e., ellipsoidal particles that are flattened on the minor axis sides, can be obtained which, owing to distinctive characteristics that differ from those of conventional polymer particles in terms of, for example, high adherence, light-diffusing ability, UV-cutting ability, tactile qualities and flowability, are useful in skin cosmetics.

Accordingly, the invention provides the following skin cosmetics.

1. A skin cosmetic containing from 0.1 to 50 wt% of polymer particles that include an oblate ellipsoidal polymer particle A which has, in projections based on the third-angle projection method, a front view, a plan view and a side view that are all elliptical, and which satisfies conditions (1) to (3) below pertaining to a flat region of the particle:

(1) the flat region has a length L whose average $L_{AV}$ is such that 0.13 $\mu$m $\leq L_{AV} \leq$ 500 $\mu$m,
(2) the flat region has a breadth D whose average $D_{AV}$ is such that 0.1 $\mu$m $\leq D_{AV} \leq$ 250 $\mu$m, and
(3) the aspect ratio L/D calculated from the length L and breadth D has an average value $P^1_{AV}$ such that $1.3 < P^1_{AV} \leq 50$.

2. The skin cosmetic of 1 above, wherein the oblate ellipsoidal polymer particle A further satisfies at least one of conditions (4) and (5) below:

(4) the aspect ratio D/T calculated from the breadth D and a lateral thickness T of the particle has an average value $P^2_{AV}$ such that $1.2 < P^2_{AV} \leq 100$, and
(5) the aspect ratio L/T calculated from the length L and the lateral thickness T of the particle has an average value $P^3_{AV}$ such that $1.56 < P^3_{AV} \leq 150$.

3. The skin cosmetic of 1 or 2 above which includes two or more types of oblate ellipsoidal polymer particle A.
4. The skin cosmetic of any of 1 to 3 above, wherein the polymer particles further include at least one type of polymer particle B having a shape different from oblate ellipsoidal polymer particle A.
5. The skin cosmetic of 4 above, wherein polymer particle B has a volume mean particle size of $L_{AV}$ or less.
6. The skin cosmetic of 4 or 5 above, wherein polymer particle B is spherical, spheroidal or ellipsoidal.
7. The skin cosmetic of any of 4 to 6 above, wherein the weight ratio of oblate ellipsoidal polymer particle A to polymer particle B is from 99:1 to 10:90.
8. The skin cosmetic of any of 1 to 7 above, further containing inorganic particles.
9. The skin cosmetic of 8 above, wherein the inorganic particles have a shape that is plate-like or flake-like.
10. The skin cosmetic of any of 1 to 9 above, further containing an oil-based ingredient.

## ADVANTAGEOUS EFFECTS OF INVENTION

The skin cosmetics of the invention include an oblate ellipsoidal polymer particle A as an essential component and therefore, from the standpoint of the optical properties, slip characteristics, adherence and other properties attributable to the particle shape, are useful as makeup preparations such as eyeliner, eyebrow, eye shadow, lipstick, cheek, foundation, concealer, face powder, makeup base and point makeup. Specifically, these optical properties make the skin appear lighter and can enhance the covering power due to a shading effect. Also, due to the slip characteristics (flow properties) particular to the oblate ellipsoidal shape of the particles, spreadability over the skin is excellent and furrows in the skin texture are finely filled, making it possible to render wrinkles and pores inconspicuous. Moreover, because the adherence is high, good effects can be obtained with even a small amount, enabling entirely new cosmetic effects to be achieved. In addition, a finished look possible only with polymer components can be realized at the same time. Finally, the strength of molded products such as pressed powder and lipstick can be enhanced, enabling useful cosmetic preparations to be obtained from a quality standpoint as well.

## BRIEF DESCRIPTION OF DRAWINGS

FIG. 1 is a diagram showing the length L, breadth D and thickness T of an oblate ellipsoidal polymer particle A.
FIG. 2 shows a scanning electron micrograph (3,000×) of oblate ellipsoidal polymer particle A1 obtained in Production Example 1.
FIG. 3 shows a scanning electron micrograph (2,000×) of oblate ellipsoidal polymer particle A2 obtained in Production Example 2.
FIG. 4 shows a scanning electron micrograph (1,000×) of oblate ellipsoidal polymer particle A3 obtained in Production Example 3.
FIG. 5 shows a scanning electron micrograph (1,000×) of ellipsoidal polymer particle B2 obtained in Comparative Production Example 2.
FIG. 6 is a diagram showing the light scattering distribution of reflected light measured in Comparative Examples 3-1 and 3-2 and in Comparative Reference Examples 3-1 and 3-2.

## DESCRIPTION OF EMBODIMENTS

[Skin Cosmetic]

The skin cosmetic of the invention contains, as an essential component, polymer particles which include the oblate ellipsoidal polymer particle A described below.

[Oblate Ellipsoidal Polymer Particle A]

The oblate ellipsoidal polymer particle A has, in projections based on the third-angle projection method, a front view, a plan view and a side view that are all elliptical, and satisfies conditions (1) to (3) below pertaining to a flat region of the particle:

(1) the flat region has a length L whose average $L_{AV}$ is such that 0.13 μm ≤ $L_{AV}$ ≤ 500 μm,
(2) the flat region has a breadth D whose average $D_{AV}$ is such that 0.1 μm ≤ $D_{AV}$ ≤ 250 μm, and
(3) the aspect ratio L/D calculated from the length L and breadth D has an average value $P^1_{AV}$ such that 1.3 < $P^1_{AV}$ ≤ 50.

In this invention, the term "oblate ellipsoidal" is not limited only to shapes obtained by flattening a mathematically defined ellipsoid, but encompasses also particles of nearly ellipsoidal shape having a flat region, including nearly oblate ellipsoidal shapes such as flat rectangular shapes with rounded corners and flat oval shapes.

In the oblate ellipsoidal polymer particle A, for makeup with a smooth finish, $L_{AV}$ is preferably such that 1.0 μm ≤ $L_{AV}$ ≤ 300 μm, more preferably such that 5.0 μm ≤ $L_{AV}$ ≤ 150 μm, and even more preferably such that 8.0 μm ≤ $L_{AV}$ ≤ 100 μm. $D_{AV}$ is preferably such that 0.1 μm ≤ $D_{AV}$ ≤ 150 μm, more preferably such that 0.5 μm ≤ $D_{AV}$ ≤ 100 μm, and even more preferably such that 0.8 μm ≤ $D_{AV}$ ≤ 80 μm. $P^1_{AV}$ is preferably such that 1.4 < $P^1_{AV}$ ≤ 40, more preferably such that 1.6 < $P^1_{AV}$ ≤ 35, and even more preferably such that 1.8 < $P^1_{AV}$ ≤ 30.

Given that a larger surface area of contact between the particle and a solid surface increases the adhesive strength of the particle and that a smaller resistance to fluids is less likely to result in the release or shedding of particles, oblate ellipsoidal polymer particle A preferably has a shape that also satisfies at least one of conditions (4) and (5) below:

(4) the aspect ratio D/T calculated from the breadth D and a lateral thickness T of the particle has an average value $P^2_{AV}$ such that 1.2 < $P^2_{AV}$ ≤ 100, and
(5) the aspect ratio L/T calculated from the length L and the lateral thickness T of the particle has an average value $P^3_{AV}$ such that 1.56 < $P^3_{AV}$ ≤ 150.
$P^2_{AV}$ is preferably such that 1.3 ≤ $P^2_{AV}$ ≤ 50, more preferably such that 1.5 ≤ $P^2_{AV}$ ≤ 30, and even more preferably such that 2.0 ≤ $P^2_{AV}$ ≤ 20. $P^3_{AV}$ is preferably such that 1.7 ≤ $P^3_{AV}$ ≤ 100, more preferably such that 2.0 ≤ $P^3_{AV}$ ≤ 50, and even more preferably such that 3.0 ≤ $P^3_{AV}$ ≤ 30.
Oblate ellipsoidal polymer particle A preferably possesses at least one of the following characteristics: fine irregularities at the particle surface, a high porosity, or a relatively large specific surface area. It is preferable in particular for the particle to be porous or to have attached to or included within at least a surface or a surface layer portion thereof fine particles that satisfy condition (6) below. Oblate ellipsoidal polymer particle A more preferably has an uneven surface shape owing to such fine particles.
(6) The fine particles attached to or included within the particle surface or surface layer portion have a particle size SP such that 1/1,000×$D_{AV}$ ≤ SP ≤ 1/2×$D_{AV}$.

SP is more preferably such that 1/100×$D_{AV}$ ≤ SP ≤ 1/2×$D_{AV}$, and even more preferably such that 1/20×$D_{AV}$ ≤ SP ≤ 1/2×$D_{AV}$. These fine particles are preferably composed of the same ingredients as oblate ellipsoidal polymer particle A, and are preferably attached to or included within the surface or surface layer portion of oblate ellipsoidal polymer particle A.

Oblate ellipsoidal polymer particle A has a volume mean particle size $MV_A$ which is preferably from 0.1 to 500 μm, more preferably from 0.5 to 300 μm, and even more preferably from 1.0 to 150 μm. In this invention, "volume mean particle size" is a measured value obtained by the laser scattering diffraction method and refers to, in the case of ellipsoidal, needle-shaped or rod-shaped particles and unusually shaped particles, the mean diameter of spheres having the same volumes as the particles (i.e., mean sphere-equivalent diameter of the particles).

Oblique ellipsoidal polymer particle A has a ratio SB/SD between the actual specific surface area SB of the oblique ellipsoidal polymer particle and the theoretical specific surface area SD of a spherical particle calculated from the volume mean particle size of the oblique ellipsoidal elliptical polymer particle which preferably satisfies the condition SB/SD ≥ 1.2, more preferably satisfies the condition SB/SD ≥ 1.5, even more preferably satisfies the condition SB/SD ≥ 1.8, and most preferably satisfies the condition SB/SD ≥ 2.0. The actual specific surface area SB of oblique ellipsoidal polymer particle A, although not particularly limited, is preferably from 0.1 to 30 $m^2/g$, more preferably from 0.5 to 20 $m^2/g$, and even more preferably from 1 to 10 $m^2/g$. The specific surface area SB in this invention is a value measured by the nitrogen gas adsorption method.

Oblate ellipsoidal polymer particle A has a bulk density of preferably from 0.01 to 0.7 g/mL, more preferably from 0.05 to 0.65 g/mL, and even more preferably from 0.1 to 0.6 g/mL.

Oblate ellipsoidal polymer particle A preferably has an affinity for at least aqueous systems or oil systems, and more preferably has an affinity for both. Specifically, it is preferable for the water absorption to be at least 60 mL per 100 g of particles and/or the oil absorption to be at least 60 mL per 100 g of particles, and more preferable for the water absorption to be at least 60 mL per 100 g of particles and the oil absorption to be at least 60 mL per 100 g of particles. In particular, the water absorption and the oil absorption are both more preferably at least 80 mL per 100 g of particles, even more preferably at least 100 mL per 100 g of particles, and most preferably at least 120 mL per 100 g of particles.

Moreover, oblate ellipsoidal polymer particle A, from the standpoint of increasing the heat resistance and chemical resistance, is preferably a crosslinked polymer particle. The crosslinking method is not particularly limited, although in the subsequently described method for producing oblate ellipsoidal polymer particle A, the crosslinking method may be, for example, one which carries out the polymerization reaction using a polyfunctional monomer that contributes as a crosslinking agent. By having the polymer be crosslinked, the resulting polymer particles can be endowed with an excellent heat resistance such that, when 0.5 g of the polymer particles are heated for two hours at 100°C, they maintain their initial shape (heat resistance is at least 100°C).

The material making up oblate ellipsoidal polymer particle A is not particularly limited, provided it can be obtained using a solution-polymerizable monomer. For example, the material is preferably composed of at least one selected from among styrene resins, (meth)acrylic resins, vinyl carboxylate resins, poly-N-vinyl compound resins, polyolefin resins, polydiene resins, polyester resins, silicone resins, polyurethane resins, polyamide resins, polyimide resins, epoxy resins, polyvinyl butyral resins, phenolic resins, amino resins, oxazoline resins and carbodiimide resins. These resins may be either homopolymers or copolymers. For example, "styrene resins" are resins that use a styrene compound as the main constituent unit, and include not only homopolymers of styrene compounds, but also copolymers of styrene compounds or of a styrene compound and another monomer.

Exemplary styrene resins include (co)polymers of styrene compounds, and copolymers of styrene and an olefin or a conjugated diene, such as styrene-(meth)acrylic acid copolymers, styrene-(meth)acrylic ester copolymers, acrylonitrile-styrene copolymers, acrylonitrile-chlorinated polyethylene-styrene copolymers, styrene-maleic anhydride copolymers or modified forms thereof, styrene-butadiene block copolymers (SBR), styrene-butadiene-styrene block copolymers (SBS), hydrogenated styrene-butadiene-styrene block copolymers (SEBS), styrene-isoprene block copolymers (SIR), styrene-isoprene-styrene block copolymers (SIS) and hydrogenated styrene-isoprene-styrene block copolymers (SEPS).

Exemplary (meth)acrylic resins include (meth)acrylic acid (co)polymers, (meth)acrylate ester (co)polymers, (meth)acrylic acid-(meth)acrylate ester copolymers, vinyl carboxylate-(meth)acrylic acid copolymers, vinyl carboxylate-(meth)acrylate ester copolymers, olefin-(meth)acrylic acid copolymers such as ethylene-acrylic acid copolymers, olefin-(meth)acrylate ester copolymers such as ethylene-acrylic ester copolymers, N-vinyl compound-(meth)acrylic acid copolymers, N-vinyl compound-(meth)acrylate ester copolymers, conjugated diene-(meth)acrylic acid copolymers and conjugated diene-(meth)acrylate ester copolymers.

Exemplary vinyl carboxylate resins include (co)polymers of vinyl carboxylates, olefin-vinyl carboxylate copolymers such as ethylene-vinyl acetate copolymers, and vinyl carboxylate-conjugated diene copolymers. Exemplary poly-N-vinyl compound resins include (co)polymers of N-vinyl compounds, olefin-N-vinyl compound copolymers and conjugated diene-N-vinyl compound copolymers. Exemplary polyolefin resins include polyolefins, polyfluorinated olefins, copolymers of olefins and/or fluorinated polyolefins, and olefin-conjugated diene copolymers. Exemplary polydiene resins include (co)polymers of conjugated dienes.

Two or more resins made of unsaturated monomers, such as the above styrene resins, (meth)acrylic resins, vinyl carboxylate resins, poly-N-vinyl compound resins, polyolefin resins and polydiene resins, may be formed into a copolymer in accordance with the intended use and purpose.

Exemplary polyester resins include polyester resins made of an acid component that is primarily terephthalic acid or dimethyl terephthalate, and a glycol component that is primarily at least one alkylene glycol selected from among ethylene glycol, diethylene glycol, trimethylene glycol and butylene glycol; and polylactic acid. Specific examples include polyethylene terephthalate, polyethylene naphthalate, polybutylene terephthalate, polybutylene naphthalate, polytrimethylene terephthalate, polycyclohexylenedimethylene terephthalate, polycyclohexylenedimethylene naphthalate and polylactic acid.

Exemplary silicone resins include, without particular limitation, those containing silicon-silicon bonds, silicon-carbon bonds, siloxane bonds or silicon-nitrogen bonds on the molecular chain. Illustrative examples include polysiloxane, polycarbosilane and polysilazane.

Exemplary polyurethane resins include polyurethane resins obtained by polymerizing a polyol and a polyisocyanate. Examples of the polyol in this case include ethylene glycol, diethylene glycol, propylene glycol, dipropylene glycol, glycerol, 1,1,1-trimethylolpropane, 1,2,5-hexanetriol, 1,3-butanediol, 1,4-butanediol, 4,4'-dihydroxyphenylpropane, 4,4'-dihydroxyphenylmethane and pentaerythritol. Examples of the polyisocyanate include 4-tolylene diisocyanate, 2,6-tolylene diisocyanate, 4,4'-diphenylmethane diisocyanate, 2,4'-diphenylmethane diisocyanate, p-phenylene diisocyanate, isophorone diisocyanate and xylylene diisocyanate.

Examples of polyamide resins include polyamide resins obtained by polycondensing a dicarboxylic acid such as adipic acid, heptanedicarboxylic acid, octanedicarboxylic acid, nonanedicarboxylic acid, undecanedicarboxylic acid or dodecanedicarboxylic acid with a diamine such as tetramethylenediamine, hexamethylene diamine, octamethylenediamine, nonamethylenediamine, undecamethylenediamine or dodecamethylenediamine. Additional examples include polyamide resins obtained by the ring-opening polymerization of a lactam such as α-pyrrolidone, ε-caprolactam, ω-laurolactam or ε-enantholactam. Specific examples include nylon-6, nylon-11, nylon-12, nylon-6,6 and nylon-6,T.

Examples of polyimide resins include polyimide resins obtained by polymerizing a diamine such as o-phenylenediamine, m-phenylenediamine, p-phenylenediamine, 4,4'-diaminodiphenyl ether, 1,4-bis(aminomethyl)cyclohexane, 1,3-bis(aminomethyl)cyclohexane, 1,3-propanediamine, 1,4-butanediamine, 1,5-pentanediamine or 1,6-hexanediamine with a tetracarboxylic dianhydride such as 4,4'-hexafluoropropylidenebisphthalic dianhydride, 4,4'-biphthalic anhydride, diphenyl-2,3,3',4'-tetracarboxylic dianhydride, diphenyl-2,2',3,3'-tetracarboxylic dianhydride or pyromellitic dianhydride.

Exemplary epoxy resins include polyepoxides, aromatic polyepoxy compounds, glycidyl ethers of polyhydric phenols, glycidyl esters of polyhydric phenols, glycidyl aromatic polyamines, alicyclic polyepoxy compounds, aliphatic polyepoxy compounds and polyglycidyl esters of polyunsaturated fatty acids. Of these, aliphatic polyepoxy compounds and aromatic polyepoxy compounds are preferred.

Examples of polyvinyl butyral resins include the reaction product of polyvinyl alcohol and butyraldehyde, and the products obtained by crosslinking between the molecules with monobutyral bonds.

Examples of phenolic resins include resins obtained using organic compounds belonging to the phenols, such as phenol and cresol.

Examples of amino resins include urea resins, melamine resins and guanamine resins.

Examples of oxazoline resins include bisoxazoline compounds and compounds having a terminal oxazoline group that are obtained by reacting two chemical equivalents of oxazoline groups on a bisoxazoline compound with one chemical equivalent of carboxyl groups on a polybasic carboxylic acid. The oxazoline compound may be a polymer having at least two oxazoline groups per molecule obtained from a polymer by, for example, addition polymerization without ring opening of the oxazoline rings. Other exemplary oxazoline resins include copolymers of an addition polymerizable oxazoline compound and a copolymerizable monomer that does not react with oxazoline groups.

Examples of carbodiimide resins include resins having at least one carbodiimide group that are obtained using one, two or more isocyanate compounds as the starting material.

Of these, the material making up oblate ellipsoidal polymer particle A is more preferably a styrene resin, a (meth)acrylic resin, a vinyl carboxylate resin, a poly-N-vinyl compound resin, a polyolefin resin, a polydiene resin, a polyester resin, a silicone resin or a polyamide resin.

Oblate ellipsoidal polymer particle A is made of preferably a (co)polymer obtained using at least one type of monomer selected from among styrene compounds, (meth)acrylic acids, (meth)acrylate esters, vinyl carboxylates, N-vinyl compounds, olefins, fluorinated olefins and conjugated dienes; and more preferably a (co)polymer containing, as essential units, recurring units obtained from at least one monomer selected from among styrene compounds, (meth)acrylic acids and (meth)acrylate esters.

Illustrative examples include preferably polystyrene, styrene-(meth)acrylic acid copolymers, styrene-(meth)acrylate ester copolymers, poly(meth)acrylic acids, polymethyl (meth)acrylate, polyethyl (meth)acrylate, polybutyl (meth)acrylate, (meth)acrylic acid-methyl (meth)acrylate copolymers, (meth)acrylate ester copolymers, polyvinyl acetate, poly-N-vinylpyrrole, poly-N-vinylcarbazole, poly-N-vinylindole, poly-N-vinylpyrrolidone, polyethylene, polypropylene, polyvinyl fluoride, polytetrafluoroethylene, polybutadiene, polyisoprene and copolymers thereof; and more preferably polystyrene, styrene-(meth)acrylic acid copolymers, styrene-(meth)acrylate ester copolymers, poly(meth)acrylic acid, polymethyl (meth)acrylate, polyethyl (meth)acrylate, polybutyl (meth)acrylate, (meth)acrylic acid-methyl (meth)acrylate copolymers and (meth)acrylate ester copolymers. As mentioned above, the resin may be a suitable cured copolymer obtained using a polyfunctional crosslinking agent.

Oblate ellipsoidal polymer particle A is suitable as an additive for cosmetic materials. While retaining such features inherent to ellipsoidal polymer particles as their low weight, tactile qualities, flowability and solution dispersibility, oblate ellipsoidal polymer particle A has, on account of its distinctive shape, an adhesive strength that differs from that of ordinary spherical particles, and is thus effective for improving the bonding strength of pressed compacts of, for example, foundation and also the holding power following application. In addition, oblate ellipsoidal polymer particle A has a UV-scattering effect and is suitable also as a UV-cutting additive. The light-scattering properties and other optical characteristics of oblate ellipsoidal polymer particle A make the skin appear lighter and can enhance the covering power due to a shading effect. Also, due to the slip characteristics particular to the particle shape, spreadability over the skin is excellent and furrows in the skin texture are finely filled, making wrinkles and pores inconspicuous, and the flowability of the overall product can be freely controlled. Moreover, by utilizing the adhesive strength and holding power, the amount of polymer addition in the overall product can be increased, enabling the discovery of entirely new cosmetic effects.

Oblate ellipsoidal polymer particle A may be of one type used alone, or two or more types which satisfy above conditions (1) to (3) may be used in combination. Using two or more types of oblate ellipsoidal polymer particle A in combination is more preferable because the skin cosmetic of the invention can be applied uniformly without gaps onto the skin, making spreadability over the skin even better.

[Method for Producing Oblate Ellipsoidal Polymer Particle A]

Because oblate ellipsoidal polymer particle A can be produced in one step by solution polymerization, and can be produced by a batch process that does not include a particle compression/transfer step, bulk production at one time without passing through a plurality of steps is possible. Solution polymerization in this invention is defined as a polymerization process that causes a polymerization reaction to proceed in a medium containing at least a monomer and a polymerization initiator, and induces polymer particles to settle out or form. Examples of solution polymerization processes include suspension polymerization, emulsion polymerization, dispersion polymerization and seed polymerization, as well as combined processes based on these.

Suspension polymerization is a process in which a monomer and agents such as a polymerization initiator that are soluble in the monomer are mechanically agitated and suspended in a medium in which these do not readily dissolve, causing the polymerization reaction to proceed in the suspended state and inducing polymer particles to settle out or form. Emulsion polymerization is a process in which a medium such as water is mixed with a monomer and agents such as an emulsifying agent (surfactant) that are poorly soluble in the medium, along with which a polymerization initiator soluble in the medium is added, causing the polymerization reaction to proceed and inducing polymer particles to settle out or form. Dispersion polymerization is a process in which the polymerization reaction is made to proceed in a uniform solution of monomer, polymerization initiator, dispersant, stabilizer and the like dissolved in a liquid medium within which the monomer dissolves but becomes insoluble with polymerization, inducing polymer particles to settle out or form. Seed polymerization is a polymerization process in which other particles serving as seeds are added beforehand at the time of polymerization and the polymerization reaction is carried out at the surface of these particles.

Oblate ellipsoidal polymer particle A can be obtained by these various types of solution polymerization, although it is preferable to produce oblate ellipsoidal polymer particle A by a solution polymerization process other than seed polymerization; that is, by suspension polymerization, emulsion polymerization, dispersion polymerization, or a combination thereof. With these methods, the seed particle preparation step required in seed polymerization can be omitted.

In this invention, by varying the types and weight ratios of the monomer, polymerization initiator and solvent in these methods, and also the ingredients and weight ratios of the dispersant and the emulsifying agent, the target oblate ellipsoidal polymer particle A can be prepared.

In particular, to efficiently obtain oblate ellipsoidal polymer particle A, it is preferable to use a mixed solvent containing three types of solvent: water, a hydrophilic organic solvent and a hydrophobic organic solvent. No particular limitation is imposed on the proportions in which the water, hydrophilic organic solvent and hydrophobic organic solvent are used. However, from the standpoint of efficiently obtaining oblate ellipsoidal polymer particle A, the weight ratio of water to hydrophilic organic solvent to hydrophobic organic solvent is preferably from 98:1:1 to 30:30:40, more preferably from 96:2:2 to 40:25:35, and even more preferably from 93:2:5 to 50:20:30. In particular, the hydrophilic organic solvent and the hydrophobic organic solvent are used in proportions which preferably satisfy the condition

hydrophilic organic solvent ≤ hydrophobic organic solvent, and more preferably satisfy the condition
hydrophilic organic solvent < hydrophobic organic solvent.

It is preferable to select a hydrophilic organic solvent and a hydrophobic organic solvent which are miscible (compatible). In addition, it is preferable to select a solvent such that the monomer serving as the chief ingredient (more than 50 wt%) of the monomers used in polymerization dissolves in at least the hydrophilic organic solvent or the hydrophobic organic solvent. In this invention, "hydrophilic organic solvent" refers to a solvent which maintains a uniform appearance as a mixed solvent with an equal volume of water. "Hydrophobic organic solvent" refers to a solvent which, when gently mixed with an equal volume of pure water at one atmosphere ($1.013\times10^5$ Pa) and a temperature of 20°C, results in a mixed liquid that cannot maintain a uniform appearance after flow has subsided.

Examples of the water include tap water, deionized water and distilled water.

Examples of hydrophilic organic solvents include methanol, ethanol, 1-propanol, 2-propanol, ethylene glycol, propylene glycol, butylene glycol, dipropylene glycol, methyl cellosolve, ethyl cellosolve, propyl cellosolve, methyl cellosolve acetate, ethyl cellosolve acetate, methyl carbitol, ethyl carbitol, butyl carbitol, ethyl carbitol acetate, acetone, tetrahydrofuran, dimethyl formamide, N-methyl-2-pyrrolidone and acetonitrile. These may be used singly or two or more may be used in admixture.

Examples of hydrophobic organic solvents include higher alcohols such as 1-butanol, 2-butanol, isobutanol, tert-butanol, 1-pentanol, 2-pentanol, 3-pentanol, 2-methyl-1-butanol, isopentyl alcohol, tert-pentyl alcohol, 1-hexanol, 2-methyl-1-pentanol, 4-methyl-2-pentanol, 2-ethylbutanol, 1-heptanol, 2-heptanol, 3-heptanol, 2-octanol, 2-ethyl-1-hexanol, benzyl alcohol and cyclohexanol; ether alcohols such as butyl cellosolve; polyethers such as polypropylene glycol and polybutylene glycol; ketones such as methyl ethyl ketone, methyl isobutyl ketone and cyclohexanone; esters such as ethyl acetate, butyl acetate, ethyl propionate and butyl carbitol acetate; aliphatic or aromatic hydrocarbons such as pentane, 2-methylbutane, n-hexane, cyclohexane, 2-methylpentane, 2,2-dimethylbutane, 2,3-dimethylbutane, heptane, n-octane, isooctane, 2,2,3-trimethylpentane, decane, nonane, cyclopentane, methyl cyclopentane, methyl cyclohexane, ethyl cyclohexane, p-menthane, dicyclohexyl, benzene, toluene, xylene, ethylbenzene, liquid paraffin, mineral oil and heat transfer medium oils; siloxane compounds such as polydimethylsiloxane, polymethylphenylsiloxane, polydiphenylsiloxane and silicone oils; and halogenated hydrocarbons such as carbon tetrachloride, trichloroethylene, chlorobenzene and tetrabromoethane. These hydrophobic organic solvents may include modified compounds and copolymers and other modified polymer compounds that are substituted with carbon, nitrogen, oxygen, hydrogen, halogen or the like, within a range that does detract from the advantageous effects of the invention. Such hydrophobic organic solvents may be used singly or two or more may be used in combination.

In particular, it is preferable for the hydrophobic organic solvent that is used to be a hydrophobic organic solvent which has 8 or more carbon atoms and does not react with the starting monomer under the polymerization conditions. By having such a hydrophobic organic solvent be present within the reaction system, the dispersibility of the polymer particles as they form can be enhanced, making more uniform control of the particle size possible. The organic compound having 8 or more carbon atoms is not particularly limited, provided that it is a solid or liquid at room temperature, is compatible with the hydrophilic organic solvent to be used and does not have an adverse influence on the polymerization reaction. However, taking into account such factors as the polymerization reaction temperature, an organic compound having a melting point of not more than 80°C is preferred, one having a melting point of not more than 60°C is more preferred, one having a melting point of not more than 40°C is even more preferred, and one having a melting point of not more than 30°C is best.

Such organic compounds are exemplified by hydrocarbon compounds, siloxane compounds and polyalkylene oxide group-containing compounds. The number of carbon atoms should be 8 or more, although taking into account the dispersion stability of the particles to be obtained, the number of carbon atoms is preferably at least 10, more preferably at least 12, and most preferably at least 15.

The molecular weight of the hydrophobic organic solvent is preferably at least 200, more preferably at least 300, even more preferably at least 500, and most preferably at least 1,000. By thus using a hydrophobic organic solvent having a high molecular weight, together with functioning as a solvent, this compound also carries out a dispersant-like role, minimizing sticking and agglomeration of the particles and making it possible to obtain polymer particles that are stably monodispersed and have a controlled particle size.

High-molecular-weight compounds having recurring units are preferred as the hydrophobic organic solvent having a molecular weight of 200 or more. Examples include hydrocarbon compounds, siloxane compounds and polyalkylene oxide group-containing compounds. These high-molecular-weight compounds are more preferably high-molecular-weight compounds which are water-soluble in a low-molecular-weight state and exhibit hydrophobicity as the molecular weight increases, or hydrophobic organic solvents obtained by polymerizing a monomer having a polar group at the interior of the molecule. By having such polar groups at the interior of the molecule, the subsequently described stabilizer readily disperses uniformly within the solvent, further contributing to particle stability. Examples of such polar groups include hydroxyl, ether and carbonyl groups.

Examples of hydrophobic organic solvents that satisfy these conditions include, of the hydrophobic organic solvents mentioned above, polyethers such as polypropylene glycol and polybutylene glycol; and siloxane compounds such as polydimethylsiloxane, polymethylphenylsiloxane, polydiphenylsiloxane and silicone oils.

The viscosity of the hydrophobic organic solvent, although not particularly limited, is preferably at least 0.001 Pa·s at 25°C.

Illustrative examples of the polymerizable monomer serving as the starting material for oblate ellipsoidal polymer particle A include:

(i) styrene compounds such as styrene, o-methylstyrene, n-methylstyrene, p-methylstyrene, $\alpha$-methylstyrene, o-ethylstyrene, m-ethylstyrene, p-ethylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, p-phenylstyrene, p-chlorostyrene and 3,4-dichlorostyrene;
(ii) (meth)acrylic acid;
(iii) hydrocarbon group-containing (meth)acrylic monomers such as methyl (meth)acrylate, ethyl (meth)acrylate, propyl (meth)acrylate, n-butyl (meth)acrylate, isobutyl (meth)acrylate, pentyl (meth)acrylate, hexyl (meth)acrylate, 2-ethylhexyl (meth)acrylate, octyl (meth)acrylate, nonyl (meth)acrylate, decyl (meth)acrylate, dodecyl (meth)acrylate, lauryl (meth)acrylate, stearyl (meth)acrylate, cyclohexyl (meth)acrylate, isobornyl (meth)acrylate, phenyl (meth)acrylate, toluyl (meth)acrylate and benzyl (meth)acrylate; fluorine-containing (meth)acrylic monomers such as 2,2,2-trifluoroethyl (meth)acrylate, 3,3,3-trifluoropropyl (meth)acrylate, 2- (perfluoroethyl)ethyl (meth)acrylate, 2-perfluoroethyl-2-perfluorobutylethyl (meth)acrylate, 2-perfluoroethyl (meth)acrylate, tetrafluoropropyl (meth)acrylate, perfluoromethyl (meth)acrylate, 1,1,1,3,3,3-hexafluoropropan-2-yl (meth)acrylate, 2-perfluoromethyl-2-perfluoroethylmethyl (meth)acrylate, 2-(perfluorohexyl)ethyl (meth)acrylate, 2-(perfluorodecyl)ethyl (meth)acrylate and 2-(perfluorohexadecyl)ethyl (meth)acrylate; silicon-containing (meth)acrylic monomers such as $\gamma$-(methacryloyloxy-

propyl)trimethoxysilane and γ-(methacryloyloxypropyl)dimethoxymethylsilane; alkoxy group-containing (meth)acrylic monomers such as (poly)ethylene glycol mono(meth)acrylate, 2-methoxyethyl (meth)acrylate and 3-methoxybutyl (meth)acrylate; (poly)alkylene glycol (meth)acrylic monomers such as (poly)propylene glycol mono(meth)acrylate; alkoxy(poly)alkylene glycol (meth)acrylic monomers such as methoxy(poly)ethylene glycol mono(meth)acrylate and methoxy(poly)propylene glycol mono(meth)acrylate; and (meth)acrylate esters containing no reactive functional groups, such as 2-chloroethyl (meth)acrylate and methyl α-chloro(meth)acrylate;

(iv) vinyl carboxylates such as vinyl acetate, vinyl propionate, vinyl benzoate, vinyl butyrate, vinyl formate, vinyl valerate and vinyl pivalate;

(v) N-vinyl compounds such as N-vinylpyrrole, N-vinylcarbazole, N-vinylindole and N-vinylpyrrolidone;

(vi) olefins such as ethylene and propylene;

(vii) fluorinated olefins such as vinyl fluoride, vinylidene fluoride, tetrafluoroethylene and hexafluoropropylene; and

(viii) conjugated dienes such as butadiene and isoprene.

These may be used singly or two or more may be used in combination.

Of these, styrene compounds, (meth)acrylic acids, (meth)acrylate esters and vinyl carboxylates as the polymerizable monomer are preferred. By using these, it is possible to easily and inexpensively obtain oblate ellipsoidal polymer particle A.

Aside from the above polymerizable monomer, an unsaturated monomer having a reactive functional group such as a hydrophilic functional group or an active hydrogen group may be used, either alone or in combination with the above polymerizable monomer. Examples of such reactive functional groups include amino, carboxyl, hydroxyl, thiol, carbonyl, ether, cyano, amide, alkylene oxide, epoxy and ionic functional groups. One type of functional group may exist by itself, or two or more types may be present together, on the unsaturated monomer. Introducing these reactive functional groups such as hydrophilic functional groups and active hydrogen groups to the interior of the particles or to the surface layer not only enables enhanced functionality such as hydrophilicity or oil resistance to be achieved, it also enables the use of these groups as auxiliary functional groups that confer various kinds of functionality, such as the formation of a composite structure with inorganic particles or other types of polymer particles, the formation of a crosslinked structure owing to reactions between the functional groups, surface treatment and surface modification due to bonding of the reactive compound, and the imparting of active substances.

Unsaturated monomers having such reactive functional groups are exemplified as shown below. In the explanation that follows, "$C_n$" means "n number of carbon atoms."

(1) Amino Group-Containing Monomers

Examples include amino group-containing (meth)acrylic monomers such as 2-aminoethyl (meth)acrylate, N-propylaminoethyl acrylate, N-ethylaminopropyl (meth)acrylate, N-phenylaminoethyl (meth)acrylate and N-cyclohexylaminoethyl (meth)acrylate; allylamine derivatives such as allylamine and N-methylallylamine; amino group-containing styrene derivatives such a p-aminostyrene; and triazine derivatives such as 2-vinyl-4,6-diamino-S-triazine. Of these, compounds having a primary or secondary amino group are preferred.

(2) Carboxyl Group-Containing Monomers

Examples include unsaturated carboxylic acids such as (meth)acrylic acid, crotonic acid, cinnamic acid, itaconic acid, maleic acid and fumaric acid; mono($C_{1-8}$ alkyl) esters of itaconic acid such as monobutyl itaconate; mono($C_{1-8}$ alkyl) esters of maleic acid such as monobutyl maleate; vinyl group-containing aromatic carboxylic acids such as vinylbenzoic acid; and salts thereof.

(3) Hydroxyl Group-Containing Monomers

Examples include hydroxyl group-containing (meth)acrylic monomers such as 2-hydroxyethyl (meth)acrylate, 2-hydroxypropyl (meth)acrylate, 3-hydroxypropyl (meth)acrylate and 4-hydroxybutyl (meth)acrylate; hydroxyalkyl vinyl ether monomers such as hydroxyethyl vinyl ether and hydroxybutyl vinyl ether; and hydroxyl group-containing allyl monomers such as allyl alcohol and 2-hydroxyethyl allyl ether.

(4) Thiol (Mercapto) Group-Containing Monomers

Examples include thiol group-containing (meth)acrylic monomers such as N-(2-mercaptoethyl) acrylamide, N-(2-mercapto-1-carboxyethyl) acrylamide, N-(2-mercaptoethyl) methacrylamide, N-(4-mercaptophenyl) acrylamide, N-(7-mercaptonaphthyl) acrylamide, maleic acid mono(2-mercaptoethylamide), 2-mercaptoethyl (meth)acrylate and 2-mercapto-1-carboxyethyl (meth)acrylate.

(5) Carbonyl Group-Containing Monomers

Examples include vinyl group-containing ketones such as vinyl methyl ketone, vinyl hexyl ketone and methyl isopropenyl ketone.

(6) Ether Group-Containing Monomers

Examples include vinyl group-containing ether monomers such as vinyl methyl ether, vinyl ethyl ether and vinyl isobutyl ether.

(7) Cyano Group-Containing Monomers

Examples include acrylonitrile, methacrylonitrile, hexenenitrile, 4-pentenenitrile and p-cyanostyrene.

(8) Amide Group-Containing Monomers

Examples include (meth)acrylamide, α-ethyl (meth)acrylamide, N-methyl (meth)acrylamide, N-butoxymethyl (meth)acrylamide, diacetone (meth)acrylamide, N,N-dimethyl (meth)acrylamide, N,N-diethyl (meth)acrylamide, N,N-dimethyl-p-styrene sulfonamide, N,N-dimethylaminoethyl (meth)acrylamide, N,N-diethylaminoethyl (meth)acrylamide, N,N-dimethylaminopropyl (meth)acrylamide and N,N-diethylaminopropyl (meth)acrylamide.

(9) Epoxy Group-Containing Monomers

Examples include epoxy group-containing (meth)acrylic monomers such as glycidyl (meth)acrylate, (β-methyl)glycidyl (meth)acrylate and 3,4-epoxycyclohexyl (meth)acrylate; epoxy group-containing vinyl monomers such as allyl glycidyl ether and 3,4-epoxyvinylcyclohexane; and di(β-methyl)glycidyl maleate and di(β-methyl)glycidyl fumarate.

(10) Ionic Functional Group-Containing Monomers

The ionic functional groups may be anionic functional groups or cationic functional groups. Examples of anionic functional groups include carboxyl groups, sulfonic acid groups, phosphoric acid groups, phenolic hydroxyl groups, and salts thereof. Examples of cationic functional groups include amino groups, imidazole groups, pyridine groups, amidino groups, and salts thereof.

Anionic functional groups are especially preferred because there are numerous widely used products and plentiful types, and also because the size, shape and other particle characteristics can be efficiently controlled. In addition, of these, because introduction to the interior of the molecule is easy and the stability and safety are excellent, it is preferable for the anionic functional group to be one or more functional group selected from among carboxylic groups, sulfonic acid groups, phosphoric acid groups and derivatives thereof.

As for compounds capable of becoming counterions for these ionic functional groups, examples of counterions for anionic functional groups include metal cations, ammonium cations, pyridinium cations and phosphonium cations; examples of counterions for cationic functional groups include halide ions such as the chloride, bromide and iodide ions.

Of the above unsaturated monomers having reactive functional groups, monomers having a hydroxyl group, carboxyl group, amino group, amide group, alkylene oxide group or ionic functional group are preferred; and monomers having a hydroxyl group, carboxyl group, ethylene oxide group or ionic functional group are more preferred. By using these functional groups, the hydrophilicity is stronger and the repulsion between particles obtained in solution is stronger. As a result, the dispersion system has a higher stability and the monodispersibility can be improved even further, enabling declines in particle size accuracy due to particle sticking and agglomeration to be mitigated and moreover making it possible to obtain polymer particles of excellent chemical resistance, reactivity, solvent dispersibility, powder dispersibility and mechanical properties.

During the polymerization reaction, depending on, for example, heat-resistant or chemical-resistant applications in which the resulting particles may be used, a crosslinking agent may be included in a suitable amount of from 0.01 to 80 wt%, based on the total weight of the polymerization ingredients. Illustrative examples of the crosslinking agent include aromatic divinyl compounds such as divinylbenzene, divinylbiphenyl and divinylnaphthalene; (poly)alkylene glycol di(meth)acrylates such as (poly)ethylene glycol di(meth)acrylate, (poly)propylene glycol di(meth)acrylate and (poly)tetramethylene glycol di(meth)acrylate; alkanediol di(meth)acrylates such as 1,6-hexanediol di(meth)acrylate, 1,8-octanediol di(meth)acrylate, 1,9-nonanediol di(meth)acrylate, 1,10-decanediol di(meth)acrylate, 1,12-dodecandiol di(meth)acrylate, 3-methyl-1,5-pentanediol di(meth)acrylate, 2,4-diethyl-1,5-pentanediol di(meth)acrylate, butylethylpropanediol di(meth)acrylate, 3-methyl-1,7-octanediol di(meth)acrylate and 2-methyl-1,8-octanediol di(meth)acrylate; alkanediol di(meth)acrylates such as 1,6-hexanediol di(meth)acrylate, 1,8-octanediol di(meth)acrylate, 1,9-nonanediol di(meth)acrylate, 1,10-decanediol di(meth)acrylate, 1,12-dodecanediol di(meth)acrylate, 3-methyl-1,5-pentanediol di(meth)acrylate, 2,4-diethyl-1,5-pentanediol di(meth)acrylate, butylethylpropanediol di(meth)acrylate, 3-methyl-1,7-octanediol di(meth)acrylate and 2-methyl-1,8-octanediol di(meth)acrylate; polyfunctional (meth)acrylates such as glycerol di(meth)acrylate, pentaerythritol tri(meth)acrylate, pentaerythritol tetra(meth)acrylate, pentaerythritol di(meth)acrylate, pentaerythritol tetra(meth)acrylate, glycerol acryloxy di(meth)acrylate, ethoxylated cyclohexane dimethanol di(meth)acrylate, ethoxylated bisphenol A di(meth)acrylate, tricyclodecane dimethanol di(meth)acrylate, propoxylated ethoxylated bisphenol A di(meth)acrylate, 1,1,1-trishydroxymethylethane di(meth)acrylate, 1,1,1-trishydroxymethylethane tri(meth)acrylate, 1,1,1-trishydroxymethylpropane tri(meth)acrylate, caprolactone-modified dipentaerythritol hexa(meth)acrylate, caprolactone-modified hydroxypivalate neopentyl glycol di(meth)acrylate, polyester (meth)acrylate and urethane (meth)acrylate; and compounds such as N,N-divinylaniline, divinyl ether, divinylsulfide and divinylsulfone. Unsaturated monomers having reactive functional groups such as the above epoxy group-containing (meth)acrylic monomers may also be used as the crosslinking agent. These crosslinking agents may be used singly or two or more may be used in combination.

Methods for producing crosslinked particles are exemplified by a method that increases the degree of crosslinking by adding the above polyfunctional unsaturated monomer; a method that increases the degree of crosslinking by copolymerizing the above-mentioned reactive group-containing unsaturated monomers under specific pH conditions; and a method (referred to below as "post-crosslinking") in which the above reactive group-containing unsaturated monomers are copolymerized to form particles, after which an organic compound that reacts with the reactive functional groups is placed, in the presence of at least one solvent selected from among water and organic solvents that does not dissolve the precipitated particles but does dissolve the organic compound, in a state where the organic compound has impregnated only the surface layer of the particles or both the surface layer and the interior of the particles, thereby causing crosslinking reactions to proceed via reactions of the functional groups contained in the particles with the reactive group on the organic compound.

Here, the organic compound for post-crosslinking is exemplified by organic compounds having, for example, a hydroxyl, carboxyl, amino, thiol, carbonyl, ether, cyano, epoxy (glycidyl), amide, isocyanate, carbodiimide, oxazoline or alkylene oxide group as the reactive functional group.

In the above production methods, it is especially preferable to use as the unsaturated monomer at least an unsaturated monomer which is hydrophobic and is liquid at 25°C. Preferred examples of such hydrophobic liquid unsaturated monomers include the (i) styrene compounds, (iii) hydrocarbon group-containing (meth)acrylic monomers, fluorine-containing (meth)acrylic monomers and epoxy group-containing (meth)acrylic monomers, (vi) olefins, (vii) fluorinated olefins and (viii) conjugated dienes mentioned above as polymerizable monomers; the (9) epoxy group-containing vinyl monomers mentioned above as reactive functional group-containing unsaturated monomers; and the aromatic divinyl compounds, (poly)alkylene glycol di(meth)acrylates, alkanediol di(meth)acrylates and polyfunctional (meth)acrylates mentioned above as crosslinking agents. The hydrophobic liquid unsaturated monomer is used in an amount of preferably from 50 to 100 wt%, more preferably from 60 to 100 wt%, even more preferably from 70 to 100 wt%, and still more preferably from 80 to 100 wt%, of the total monomer used in polymerization.

Also, in these production methods, in order to efficiently obtain oblate ellipsoidal polymer particle A, with the use of such a hydrophobic liquid unsaturated monomer, the proportion of hydrophobic liquid ingredients, including the hydrophobic liquid unsaturated monomer and the hydrophobic organic solvent, is set to preferably at least 10 wt%, more preferably at least 20 wt%, even more preferably at least 25 wt%, and most preferably at least 30 wt%, of the total amount of charged ingredients. In this case, the hydrophobic organic solvent is preferably one which has the ability to dissolve the hydrophobic liquid unsaturated monomers but does not dissolve the oblate ellipsoidal polymer particle A that forms in the polymerization reaction. Examples of such hydrophobic organic solvents include polyethers such as polypropylene glycol and polybutylene glycol, siloxane compounds such as polydimethylsiloxane, polymethylphenylsiloxane, polydiphenylsiloxane and silicone oils, liquid paraffin, and aliphatic or aromatic hydrocarbons such as heat transfer medium oils.

The viscosity at 25°C of the charged ingredients as a whole is adjusted to preferably at least 0.001 Pa·s, more preferably at least 0.002 Pa·s, even more preferably at least 0.005 Pa·s, and most preferably at least 0.01 Pa·s. The upper limit is less than 10 Pa·s. At a higher viscosity that this, the yield of oblate ellipsoidal polymer particle A may decrease. The viscosity can be easily adjusted by way of the organic solvent used and by adding, for example, the subsequently described stabilizer.

In the above production methods, it is preferable to use one polymerization initiator that dissolves in at least one solvent type from among water, hydrophilic organic solvents and hydrophobic organic solvents, or to use a combination of two or more polymerization initiators that dissolve in all of these solvent types: water, hydrophilic organic solvents and hydrophobic organic solvents.

In this case, when the three types of solvent have been mixed together by agitation and then left to stand, there arises a "fuzzy" state within which an emulsified layer (bottom layer, or water-rich layer), a dissolved layer (intermediate layer, or hydrophilic solvent-rich layer) and a separated layer (top layer, or hydrophobic solvent-rich layer) co-exist. In the polymerization reaction as well, the reaction is thought to proceed in a condition where the unsaturated monomer has dissolved into each of these layers within the fuzzy state. In a solvent system that forms this fuzzy state, by additionally using the above-described combination of polymerization initiators, the polymerization reaction on the unsaturated monomer can be made to proceed in a state where at least one of the polymerization initiators used has dissolved in all three solvents--the water, the hydrophilic organic solvent and the hydrophobic organic solvent; that is, in a state where at least one of the polymerization initiators used is present in each of the layers: the emulsified layer, the dissolved layer and the separated layer. This appears to be why the shapes of the resulting polymer particles can be more efficiently rendered into oblate ellipsoidal shapes.

Concerning the above "combination" of polymerization initiators, when the polymerization initiator is one that dissolves in each of the solvents, i.e., the water, the hydrophilic organic solvent and the hydrophobic organic solvent, a single polymerization initiator may be used by itself. However, in this invention, it is preferable to use in combination at least one water-soluble initiator that dissolves in water and at least one oil-soluble initiator that does not dissolve in water. "Water-soluble initiator" refers herein to an initiator having a solubility in water of at least about 2 g per 100 mL, and "oil-soluble initiator" refers to an initiator having a solubility in water of less than about 2 g/100 mL.

The mixing ratio between the water-soluble initiator and the oil-soluble initiator is not particularly limited, although the weight ratio of water-soluble initiator to oil-soluble initiator is preferably from 99:1 to 1:99, more preferably from 95:5 to 5:95, even more preferably from 90:10 to 10:90, still more preferably from 80:20 to 20:80, and most preferably from 70:30 to 30:70.

Various known polymerization initiators may be used as the polymerization initiators in the invention. Illustrative examples of water-soluble polymerization initiators include the following water-soluble or ionic polymerization initiators: persulfates such as ammonium persulfate, sodium persulfate and potassium persulfate; and azo initiators such as 2,2'-azobis[2-methyl-N-(2-hydroxyethyl)propionamide], 2,2'-azobis(2-amidinopropane) dihydrochloride, 2,2' -azobis(2-methyl-N-phenylpropionamidine) dihydrochloride, 2,2'-azobis[N-(4-chlorophenyl)-2-methylpropionamidine] dihydrochloride, 2,2'-azobis[N-(4-hydroxyphenyl)-2-methylpropionamidine] dihydrochloride, 2,2'-azobis[N-(4-aminophenyl)-2-methylpropionamidine] tetrahydrochloride, 2,2'-azobis[2-methyl-N-(phenylmethyl)propionamidine] dihydrochloride, 2,2'-azobis[2-methyl-N-2-propenylpropionamidine] dihydrochloride, 2,2'-azobis[N-(2-hydroxyethyl)-2-methylpropionamidine] dihydrochloride, 2,2'-azobis[2-(5-methyl-2-imidazolin-2-yl)propane] dihydrochloride, 2,2'-azobis[2-(2-imidazolin-2-yl)propane] dihydrochloride, 2,2'-azobis[2-(4,5,6,7-tetrahydro-1H-1,3-diazepin-2-yl)propane] dihydrochloride, 2,2'-azobis[2-(3,4,5,6-tetrahydropyrimidin-2-yl)propane] dihydrochloride, 2,2'-azobis[2-(5-hydroxy-3,4,5,6-tetrahydropyrimidin-2-yl)propane] dihydrochloride, 2,2'-azobis{2-[1-(2-hydroxyethyl)-2-imidazolin-2-yl] propane} dihydrochloride, 2,2'-azobis-2-cyanopropane-1-sulfonic acid disodium salt, and sodium 4,4' -azobis(4-cyanopentanoate).

Examples of oil-soluble initiators include peroxides such as benzoyl peroxide, cumene hydroperoxide and tert-butyl hydroperoxide; and azo compounds such as azobisisobutyronitrile, azobismethylbutyronitrile, azobisisovaleronitrile, dimethyl 2,2'-azobis(isobutyrate), 2,2'-azobis(N-butyl-2-methylpropionamide), 4,4'-azobis(4-cyanopentanoic acid), 2,2'-azobis(2-amidinopropane) dihydrochloride and 2,2'-azobis(N,N'-dimethylene isobutyramidine) dihydrochloride.

These polymerization initiators may be used singly or two or more may be used in combination. It is preferable for the content of the radical polymerization initiator to be generally from 0.01 to 50 parts by weight per 100 parts by weight of the unsaturated monomer.

In the above production methods, the pH of the reaction system is not particularly limited; oblate ellipsoidal polymer particle A can generally be obtained without carrying out pH adjustment. However, during the polymerization reaction (at least following the start of heating and up until the time that the reaction is complete), by adjusting the pH of the solution to between 0 and 5 or between 9 and 14 and polymerizing the unsaturated monomer, it is possible to obtain monodispersible polymer particles having few agglomerates or impurities while maintaining an oblate ellipsoidal shape. The pH of the reaction solution is preferably between 0 and 4 or between 10 and 14, more preferably between 0 and 3 or between 11 and 14, and most preferably between 0 and 2 or between 12 and 14. When the reaction is made to proceed with the pH shifted to the acidic side, the polymerization reaction proceeds more stably; hence, it is preferable for the pH to be between 0 and 5.

A known pH adjustor may be suitably used to adjust the pH. Examples of pH adjustors include acids such as citric acid, tartaric acid, lactic acid, glycolic acid, hydrochloric acid, nitric acid, sulfuric acid, sodium citrate, sodium lactate, succinic acid, acetic acid, sodium acetate, fumaric acid, malic acid and phosphoric acid; and alkalis such as sodium hydroxide, potassium hydroxide, calcium hydroxide, magnesium hydroxide, sodium carbonate, potassium carbonate, calcium carbonate, ammonium carbonate, ammonia, morpholine, triethanolamine, diethanolamine, dimethylamine, diethylamine, trimethylamine and triethylamine.

Adjustment of the pH may be carried out by, for example, the gradual dropwise addition of a pH adjustor to the reaction solution following the start of the polymerization reaction so as to shift the pH to the acidic or alkaline side.

Alternatively, when the above-mentioned persulfate is used as the polymerization initiator, because it breaks down during the polymerization reaction and forms an acid, the pH gradually decreases. In this case, a pH adjustor need not be added.

The pH of the reaction system in this invention is the pH value of the reaction solution in an agitated state, as measured with a pH meter or with pH test paper.

The ingredients and composition of the water, hydrophilic organic solvent and hydrophobic organic solvent may be suitably adjusted so as to impart certain features, such as fine surface irregularities, porosity and a large specific surface area, to oblate ellipsoidal polymer particle A. It is possible in this way to suitably modify the particle surface and interior.

In this invention, by carrying out such adjustments in the solvent composition, it is possible to control the particle size and aspect ratio, the size of fine surface irregularities and the porosity of oblate ellipsoidal polymer particle A, enabling a good balance in performance attributes such as water absorption and oil absorption to be achieved.

The content of unsaturated monomer in the reaction solution is preferably from 1 to 80 wt%, more preferably from 5 to 60 wt%, even more preferably from 10 to 50 wt%, and most preferably from 15 to 45 wt%, of the overall reaction solution. When the content of unsaturated monomer is within this range, oblate ellipsoidal polymer particle A that is a monodispersed state can be obtained in a high yield, which is practical from an industrial standpoint.

The reaction temperature at the time of polymerization is suitably set according to the types of solvent and polymerization initiator used, but is typically from about 10°C to about 200°C, preferably from 30 to 130°C, and more preferably from 40 to 90°C.

The reaction time is not particularly limited, so long as it is the time needed for the intended reaction to go substantially to completion, and is suitably set according to such factors as the type, content and concentration of the unsaturated monomer, the viscosity of the solution, and the intended particle size. For example, within the above-indicated temperature range, the reaction time is typically from 1 to 72 hours, and preferably from about 2 hours to about 24 hours.

During production of oblate ellipsoidal polymer particle A, depending on the polymerization method, (polymer) dispersants, stabilizers, emulsifying agents (surfactants) and the like may also be included in a suitable amount of from 0.01 to 50 wt%, based on the starting monomer mentioned above.

Dispersants and stabilizers are exemplified by various types of hydrophobic or hydrophilic dispersants and stabilizers, including polystyrene derivatives such as polyhydroxystyrene, polystyrenesulfonic acid, hydroxystyrene-(meth)acrylate ester copolymers, styrene-(meth)acrylate ester copolymers and styrene-hydroxystyrene-(meth)acrylate ester copolymers; poly(meth)acrylic acid derivatives such as poly(meth)acrylic acid, poly(meth)acrylamide, polyacrylonitrile, polyethyl (meth)acrylate and polybutyl (meth)acrylate; polyethers such as polyethylene glycol, poly(methyl vinyl ether), poly(ethyl vinyl ether), poly(butyl vinyl ether) and poly(isobutyl vinyl ether), and derivatives thereof; cellulose and cellulose derivatives such as methyl cellulose, cellulose acetate, cellulose nitrate, hydroxymethyl cellulose, hydroxyethyl cellulose, hydroxypropyl cellulose and carboxymethyl cellulose; polyvinyl acetate derivatives such as polyvinyl alcohol, polyvinyl butyral, polyvinyl formal and polyvinyl acetate; nitrogen-containing polymer derivatives such as polyvinyl pyridine, polyvinyl pyrrolidone, polyethyleneimine and poly-2-methyl-2-oxazoline; and polyvinyl halide derivatives such as polyvinyl chloride and polyvinylidene chloride. These may be used singly or two or more may be used in combination.

The emulsifying agents (surfactants) are exemplified by anionic emulsifying agents, including alkyl sulfates such as sodium dodecyl sulfate, alkylbenzenesulfonates such as sodium dodecylbenzenesulfonate, alkylnaphthalenesulfonates, fatty acid salts, alkyl phosphates and alkyl sulfosuccinates; cationic emulsifying agents such as alkylamine salts, quaternary ammonium salts, alkyl betaines and amine oxides; and nonionic emulsifying agents such as polyoxyethylene alkyl ethers, polyoxyethylene alkyl allyl ethers, polyoxyethylene alkyl phenyl ethers, sorbitan fatty acid esters, glycerol fatty acid esters, sucrose fatty acid esters and polyoxyethylene fatty acid esters. These may be used singly or two or more may be used in combination.

By using these dispersants, stabilizers, emulsifying agents and the like, the length, breadth, thickness and other properties of oblate ellipsoidal polymer particle A can be more stably controlled.

Depending on, for example, the intended use of the resulting particles, a catalyst (reaction accelerator) may be included at the time of the polymerization reaction. The content thereof may be set to a suitable amount that does not adversely affect the particle properties, such as from 0.01 to 20 wt% of the total weight of the unsaturated monomer.

The catalyst is not particularly limited so long as it is a positive catalyst, and may be suitably selected from among known catalysts and used. Examples include tertiary amines such as benzyldimethylamine, triethylamine, tributylamine, pyridine and triphenylamine; quaternary ammonium compounds such as triethylbenzylammonium chloride and tetramethylammonium chloride; phosphines such as triphenylphosphine and tricyclophosphine; phosphonium compounds such as benzyl trimethylphosphonium chloride; imidazole compounds such as 2-methylimidazole and 2-methyl-4-ethylimidazole; alkali metal hydroxides such as potassium hydroxide, sodium hydroxide and lithium hydroxide; alkali metal carbonates such as sodium carbonate and lithium carbonate; alkali metal salts of organic acids; and halides that exhibit Lewis acid properties, such as boron trichloride, boron trifluoride, tin tetrachloride and titanium tetrachloride, or complex salts thereof. These may be used singly or two or more may be used in combination.

At the time of the polymerization reaction, in order to, for example, adjust the size, shape and quality of the resulting oblate ellipsoidal polymer particle A, it is also possible to add a compound that is soluble in water or another polar solvent and undergoes electrolytic dissociation into cations and anions, such that the solution exhibits electrical conductivity. Examples include salts, inorganic acids, inorganic bases, organic acids, organic bases and ionic liquids. The content thereof is a suitable amount that does not adversely affect the particle properties, and may be set to, for example, from 0.01 to 80 wt% of the collective weight of the polymerization ingredients.

[Polymer Particle B]

The polymer particles in the skin cosmetic may optionally include a polymer particle B having a different shape from that of oblate ellipsoidal polymer particle A. Here, "different shape" includes also particles of a shape or size differing from that of oblate ellipsoidal polymer particle A, although a different shape that takes into account the ability to stably obtain optical properties such as a UV scattering effect, light scattering ability in the visible light region and light reflectivity is desirable. Examples of such shapes include spherical, spheroidal, ellipsoidal, pulverized, rod-like, polyhedral, rough and blocky shapes. A spheroidal shape refers here to a shape that is ellipsoidal and has an aspect ratio of less than 2.

According to studies by the inventors, oblate ellipsoidal polymer particle A has, owing to its shape, excellent optical properties compared to conventional spherical or spheroidal particles of the same composition. However, due to its flowability during the production or formulation of molded products and compositions, this particle tends to orient itself in the direction of flow, in which case the optical properties may not be stably obtained. In such cases, the addition of polymer particle B imparts a sterically hindering effect, enabling the anisotropic characteristics to be stably controlled. It is therefore possible, with polymer particle B, to maintain stable optical properties without adversely affecting the characteristics of oblate ellipsoidal polymer particle A. From this standpoint, and also to adjust the slip characteristics, it is preferable for polymer particle B to be spherical, spheroidal or ellipsoidal.

The polymer particle B has a volume mean particle size ($MV_B$) which is the same as or less than $L_{AV}$ of oblate ellipsoidal polymer particle A, more preferably satisfies the condition $1/5 \times D_{AV} \leq MV_B \leq L_{AV}$, even more preferably satisfies the condition $1/3 \times D_{AV} \leq MV_B \leq 0.8 \times L_{AV}$, still more preferably satisfies the condition $1/2 \times D_{AV} \leq MV_B \leq 0.6 \times L_{AV}$, and most preferably satisfies the condition $D_{AV} \leq MV_B \leq 1/2 \times L_{AV}$. When $MV_B$ is the same as or less than $L_{AV}$, sufficient steric hindrance can be imparted, enabling the optical properties of oblate ellipsoidal polymer particle A to be stably obtained.

From the standpoint of production efficiency, the polymer making up polymer particle B is preferably the same as the polymer making up oblate ellipsoidal polymer particle A or at least includes the same ingredients. When improvement in the light-diffusing ability within the visible light region is also a consideration, it is effective to give polymer particles A and B ingredient compositions that have different refractive indices.

Polymer particle B may be a mixture of two or more types, so long as it satisfies the above conditions relating to $MV_B$.

To further enhance the UV scattering effect and visible light scattering effect, it is preferable for either or both of oblate ellipsoidal polymer particle A and polymer particle B to possess at least one of the following characteristics: fine irregularities at the particle surface, a high porosity, and a relatively large specific surface area.

Either or both of oblate ellipsoidal polymer particle A and polymer particle B may be a composite particle having a core-shell structure, or a composite particle obtained by the physical or chemical addition of other fine particles. Examples of methods for producing composite particles include (1) incorporating other fine particles at the time of parent particle production, (2) using the polarity of ionic functional groups present at the surface of the parent particles following parent particle production to add other fine particles, and (3) chemical methods such as addition polymerization, polycondensation, addition condensation and seed polymerization.

As used herein, "other fine particles" refers to organic or inorganic particles which are smaller than oblate ellipsoidal polymer particle A and particle B serving as the parent particles. The preferred particle size of the other fine particles varies according to the size of the parent particles, but is generally in the range of about 0.005 to about 50 μm.

Such organic particles are exemplified by particles composed of polymerizable monomers that may be used in polymer particle production, curable particles, and organic pigments.

The inorganic particles are exemplified by metals, metal oxides, hydrated metal oxides and inorganic pigments, such as copper powder, iron powder, gold powder, aluminum oxide, titanium oxide, zinc oxide, silicon oxide, tin oxide, copper oxide, iron oxide, magnesium oxide, manganese oxide, calcium carbonate, magnesium hydroxide and aluminum hydroxide.

These fine particles are exemplified by commercial products that may be used directly as is or may be used following surface modification with a surface treatment agent such as a coupling agent.

In particular, metal oxide fine particles having a particle size of 0.005 to 10 μm, especially titanium oxide, zinc oxide or silicon oxide, may be added to oblate ellipsoidal polymer particle A and polymer particle B in order to control the refractive index and improve the UV scattering effect. These may be used singly or two or more may be used in combination.

These metal oxide fine particles can be incorporated by, during production of the polymer particles, adding from 0.1 to 50 wt % of the fine particles, based on the total amount of the polymerization ingredients, and physically/chemically adsorbing or bonding the fine particles within the resulting polymer particles. By thus including a suitable amount of inorganic particles in or coating a suitable amount of inorganic particles on the polymer fine particles to form a composite, it is possible to further enhance the UV scattering effects while retaining gloss.

Also, an active material may be physically, mechanically or chemically bonded, as appropriate, to the oblate ellipsoidal polymer particle A or polymer particle B. Known techniques may be employed for such physical, mechanical or chemical bonding. By way of illustration, examples of surface treatments that are widely used on powders and the like include silicone compound treatment, fluorine compound treatment, amino acid compound treatment, fatty acid compound treatment, and organic coupling treatments using silane compounds or titanate compounds. In the case of ingredients that dissolve, use can be made of techniques that cause the ingredient to be absorbed/adsorbed to the particle interior or onto a surface layer; use can be made of coloring techniques with dyes or the like; and use can be made of known chemical bonding techniques that induce adsorption by chemically bonding reactive groups present at the particle interior or on a surface layer with reactive groups on the active material.

Here, "reactive groups" refers to polymerizable unsaturated bond-containing groups such as $\alpha,\beta$-unsaturated carbonyl groups, $\alpha,\beta$-unsaturated nitrile groups, halovinyl groups, halovinylidene groups, aromatic vinyl groups, heterocyclic vinyl groups, conjugated dienes and vinyl carboxylates; and also carboxyl, carbonyl, epoxy, isocyanate, hydroxyl, amide, cyano, amino, epoxy, chloromethyl, glycidyl ether, lithio, ester, formyl, nitrile, nitro, carbodiimide and oxazoline groups.

The method for producing polymer particle B is not particularly limited, provided that it is a method capable of obtaining particles having the above-described shape, such as grinding or solution polymerization. Solution polymerization is exemplified by the above-described method.

According to studies by the inventors, when polymer particle B has the same ingredients as oblate ellipsoidal polymer particle A, suitable production of the desired mixed particles can be easily carried out by varying the types and relative amounts of the monomers and solvents, and also the relative amounts of dispersants and emulsifying agents that are used as needed.

In the skin cosmetic of the invention, the content of polymer particles is from 0.1 to 50 wt%. The lower limit is preferably 0.2 wt%, more preferably 0.3 wt%, and more preferably 0.5 wt%. The upper limit is preferably 30 wt%, more preferably 20 wt%, and even more preferably 15 wt%. The polymer particle content may be suitably adjusted within this range according to the intended use and desired qualities of the skin cosmetic, such as the light scattering properties, including the UV scattering effect and shading effect, as well as the flow properties, moldability, improved adherence and finished look.

When the polymer particles include both oblate ellipsoidal polymer particle A and polymer particle B, the mixing ratio (weight ratio) of the oblate ellipsoidal polymer particle A to polymer particle B is preferably from 99:1 to 10:90, more preferably from 98:2 to 15:85, even more preferably from 97:3 to 20:80, and most preferably from 95:5 to 30:70. At a mixing ratio in this range, the optical properties possessed by the oblate ellipsoidal polymer particle A can be stably obtained.

[Other Ingredients]

Ingredients which are typically used in cosmetic preparations may be suitably used as the remaining ingredients other than the above polymer particles included in the skin cosmetic of the invention (such ingredients are referred to below as "other ingredients"). Such other ingredients are included in an amount of from 50 to 99.9 wt% of the skin cosmetic of the invention.

[Inorganic Particles]

The skin cosmetic of the invention preferably includes, as another ingredient, inorganic particles in order to suitably adjust, for example, adherence to the skin, spreadability, gloss, coloration and hiding ability. The inorganic particles are exemplified by inorganic particles of various sizes and shapes, including mica, talc, kaolin, sericite, montmorillonite, kaolinite, mica, muscovite, phlogopite, synthetic mica, ruby mica, biotite, vermiculite, magnesium carbonate, calcium carbonate, aluminum silicate, barium silicate, calcium silicate, magnesium silicate, strontium silicate, metal salts of tungstic acid, magnesium, zeolite, barium sulfate, calcined calcium sulfate, calcium phosphate, fluorapatite, hydroxyapatite, ceramic powder, bentonite, smectite, clay, mud, metal soaps (e.g., zinc myristate, calcium palmitate, aluminum stearate), red iron oxide, yellow iron oxide, black iron oxide, ultramarine, prussian blue, carbon black, titanium oxide, finely divided and very finely divided titanium oxide, zinc oxide, finely divided and very finely divided zinc oxide, alumina, silica, fumed silica (very finely divided silicic anhydride), titanium mica, fish scale guanine, boron nitride, photochromic pigments, synthetic fluorophlogopite, gold, aluminum, and finely divided composite powder, as well as hydrophobized or hydrophilized inorganic particles obtained by treating any of the foregoing with various types of surface treatment agents, including silicones such as hydrogensilicones or cyclic hydrogensilicones, other silane compounds and titanium coupling agents.

The shape of the inorganic particles is exemplified by, without particular limitation, spherical, spheroidal, platy, flaky, pulverized, rod-like, polyhedral, rough, and block-like shapes. Of these, from the standpoint of improving adherence, a platy or flaky shape is preferred.

The inorganic particles have a volume mean particle size which is preferably from 0.01 to 300 μm, and more preferably from 0.1 to 200 μm. At a volume mean particle size in this range, a smooth spreadability can be obtained during application, in addition to which the various powders give rise to steric hindrance on the skin, improving the soft focus effect. At a volume mean particle size in excess of 300 μm, grittiness arises, worsening the sensation upon use.

The content of the inorganic particles is preferably from 1.0 to 99.9 wt%, and more preferably from 5.0 to 95.0 wt%, of the other ingredients. When the amount of inorganic particles included is within this range, a sufficient cosmetic effect is obtained. At an amount in excess of 99.0 wt%, the excess powder ingredients make the finish powdery and give rise to squeakiness. The inorganic particles may be of one type used alone, or two or more types may be used in combination.

[Oily Ingredient]

The skin care treatment of the invention preferably includes, as another ingredient, an oily ingredient. Exemplary oily ingredients include higher alcohols, higher fatty acids, fatty oils, waxes, hydrocarbons, silicones and fatty acid esters.

Specific examples of oily ingredients include higher alcohols such as cetanol, myristyl alcohol, oleyl alcohol, lauryl alcohol, cetostearyl alcohol, stearyl alcohol, arachyl alcohol, behenyl alcohol, jojoba alcohol, chimyl alcohol, selachyl alcohol, batyl alcohol, hexyl decanol, isostearyl alcohol, 2-octyl dodecanol and dimer diol; aralkyl alcohols such as benzyl alcohol, and derivatives thereof; higher fatty acids such as lauric acid, myristic acid, palmitic acid, stearic acid, isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteiso-heneicosanoic acid, branched long-chain fatty acids, dimer acid and hydrogenated dimer acid, as well as metal salts thereof, such as aluminum salts, calcium salts, magnesium salts, zinc salts and potassium salts, and amides and other nitrogen-containing derivatives thereof; hydrocarbons such as liquid paraffins (mineral oils), heavy (liquid) isoparaffins, light (liquid) isoparaffins, α-olefin oligomers, polyisobutene (polyisobutylene), hydrogenated polyisobutene, polybutene, squalane, olive-derived squalane, squalene, vaseline and solid paraffins; waxes such as candelilla wax, carnauba wax, rice wax, japan wax, beeswax, montan wax, ozokerite, ceresin, paraffin wax, microcrystalline wax, petrolatum, Fischer-Tropsch waxes, polyethylene waxes and ethylene-propylene copolymers; vegetable oils and fats such as coconut oil, palm oil, palm kernel oil, safflower oil, olive oil, castor oil, avocado oil, sesame oil, teaseed oil, evening primrose oil, camellia oil, wheat germ oil, macadamia nut oil, hazelnut oil, candlenut oil, rosehip oil, meadowfoam oil, persic oil, tea tree oil, mentha oil, corn oil, rapeseed oil, sunflower oil, wheat germ oil, linseed oil, cottonseed oil, soybean oil, peanut oil, rice bran oil, cocoa butter, shea butter, hydrogenated coconut oil, hydrogenated castor oil, jojoba oil and hydrogenated jojoba oil; animal oils and fats such as beef tallow, milk fat, horse fat, egg yolk oil, mink oil and turtle oil; animal waxes such as spermaceti, lanolin and orange roughy oil; lanolins such as liquid lanolin, reduced lanolin, adsorption refined lanolin, acetylated lanolin, acetylated liquid lanolin, hydroxylated lanolin, polyoxyethylene lanolin, lanolin fatty acids, hard lanolin fatty acids, lanolin alcohols, acetylated lanolin alcohols and acetic acid (cetyl/lanolyl) esters; phospholipids such as lecithin, phosphatidylcholine, phosphatidylethanolamine, phosphatidylserine, phosphatidylglycerol, phosphatidylinositol, sphingophospholipids such as sphingomyelin, phosphatidic acid and lysolecithin; phospholipid derivatives such as hydrogenated soybean phospholipids, partially hydrogenated soybean phospholipids, hydrogenated egg yolk phospholipids and partially hydrogenated egg yolk phospholipids; sterols such as cholesterol, dihydrocholesterol, lanosterol, dihydrolanosterol, phytosterol and cholic acid; sapogenins; saponins; sterol esters such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, alkyl esters of acylsarcosines such as isopropyl N-lauroyl sarcosinate, cholesteryl 12-hydroxystearate, cholesteryl macadamiate, phytosteryl macadamiate, phytosteryl isostearate, cholesteryl esters of soft lanolin fatty acids, cholesteryl esters of hard lanolin fatty acids, cholesteryl esters of branched long-chain fatty acids and cholesteryl esters of long-chain α-hydroxy fatty acids; lipid complexes such as phospholipid/cholesterol complexes and phospholipid/phytosterol complexes; carboxylic acid esters of monoalcohols such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl esters of lanolin fatty acids, hexyldecyl dimethyloctanoate, octyldodecyl erucate, hydrogenated castor oil isostearate, ethyl oleate, ethyl avocadoate, isopropyl myristate, isopropyl palmitate, octyl palmitate, isopropyl isostearate, 2-ethylhexyl hydroxystearate, isopropyl lanolate, diethyl sebacate, diisopropyl sebacate, dioctyl sebacate, diisopropyl adipate, dibutyloctyl sebacate, diisobutyl adipate, dioctyl succinate and triethyl citrate; hydroxy acid esters such as cetyl lactate, diisostearyl malate and hydrogenated castor oil monoisostearate; polyhydric alcohol fatty acid esters such as glyceryl trioctanoate, glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate/eicosadioate, trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, pentaerythrityl hydrogenated rosin, ditrimethylolpropane triethylhexanoate, ditrimethylolpropane (isostearate/sebacate), pentaerythrityl triethylhexanoate, dipentaerythrityl (hydroxystearate/stearate/rosinate), diglyceryl diisostearate, sucrose tetraisostearate, polyglyceryl tetraisostearate, polyglyceryl-10 nonaisostearate, polyglyceryl-8 deca(erucate/isostearate/ricinoleate), diglyceryl (hexyldecanoate/sebacate) oligoester and glycol distearate (ethylene glycol distearate); derivatives of dimer acids and dimer diols, such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensates, hydrogenated castor oil dimer dilinoleate and hydroxyalkyl dimer dilinoleyl ethers; fatty acid alkanolamides such as cocamide monoethanolamide (cocamide MEA), cocamide diethanolamide (cocamide DEA), lauramide monoethanolamide (lauramide MEA), lauramide diethanolamide (lauramide DEA), lauramide monoisopropanolamide (lauramide MIPA), palmitamide monoethanolamide (palmitamide MEA), palmitamide diethanolamide (palmitamide DEA) and cocamide methyl ethanolamide (cocamide methyl MEA); various types of silicones, including methicone (monomethyl polysiloxane), dimethicone (dimethyl polysiloxane), highly polymerized dimethicone (highly polymerized dimethyl polysiloxane), methyl phenyl polysiloxane, cyclomethicone (octamethyl cyclotetrasiloxane), decamethyl cyclopentasiloxane, tetrahydrotetramethyl cyclotetrasiloxane, methyl cyclopolysiloxane, cyclopentasiloxane and dodecamethylcyclohexasiloxane; and fluorinated oils such as perfluorodecane, perfluorooctane and perfluoropolyether.

In particular, to further enhance the properties of oblate ellipsoidal polymer particle A and also from the standpoint of, for example, the moisturizing properties possessed by oblate ellipsoidal polymer particle A in foundation and other cosmetics applied directly to the skin and in cosmetics to which adherence, long-lasting performance and stability are to be imparted, the content of such oily ingredients is preferably at least 0.1 wt%, more preferably at least 1.0 wt%, and even more preferably at least 3.0 wt%, of the other ingredients. The upper limit is preferably 90 wt%, more preferably 80 wt%, and even more preferably 70 wt%. Examples of especially preferred oily ingredients include higher alcohols, hydrocarbons, vegetable oils and fats, and various types of silicones. The oily ingredient may be of one type used alone, or two or more types may be used in combination.

Other ingredients that may be added include powders such as organic powders; inorganic salts; perfumes; colors, colorants, dyes and pigments; various types of surfactants such as nonionic surfactants, anionic surfactants, cationic surfactants and amphoteric surfactants; alcohols such as lower alcohols, polyhydric alcohols, sugars and sterols; solvents; propellants; polymers; thickeners; gelling agents; ultraviolet absorbers; antioxidants; reducing agents; oxidizing agents; moisturizers; texture improvers; sequestrants; preservatives; antimicrobial agents; animal and plant extracts; pH adjustors; acids and alkalis; whitening agents; vitamins and derivatives thereof; antiphlogistics and anti-inflammatory agents; hair growth-promoting agents; blood circulation promoters; stimulants; hormones; anti-wrinkle agents; anti-aging agents; skin-firming agents; cooling agents; warming agents; wound-healing promoters; irritation relievers; analgesics; cell activators; antipruritics; exfoliates and keratolytic agents; antiperspirants; algefacients; astringents; enzymes; nucleic acids; and water. These ingredients may be suitably included in ranges that do not detract from the advantageous effects of the invention. These ingredients may be of one type used alone, or two or more types may be used in combination.

Specific examples of these ingredients are given below. Preferred examples of organic powders include organic powders of various sizes and shapes, such as starch, cellulose, lauroyl lysine, silk powder, nylon powder and fibers, polyamide powder and fibers, polyester powder and fibers, polyethylene powder and fibers, polypropylene powder and fibers, poly(methyl methacrylate)/polyester laminated powder (acrylic powder and fibers), polystyrene powder and fibers, styrene-acrylic acid copolymer resin powder, vinyl resin powder, urea resin powder, fluororesin powder, acrylic resin powder, epoxy resin powder, silicone resin powder, benzoguanamine resin powder, polyethylene terephthalate/polymethyl methacrylate laminated powder, polyethylene terephthlate/aluminum/epoxy laminated powder, urethane powder, silicone powder, Teflon® powder, polylactic acid, bioplastics, and biodegradable polymer powders and fibers that are completely or partially biodegradable, such as (modified) polyvinyl alcohols, as well as surface-treated powders and organic-inorganic composite powders of the above.

Preferred examples of inorganic salts include sodium chloride-containing salts such as common salt (sodium chloride), refined solar salt, rock salt, sea salt and native salt; potassium chloride, aluminum chloride, calcium chloride, magnesium chloride, bittern, zinc chloride and ammonium chloride; sodium sulfate, aluminum sulfate, aluminum potassium sulfate (alum), aluminum ammonium sulfate, barium sulfate, calcium sulfate, potassium sulfate, magnesium sulfate, zinc sulfate, iron sulfate and copper sulfate; sodium phosphates such as sodium dihydrogen phosphate, disodium hydrogen phosphate and trisodium phosphate; potassium phosphates, calcium phosphates and magnesium phosphates.

Preferred examples of perfumes include synthetic and natural perfumes, as well as various blended perfumes, such as acetyl cedrene, amyl cinnamaldehyde, allyl amyl glycolate, β-ionone, Iso E Super, isobutyl quinoline, iris oil, irone, indole, ylang ylang oil, undecanal, undecenal, γ-undecalactone, estragole, eugenol, oakmoss, opoponax resinoid, orange oil, aurantiol, galaxolide, carvacrol, L-carvone, camphor, canon, carrot seed oil, clove oil, methyl cinnamate, geraniol, geranyl nitrile, isobornyl acetate, geranyl acetate, dimethyl benzyl carbinyl acetate, styrallyl acetate, cedryl acetate, terpinyl acetate, p-tert-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, thymol, tuberose absolute, decanal, decalactone, terpineol, γ-terpinene, triplal, nerol, nonanal, 2,6-nonadienol, nonalactone, patchouli alcohol, vanilla absolute, vanillin, basil oil, patchouli oil, hydroxycitronellal, α-pinene, piperitone, phenethyl alcohol, phenyl acetaldehyde, petigrain oil, hexyl cinnamaldehyde, cis-3-hexenol, peru balsam, vetiver oil, vetiverol, peppermint oil, pepper oil, heliotropin, bergamot oil, benzyl benzoate, borneol, myrrh resinoid, musk ketone, methyl nonyl acetaldehyde, γ-methyl ionone, menthol, L-menthol, L-menthone, eucalyptus oil, β-ionone, lime oil, lavender oil, D-limonene, linalool, lyral, lilial, lemon oil, rose absolute, rose oxide, rose oil, rosemary oil and various essential oils.

Preferred examples of colors, coloring agents, dyes and pigments include certified colors such as Brown No. 201, Black No. 401, Violet No. 201, Violet No. 401, Blue No. 1, Blue No. 2, Blue No. 201, Blue No. 202, Blue No. 203, Blue No. 204, Blue No. 205, Blue No. 403, Blue No. 404, Green No. 201, Green No. 202, Green No. 204, Green No. 205, Green No. 3, Green No. 401, Green No. 402, Red No. 102, Red No. 104-1, Red No. 105-1, Red No. 106, Red No. 2, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Red No. 228, Red No. 230-1, Red No. 230-2, Red No. 231, Red No. 232, Red No. 3, Red No. 401, Red No. 404, Red No. 405, Red No. 501, Red No. 502, Red No. 503, Red No. 504, Red No. 505, Red No. 506, Orange No. 201, Orange No. 203, Orange No. 204, Orange No. 205, Orange No. 206, Orange No. 207, Orange No. 401, Orange No. 402, Orange No. 403, Yellow No. 201, Yellow No. 202-1, Yellow No. 202-2, Yellow No. 203, Yellow No. 204, Yellow No. 205, Yellow No. 4, Yellow No. 401, Yellow No. 402, Yellow No. 403-1, Yellow No. 404, Yellow No. 405, Yellow No. 406, Yellow No. 407 and Yellow No. 5; other acid dyes such as Acid Red 14; basic dyes such as Arianor Sienna Brown, Arianor Madder Red, Arianor Steel Blue and Arianor Straw Yellow; nitro dyes such as HC Yellow 2, HC Yellow 5, HC Red 3, 4-hydroxypropylamino-3-nitrophenol, N,N'-bis(2-hydroxyethyl)-2-nitro-p-phenylenediamine, HC Blue 2 and Basic Blue 26; disperse dyes; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher; inorganic black pigments such as black iron oxide and titanium suboxide; inorganic violet pigments such as mango violet and cobalt violet; inorganic green pigments such as chromium oxide, chromium hydroxide, and cobalt titanate; inorganic blue pigments such as ultramarine and prussian blue; pearlescent pigments such as titanium oxide-coated mica, titanium oxide-coated bismuth oxychloride, titanium oxide-coated talc, colored titanium oxide-coated mica, bismuth oxychloride and fish scale guanine; metal powder pigments such as aluminum powder, copper powder and gold; surface-treated inorganic and metal powder pigments such as silicone-coated pigments and hydrophobized pigments; organic pigments such as zirconium, barium or aluminum lakes of Red No. 201, Red No. 202, Red No. 204, Red No. 205, Red. No. 220, Red No. 226, Red No. 228, Red No. 405, Orange No. 203, Orange No. 204, Yellow No. 205, Yellow No. 401, Blue No. 404, Red No. 3, Red No. 104, Red No. 106, Red No. 227, Red No. 230, Red No. 401, Red No. 505, Orange No. 205, Yellow No. 4, Yellow No 5, Yellow No. 202, Yellow No. 203, Green No. 3 and Blue No. 1; hydrophobized, hydrophilized and other surface-treated organic pigments; natural colors and dyes, including anthraquinones such as astaxanthin and alizarin, naphthoquinones such as anthocyanidin, β-carotene, catechol, capsanthin, chalcone, carthamin, quercetin, crocin, chlorophyll, curcumin, cochineal and shikonin, bixin, flavones, betacyanidine, henna, hemoglobin, lycopene, riboflavin and rutin; oxidative dye intermediates and couplers such as p-phenylenediamine, toluene-2,5-diamine, o-, m- and p-aminophenols, m-phenylenediamine, 5-amino-2-methylphenol, resorcinol, 1-naphthol and 2,6-diaminopyridine, as well as salts thereof; autoxidative dyes such as indoline; and dihydroxyacetone.

Examples of surfactants include anionic surfactants, nonionic surfactants, cationic surfactants, amphoteric surfactants and polymeric surfactants.

Preferred examples of anionic surfactants include fatty acid salts such as potassium laurate and potassium myristate; alkyl sulfates such as sodium lauryl sulfate, triethanolamine lauryl sulfate and ammonium lauryl sulfate; polyoxyethylene alkyl sulfates such as sodium laureth sulfate and triethanolamine laureth sulfate; acyl N-methylamino acid salts such as sodium cocoyl methyl taurate, potassium cocoyl methyl taurate, sodium lauroyl methyl taurate, sodium myristoyl methyl taurate, sodium lauroyl methyl alaninate, sodium lauroyl sarcosinate, triethanolamine lauroyl sarcosinate and sodium lauroyl glutamate methyl alaninate; acyl amino acid salts such as sodium cocoyl glutamate, triethanolamine cocoyl glutamate, sodium lauroyl glutamate, sodium myristoyl glutamate, sodium stearoyl glutamate, ditriethanolamine palmitoyl aspartate and triethanolamine cocoyl alaninate; polyoxyethylene alkyl ether acetates such as sodium laureth acetate; succinates such as sodium lauroyl monoethanolamide succinate; fatty acid alkanolamide ether carboxylates; acyl lactates; polyoxyethylene fatty amine sulfates; fatty acid alkanolamide sulfates; fatty acid glyceride sulfates such as sodium hydrogenated cocoglyceride sulfate; alkylbenzene polyoxyethylene sulfates; olefin sulfonates such as sodium $\alpha$-olefin sulfonates; alkyl sulfosuccinates such as disodium lauryl sulfosuccinate and sodium dioctyl sulfosuccinate; alkyl ether sulfosuccinates such as disodium laureth sulfosuccinate, sodium monolauroyl monoethanolamide polyoxyethylene sulfosuccinate and sodium lauryl polypropylene glycol sulfosuccinate; alkylbenzene sulfonates such as sodium tetradecylbenzene sulfonate and triethanolamine tetradecylbenzene sulfonate; alkyl naphthalene sulfonates; alkane sulfonates; $\alpha$-sulfo fatty acid methyl esters; acyl isethionates; alkyl glycidyl ether sulfonates; alkyl sulfoacetates; alkyl ether phosphates such as sodium laureth phosphate, sodium dilaureth phosphate, sodium trilaureth phosphate and sodium monooleth phosphate; alkyl phosphates such as potassium lauryl phosphate; sodium caseinate; alkyl aryl ether phosphates; fatty acid amide ether phosphates; phospholipids such as phosphatidylglycerol, phosphatidylinositol, and phosphatidic acid; and silicone-based anionic surfactants such as carboxylic acid-modified silicones, phosphoric acid-modified silicones and sulfuric acid-modified silicones.

Preferred examples of nonionic surfactants include polyoxyethylene lauryl ether, polyoxyethylene myristyl ether, polyoxyethylene cetyl ether, polyoxyethylene isocetyl ether, polyoxyethylene cetostearyl ether, polyoxyethylene isostearyl ether, polyoxyethylene oleyl cetyl ether, polyoxyethylene oleyl ether, polyoxyethylene behenyl ether, polyoxyethylene tridecyl ether, polyoxyethylene octyl dodecyl ether, polyoxyethylene butyl ether, polyoxyethylene $C_{12-14}$ alkyl ether, polyoxyethylene octyl phenyl ether, polyoxyethylene dinonyl phenyl ether, polyoxyethylene nonyl phenyl ether, polyoxyethylene cholesteryl ether, polyoxyethylene cholestanol ether, polyoxyethylene phytosterol, polyoxyethylene lanolin alcohol, polyoxyethylene lanolin alcohol acetate, polyoxyethylene lanolin, polyoxyethylene liquid lanolin, polyoxyethylene reduced lanolin, polyoxyethylene polyoxypropylene liquid lanolin, polyethylene glycol octanoate/polyoxyethylene nonyl phenyl ether mixture, polyoxyethylene hydrogenated castor oil, polyoxyethylene castor oil, polyoxyethylene hydrogenated castor oil monostearate, polyoxypropylene glyceryl ether, polyoxypropylene diglyceryl ether, polyoxypropylene sorbitol, polyoxypropylene cetyl ether, polyoxypropylene stearyl ether, polyoxypropylene lanolin alcohol ether, polyoxypropylene hydrogenated lanoline, polyoxyethylene polyoxypropylene lanoline, polyoxyethylene polyoxypropylene glyceryl ether, polyoxyethylene polyoxypropylene lauryl ether, polyoxyethylene polyoxypropylene cetyl ether, polyoxyethylene polyoxypropylene stearyl ether, ethylenediamine tetra(polyoxyethylene propylene), sorbitan monolaurate, sorbitan monopalmitate, sorbitan monostearate, sorbitan monoisostearate, sorbitan monooleate, sorbitan cocoate, sorbitan sesquiisostearate, sorbitan sesquioleate, sorbitan distearate, sorbitan sesquistearate, sorbitan tristearate, sorbitan trioleate, sorbitan polyoxyethylene monolaurate, sorbitan polyoxyethylene monopalmitate, sorbitan polyoxyethylene monostearate, sorbitan polyoxyethylene monooleate, sorbitan polyoxyethylene tristearate, sorbitan polyoxyethylene trioleate, sorbitan polyoxyethylene tetraoleate, $C_{8-16}$ alkylglucosides, methylglucoside sesquistearate, polyoxyethylene methylglucoside oleate, polyoxyethylene methylglucoside sesquistearate, polyoxyethylene methylglucoside distearate, polyoxypropylene methylglucoside, polyoxyethylene glucoside distearate, ethylene glycol monostearate, ethylene glycol distearate, ethylene glycol dilaurate, propylene glycol laurate, self-emulsifying propylene glycol stearate, propylene glycol isostearate, propylene glycol oleate, propylene glycol ricinoleate, propylene glycol di(caprylate/caprate), propylene glycol distearate, propylene glycol diisostearate, propylene glycol dioleate, polyethylene glycol monolaurate, polyethylene glycol monopalmitate, polyethylene glycol monostearate, polyethylene glycol monoisostearate, polyethylene glycol monooleate, polyethylene glycol dilaurate, polyethylene glycol distearate, polyethylene glycol diisostearate, polyethylene glycol dioleate, polyethylene glycol lanolate, glyceryl laurate, glyceryl myristate, glyceryl palmitate, lipophilic monostearyl glycerol, self-emulsifying monostearyl glycerol, glyceryl isostearate, glyceryl oxystearate, glyceryl oleate, lipophilic glyceryl oleate, glyceryl ricinoleate, glyceryl behenate, glyceryl erucate, glyceryl cocoate, glyceryl sesquioleate, glyceryl diisostearate, glyceryl triisopalmitate, hydrogenated glyceryl soyate, glyceryl trilaurate, glyceryl acetate monostearate, glyceryl acetate ricinoleate, polyglyceryl monostearate, polyglyceryl monooleate, polyglyceryl distearate, polyglyceryl diisostearate, polyglyceryl dioleate, condensed polyglyceryl ricinoleate, polyoxyethylene glyceryl laurate, polyoxyethylene glyceryl monostearate, polyoxyethylene glyceryl oleate, polyoxyethylene glyceryl cocoate, polyoxyethylene glyceryl trioleate, polyoxyethylene hydrogenated castor oil laurate, polyoxyethylene myristyl ether myristate, polyethylene cetyl ether stearate, polyoxyethylene stearyl ether stearate, polyoxyethylene hydrogenated castor oil isostearate, polyoxyethylene hydrogenated castor oil triisostearate, polyoxyethylene beef tallow alkyl hydroxymyristylene ether, polyoxyethylene $C_{12-15}$ alkyl ether propyl acetate, polyoxyethylene $C_{12-15}$ alkyl ether hexadecyl acetate, polyoxyethylene trimethylolpropane distearate, polyoxyethylene trimethylolpropane tristearate, polyoxyethylene polyoxypropylene trimethylolpropane, diglycerin oleyl ether, isostearic acid glyceryl ether, batyl alcohol, palmitamide, stearamide, polyoxyethylene ricinoleamide, lauroyl ethanolamide, palmitoyl ethanolamide, stearoyl ethanolamide, cocoyl ethanolamide, polyoxyethylene lauroyl monoethanolamide, polyoxyethylene cocoyl monoethanolamide, lauroyl diethanolamide, lauroyl myristoyl diethanolamide, myristoyl diethanolamide, stearoyl diethanolamide, oleyl diethanolamide, isostearoyl diethanolamide, linoleoyl diethanolamide, cocoyl diethanolamide, palm kernel oil fatty acid diethanolamide, hydrogenated tallow oil diethanolamide, polyoxyethylene cocoyl diethanolamide, lauroyl isopropanolamide, oleyl isopropanolamide, stearoyl ethanolamide, lauryl dimethylamine oxide solution, lauryl dimethylamine oxide solution, myristyl dimethylamine oxide solution, stearyl dimethylamine oxide, cocoalkyl dimethylamine oxide solution, polyoxyethylene cocoalkyl dimethylamine oxide, dihydroxyethyl lauramine oxide solution, lauroyl dioctyldodecyl glutamate, stearoyl dioctyldodecyl glutamate, lauroyl glutamate polyoxyethylene octyldodecyl ether diester, lauroyl glutamate dipolyoxyethylene stearyl ether, polyoxyethylene hydrogenated castor oil pyroglutamate isostearate diester, polyoxyethylene glyceryl pyroglutamate isostearate diester, polyether-modified silicones such as dimethicone copolyol, polysiloxane-oxyalkylene copolymers such as polydimethylsiloxane (dimethicone)-polyoxyethylene copolymer, and silicone-based nonionic surfactants such as sugar-modified silicones.

Preferred examples of cationic surfactants include alkyl trimethylammonium chlorides such as behentrimonium chloride, steartrimonium chloride, cetrimonium chloride and laurtrimonium chloride; alkyl trimethylammonium bromides such as steartrimonium bromide; dialkyl dimethylammonium chlorides such as disteardimonium chloride and dicocodimonium chloride; fatty acid amide amines such as stearamidopropyl dimethylamine and stearamidoethyl diethylamine, as well as salts thereof; alkyl ether amines such as stearoxypropyl dimethylamine and salts or quaternary salts thereof; quaternary ammonium salts of fatty acid amides, such as branched long-chain ($C_{12-31}$) fatty acid aminopropylethyldimethylammonium ethyl sulfates and lanolin fatty acid aminopropylethyldimethylammonium ethyl sulfates; polyoxyethylene alkylamines and salts or quaternary salts thereof; alkylamine salts; fatty acid amide guanidine salts; alkyl ether ammonium salts; alkyl trialkylene glycol ammonium salts; benzalkonium salts; benzethonium salts; pyridinium salts such as cetylpyridinium chloride; imidazolinium salts; alkyl isoquinolinium salts; dialkyl morpholinium salts; polyamine fatty acid derivatives; and silicone-based cationic surfactants, including amino-modified silicones such as aminopropyl dimethicone and amodimethicone, cation-modified silicones, cation-modified and polyether-modified silicones and amino-modified and polyether-modified silicones.

Preferred examples of amphoteric surfactants include N-alkyl-N,N-dimethylamino acid betaines such as lauryl betaine (lauryl dimethylaminoacetic acid betaine); fatty acid amidoalkyl-N,N-dimethylamino acid betaines such as cocamidopropyl betaine and lauramidopropyl betaine; imidazoline betaines such as sodium cocoamphoacetate and sodium lauroamphoacetate; alkyl sulfobetaines such as alkyl dimethyltaurines; betaine sulfates such as alkyl dimethylaminoethanol sulfates; betaine phosphates such as alkyl dimethylaminoethanol phosphates; phospholipids such as phosphatidylcholine, phosphatidylethanolamine, phosphatidylserine, sphingophospholipids such as sphingomyelin, lysolecithin, hydrogenated soybean phospholipid, partially hydrogenated soybean phospholipid, hydrogenated egg yolk phospholipid, partially hydrogenated egg yolk phospholipid and hydroxylated lecithin; and silicone-based amphoteric surfactants.

Preferred examples of polymeric surfactants include polyvinyl alcohol, sodium alginate, starch derivatives, tragacanth gum, acrylic acid-alkyl methacrylate copolymers, and various silicone-based surfactants.

Preferred examples of alcohols, solvents and propellants include lower alcohols such as ethanol, 2-propanol (isopropyl alcohol), butanol and isobutyl alcohol; glycols such as propylene glycol, butylene glycol, diethylene glycol, dipropylene glycol and isopentyldiol; glycol ethers such as diethylene glycol monoethyl ether (ethoxydiglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether and dipropylene glycol monoethyl ether; glycol ether esters such as ethylene glycol monoethyl ether acetate, diethylene glycol monoethyl ether acetate and propylene glycol monoethyl ether acetate; glycol esters such as diethoxyethyl succinate and ethylene glycol disuccinate; benzyl alcohol, benzyloxyethanol, propylene carbonate, dialkyl carbonates, acetone, ethyl acetate, N-methylpyrrolidone, toluene, fluorocarbons and next-generation fluorocarbons, and propellants such as LPG, dimethyl ether and carbon dioxide gas.

Preferred examples of polymers (including synthetic polymers), thickeners and gelling agents include guar gum, locust bean gum, quince seed, carrageenan, galactan, gum arabic, tara gum, tamarind, furcellaran, karaya gum, *Abelmoschus manihot,* cara gum, tragacanth gum, pectin, pectic acid and salts thereof such as the sodium salt, alginic acid and salts thereof such as the sodium salt, and mannan; starches such as rice starch, corn starch, potato starch and wheat starch; xanthan gum, dextran, succinoglucan, curdlan, hyaluronic acid and salts thereof, xanthan gum, pullulan, gellan gum, chitin, chitosan, agar, brown algae extract, chondroitin sulfate, casein, collagen, gelatin, and albumin; cellulose and derivatives thereof, such as methyl cellulose, ethyl cellulose, hydroxyethyl cellulose, hydroxypropyl cellulose, hydroxypropyl methylcellulose, carboxymethyl cellulose and salts thereof such as the sodium salt, methylhydroxypropyl cellulose, sodium cellulose sulfate, dialkyldimethylammonium cellulose sulfate, crystalline cellulose and cellulose powder; starch derivatives such as soluble starch, starch polymers such as carboxymethyl starch, methylhydroxypropyl starch and methyl starch, starch hydroxypropyltrimonium chloride and aluminum corn starch octenylsuccinate; alginic acid derivatives such as sodium alginate and propylene glycol alginate; polyvinylpyrrolidone (PVP), polyvinyl alcohol (PVA), vinylpyrrolidone-vinyl alcohol copolymers and polyvinyl methyl ether; polyethylene glycol, polypropylene glycol and polyoxyethylene-polyoxypropylene copolymers; (meth)acrylate copolymers; amphoteric methacrylate ester copolymers such as (methacryloyloxyethylcarboxybetaine/alkyl methacrylate) copolymers and (acrylate/stearyl acrylate/ethylamine oxide methacrylate) copolymers; (dimethicone/vinyl dimethicone) crosspolymers, (alkyl acrylate/diacetone acrylamide) copolymers and (alkyl acrylate/diacetone acrylamide) copolymer AMP; partially saponified polyvinyl acetate and maleic acid copolymers; vinylpyrrolidone-dialkylaminoalkyl methacrylate copolymers; acrylic resin alkanolamines; polyesters and water-dispersible polyesters; polyacrylamides; copolymers of polyacrylate esters such as polyethyl acrylate, carboxyvinyl polymers, poly(meth)acrylic acid and salts thereof such as the sodium salt, and acrylic acid-methacrylate ester copolymers; acrylic acid-alkyl methacrylate copolymers; cationized celluloses such as polyquaternium-10, diallyldimethylammonium chloride-acrylamide copolymers such as polyquaternium-7, acrylic acid-diallyldimethylammonium chloride copolymers such as polyquaternium-22, acrylic acid-diallyldimethylammonium chloride-acrylamide copolymers such as polyquaternium-39, acrylic acid-cationized methacrylate ester copolymers, acrylic acid-cationized methacrylamide copolymers, acrylic acid-methyl acrylate-methacrylamidopropyltrimethylammonium chloride copolymers such as polyquaternium-47, and methacryloyl chloride choline ester polymers; cationized polysaccharides such as cationized oligosaccharides, cationized dextran and guar hydroxypropyltrimonium chloride; polyethyleneimines; cationic polymers; copolymers of 2-methacryloyloxyethyl phosphorylcholine and butyl methacrylate, such as polyquaternium-51; polymer emulsions such as acrylic resin emulsions, poly(ethyl acrylate) emulsions, poly(alkyl acrylate) emulsions, polyvinyl acetate resin emulsions, natural rubber latex and synthetic latex; nitrocellulose; polyurethanes and various copolymers thereof; various silicones such as trimethylsiloxysilicic acid, phenyl trimethicone, trimethyl pentaphenyl trisiloxane, diphenyl dimethicone, phenyl dimethicone, stearoxypropyl dimethylamine, (aminoethyl aminopropyl methicone/dimethicone) copolymer, dimethiconol, dimethiconol crosspolymer, silicone resins, silicone rubbers, amino-modified silicones such as aminopropyl dimethicone and amodimethicone, cation-modified silicones, polyether-modified silicones such as dimethicone polyol, polyglycerol-modified silicones, sugar-modified silicones such as tri(trimethylsiloxy)silylpropylcarbamic acid pullulan, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones such as trifluoroalkyldimethyltrimethylsiloxysilicic acid, cation-modified and polyether-modified silicones, amino-modified and polyether-modified silicones, alkyl-modified and polyether-modified silicones, and polysiloxane-oxyalkylene copolymers; various silicone copolymers such as acrylic-silicone graft copolymers; various fluoropolymers; 12-hydroxystearic acid and salts thereof; dextrin fatty acid esters such as dextrin palmitate, dextrin palmitate/octanoate and dextrin myristate; and also silicic anhydride, fumed silica (very finely divided silicic anhydride), aluminum magnesium silicate, sodium magnesium silicate, metallic soaps, metal dialkyl phosphates, bentonite, hectorite, distearyldimonium hectorite, organo-modified clay minerals such as organo-modified bentonite, sucrose fatty acid esters such as sucrose acetate stearate and sucrose mixed fatty acid esters, and fructooligosaccharide fatty acid esters.

Preferred examples of ultraviolet absorbers include benzoic acid-based ultraviolet absorbers such as p-aminobenzoic acid, monoglycerol p-aminobenzoate, ethyl N,N-dipropoxy-p-aminobenzoate, ethyl N,N-diethoxy-p-aminobenzoate, ethyl N,N-dimethyl-p-aminobenzoate, butyl N,N-dimethyl-p-aminobenzoate and ethyl N,N-dimethyl-p-aminobenzoate; anthranilic acid-based ultraviolet absorbers such as homomenthyl N-acetylanthranilate; salicylic acid-based ultraviolet absorbers such as salicylic acid and its sodium salt, amyl salicylate, menthyl salicylate, homomenthyl salicylate, octyl salicylate, phenyl salicylate, benzyl salicylate and p-isopropanol phenyl salicylate; cinnamic acid-based ultraviolet absorbers such as octyl cinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, 2-ethylhexyl p-methoxycinnamate (octyl p-methoxycinnamate), 2-ethoxyethyl p-methoxycinnamate (cinoxate), cyclohexyl p-methoxycinnamate, ethyl $\alpha$-cyano-$\beta$-phenylcinnamate, 2-ethylhexyl-$\alpha$-cyano-$\beta$-phenylcinnamate (octocrylene), glyceryl mono-2-ethylhexanoyl-di-p-methoxycinnamate, and ferulic acid and derivatives thereof; benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone and 4-hydroxy-3-carboxybenzophenone; 3-(4'-methylbenzylidene)-d,1-camphor and 3-benzylidene-d,1-camphor; 2-phenyl-5-methylbenzoxazole; 2,2' -hydroxy-5-methylphenylbenzotriazole; 2-(2'-hydroxy-5'-tert-octylphenyl)benzotriazole; 2-(2'-hydroxy-5'-methylphenyl)benzotriazole; dibenzalazine; dianisoylmethane; 5-(3,3-dimethyl-2-norbornylidene)-3-pentan-2-one; dibenzoylmethane derivatives such as 4-tert-butyl methoxydibenzoylmethane; octyl triazone; urocanic acid and urocanic acid derivatives such as ethyl urocanate; and 2-(2'-hydroxy-5'-methylphenyl)benzotriazole, 1-(3,4-dimethoxyphenyl)-4,4-dimethyl-1,3-pentanedione, hydantoin derivatives such as 2-ethylhexyl dimethoxybenzylidene dioxoimidazolidine propionate, phenylbenzimidazole sulfonic acid, terephthalylidene dicamphor sulfonic acid, drometrizole trisiloxane, methyl anthranilate, rutin and derivatives thereof, and oryzanol and derivatives thereof.

Preferred examples of antioxidants include tocopherol (vitamin E) and tocopherol derivatives such as tocopherol acetate; BHT and BHA; gallic acid derivatives such as propyl gallate; vitamin C (ascorbic acid) and derivatives thereof; erythorbic acid and derivatives thereof; sulfites such as sodium sulfite; bisulfites such as sodium bisulfite; thiosulfates such as sodium thiosulfate; metabisulfites; thiotaurine and hypotaurine; thioglycerol, thiourea, thioglycolic acid and cysteine hydrochloride.

Preferred examples of reducing agents include thioglycolic acid, cysteine and cysteamine.

Preferred examples of oxidizing agents include hydrogen peroxide solution, ammonium persulfate, sodium bromate and percarbonic acid.

Preferred examples of preservatives and antimicrobial agents include hydroxybenzoic acids and salts or esters thereof, such as methylparaben, ethylparaben, propylparaben and butylparaben; salicylic acid; sodium benzoate; phenoxyethanol; 1,2-diols such as 1,2-pentanediol and 1,2-hexanediol; isothiazolinone derivatives such as methylchloroisothiazolinone and methylisothiazolinone; imidazolinium urea; dehydroacetic acid and salts thereof; phenols; halogenated bisphenols such as triclosan, acid amides; quaternary ammonium salts; trichlorocarbanide, zinc pyrithione, benzalkonium chloride, benzethonium chloride, sorbic acid, chlorhexidine, chlorhexidine gluconate, halocarban, hexachlorophene and hinokitiol; other phenols such as phenol, isopropylphenol, cresol, thymol, p-chlorophenol, phenylphenol and phenylphenol sodium; phenyl ethyl alcohol, photosensitizers, antimicrobial zeolite, and silver ions.

Preferred examples of sequestrants (chelating agents) include edetates (ethylenediaminetetraacetates) such as EDTA, EDTA-2Na, EDTA-3Na, and EDTA-4Na; hydroxyethylethylenediaminetriacetates such as HEDTA-3Na; pentetates (diethylenetriaminepentaacetate); phytic acid; phosphonic acids such as etidronic acid and salts thereof such as the sodium salts; sodium oxalate; polyamino acids such as polyaspartic acid and polyglutamic acid; sodium polyphosphate, sodium metaphosphate, and phosphoric acid; sodium citrate, citric acid, alanine, dihydroxyethylglycine, gluconic acid, ascorbic acid, succinic acid and tartaric acid.

Preferred examples of plant, animal and microbial extracts include iris extract, *Angelica keiskei* extract, *Thujopsis dolabrata* extract, asparagus extract, avocado extract, *Hydrangea macrophylla* leaf extract, almond extract, *Althea officinalis* root extract, arnica extract, aloe extract, apricot extract, apricot kernel extract, ginkgo extract, *Artemisia capillaris* flower extract, fennel fruit extract, turmeric root extract, oolong tea extract, uva-ursi extract, rose fruit extract, *Echinacea angustifolia* leaf extract, *Isodonis japonicus* extract, Scutellaria root extract, Phellodendron bark extract, *Coptis japonica* rhizome extract, barley extract, *Panax ginseng* extract, St. John's wort extract, *Lamium album* extract, *Ononis spinosa* extract, *Nasturtium officinale* extract, orange extract, dried sea water, seaweed extract, Japanese persimmon leaf extract, *Pyracantha fortuneana* fruit extract, hydrolyzed elastin, hydrolyzed wheat powder, hydrolyzed silk, Pueraria root extract, *Chamomilla recutita* extract, oil-soluble *Chamomilla recutita* extract, carrot extract, *Artemisia capillaris* extract, *Avena sativa* extract, *Hibiscus sabdariffa* extract, licorice extract, oil-soluble licorice extract, kiwi fruit extract, kiou extract, *Auricularia auricula-judae* extract, Cinchona bark extract, cucumber extract, *Paulownia tomentosa* leaf extract, guanosine, guava extract, Sophora root extract, *Gardenia jasminoides* extract, *Sasa veitchii* extract, *Sophora angustifolia* root extract, walnut extract, chestnut extract, grapefruit extract, *Clematis vitalba* leaf extract, purple rice extract, brown sugar extract, black vinegar, chlorella extract, mulberry extract, gentian extract, geranium herb extract, black tea extract, yeast extract, magnolia bark extract, coffee extract, burdock root extract, rice extract, fermented rice extract, fermented rice bran extract, rice germ oil, comfrey extract, collagen, bilberry extract, *Asarum sieboldi* root extract, Bupleurum root extract, umbilical extract, saffron extract, salvia extract, *Saponaria officinalis* extract, bamboo grass extract, *Crataegus cuneata* fruit extract, *Coriandum sativum* fruit extract, *Zanthoxylum piperitum* peel extract, shiitake mushroom extract, *Rehmannia chinensis* extract, *Lithospermum officinale* root extract, perilla leaf extract, *Tilia cordata* flower extract, *Spiraea ulmaria* extract, jatoba extract, peony root extract, ginger extract, *Acorus calamus* root extract, *Betula alba* extract, *Tremella fuciformis* extract, *Equisetum arvense* extract, stevia extract, stevia fermentation product, *Tamarix chinensis* extract, *Hedera helix* extract, *Crataegus oxyacantha* extract, *Sambucus nigra* flower extract, *Achillea millefolium* extract, *Mentha piperita* extract, sage extract, mallow extract, *Cnidium officinale* root extract, *Swertia japonica* extract, mulberry root bark extract, rhubarb extract, soybean extract, *Zizyphus jujuba* fruit extract, thyme extract, dandelion extract, lichen extract, tea extract, clove extract, *Imperata cylindrica* root extract, *Citrus unshiu* peel extract, tea tree oil, *Rubus suavissimus* extract, capsicum extract, *Angelica acutiloba* root extract, *Calendula officinalis* extract, peach kernel extract, bitter orange peel extract, *Houttuynia cordata* extract, tomato extract, natto extract, carrot extract, garlic extract, *Rosa canina* fruit extract, hibiscus extract, *Ophiopogon japonicus* root extract, lotus extract, parsley extract, birch extract, honey, witch hazel extract, *Parietaria officinalis* extract, *Rabdosia japonica* extract, bisabolol, cypress extract, Bifidobacterium extract, loquat extract, *Tussilago farfara* extract, Japanese butterbur scape extract, *Poria cocos* sclerotium extract, butcher's broom extract, grape extract, grape seed extract, propolis, Luffa extract, safflower extract, peppermint extract, *Tilia platyphyllos* flower extract, peony root extract, hop extract, *Rosa rugosa* extract, pine extract, horse chestnut extract, skunk cabbage extract, *Sapindus mukurossi* extract, lemon balm extract, *Nemacystus decipiens* extract, peach extract, *Centaurea cyanus* flower extract, eucalyptus extract, *Saxifraga sarmentosa* extract, *Citrus junos* fruit extract, lily extract, coix seed extract, *Artemisia vulgaris* extract, lavender extract, green tea extract, egg shell membrane extract, apple extract, rooibos tea extract, lychee extract, lettuce extract, lemon extract, *Forsythia suspensa* fruit extract, *Astragalus sinicus* extract, rose extract, rosemary extract, *Anthemis nobilis* extract, royal jelly extract and *Sanguisorba officinalis* extract.

Preferred examples of pH adjustors, acids and alkalis include citric acid, sodium citrate, lactic acid, sodium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propanediol, 2-amino-2-methyl-1-propanol, 2-amino-2-hydroxymethyl-1,3-propanediol, arginine, sodium hydroxide, potassium hydroxide, ammonia water, guanidine carbonate and ammonium carbonate.

Preferred examples of moisturizers and texture improvers include polyols and polymers thereof such as glycerin, butylene glycol, propylene glycol, 3-methyl-1,3-butanediol, 1,3-propanediol, 2-methyl-1,3-propanediol, trimethylolpropane, pentaerythritol, hexylene glycol, diglycerin, polyglycerin, diethylene glycol, polyethylene glycol, dipropylene glycol, polypropylene glycol and ethylene glycol-propylene glycol copolymers; glycol alkyl ethers such as diethylene glycol monoethyl ether (ethoxydiglycol), ethylene glycol monoethyl ether, ethylene glycol monobutyl ether and diethylene glycol dibutyl ether; sugar alcohols such as sorbitol, xylitol, erythritol, mannitol and maltitol; saccharides and derivatives thereof such as glucose, fructose, galactose, mannose, threose, xylose, arabinose, fucose, ribose, deoxyribose, maltose, trehalose, lactose, raffinose, gluconic acid, glucuronic acid, cyclodextrins (α-, β- and γ-cyclodextrins, and modified cyclodextrins such as maltosyl cyclodextrin and hydroxyalkylated cyclodextrins), β-glucan, chitin, chitosan, heparin and derivatives thereof, pectin, arabinogalactan, dextrin, dextran, glycogen, ethyl glucoside, and glucosylethyl methacrylate polymers or copolymers; hyaluronic acid and sodium hyaluronate; sodium chondroitin sulfate; mucoitin sulfate, charonin sulfate, kerato sulfate, and dermatan sulfate; *Tremella fuciformis* extract and *Tremella fuciformis* polysaccharides; fucoidan; tuberose polysaccharides and naturally occurring polysaccharides; organic acids such as citric acid, tartaric acid and lactic acid, as well as salts thereof; urea; 2-pyrrolidone-5-carboxylic acid and salts thereof such as the sodium salt; amino acids such as betaine (trimethylglycine), proline, hydroxyproline, arginine, lysine, serine, glycine, alanine, phenylalanine, tyrosine, β-alanine, threonine, glutamic acid, glutamine, asparagine, aspartic acid, cysteine, cystine, methionine, leucine, isoleucine, valine, tryptophan, histidine and taurine, as well as salts thereof; protein peptides and derivatives thereof such as collagen, fish collagen, atelocollagen, gelatin, elastin, collagen degradation peptides, hydrolyzed collagen, hydroxypropylammonium chloride hydrolyzed collagen, elastin degradation peptides, keratin degradation peptides, hydrolyzed keratin, conchiolin degradation peptides, hydrolyzed conchiolin, silk protein degradation peptides, hydrolyzed silk, sodium lauroyl hydrolyzed silk, soy protein degradation peptides, wheat protein degradation peptides, hydrolyzed wheat protein, casein degradation peptides and acylated peptides; acylated peptides such as palmitoyl oligopeptide, palmitoyl pentapeptide and palmitoyl tetrapeptide; silylated peptides; lactobacillus culture, yeast extracts, eggshell membrane protein, bovine submaxillary mucin, hypotaurine, sesame lignan glycosides, glutathione, albumin and whey; choline chloride and phosphorylcholine; animal and plant extract components such as placenta extract, elastin, collagen, aloe extract, witch hazel solution, Luffa solution, *Chamomilla recutita* extract, licorice extract, comfrey extract, silk extract, *Rosa roxburghii* extract, *Achillea millefolium* extract, eucalyptus extract and melilot extract; and ceramides such as natural ceramides (types 1, 2, 3, 4, 5 and 6), hydroxyceramides, pseudoceramides, sphingoglycolipids, ceramide-containing extracts and glucosylceramide-containing extracts.

Preferred examples of whitening agents include hydroquinone glycosides such as arbutin and α-arbutin, as well as esters thereof; ascorbic acid and ascorbic acid derivatives, including ascorbyl phosphates such as sodium ascorbyl phosphate and magnesium ascorbyl phosphate, ascorbyl fatty acid esters such as ascorbyl tetraisopalmitate, alkyl ethers of ascorbic acid such as ethyl ascorbate, ascorbyl glucosides such as ascorbyl 2-glucoside and fatty acid esters thereof, sulfate esters of ascorbic acid and tocopheryl ascorbyl phosphate; kojic acid, ellagic acid, tranexamic acid and derivatives thereof, ferulic acid and derivatives thereof, placenta extract, glutathione, oryzanol, butylresorcinol, and plant extracts such as oil-soluble *Chamomilla recutita* extract, oil-soluble licorice extract, *Tamarix chinensis* extract and Saxifraga sarmentosa extract.

Preferred examples of vitamins and derivatives thereof include vitamin A compounds such as retinol, retinol acetate and retinol palmitate; the vitamin B group such as thiamine hydrochloride, thiamine sulfate, riboflavin, riboflavin acetate, pyridoxine hydrochloride, pyridoxine dioctanoate, pyridoxine dipalmitate, flavin adenine dinucleotide, cyanocobalamin, folic acids, nicotinic acid compounds such as nicotinamide and benzyl nicotinate, and cholines; vitamin C compounds such as ascorbic acid and the sodium and other salts thereof; vitamin D; vitamin E compounds such as α-, β-, γ- and δ-tocopherols; other vitamins such as pantothenic acid and biotin; ascorbic acid derivatives, including ascorbyl phosphates such as sodium ascorbyl phosphate and magnesium ascorbyl phosphate, fatty acid esters of ascorbic acid such as ascorbyl tetraisopalmitate, ascorbyl stearate, ascorbyl palmitate and ascorbyl dipalmitate, alkyl ethers of ascorbic acid such as ethyl ascorbate, ascorbyl glucosides such as ascorbyl 2-glucoside and fatty acid esters thereof, and tocopheryl ascorbyl phosphate; and vitamin derivatives, including tocopherol derivatives such as tocopherol nicotinate, tocopherol acetate, tocopherol linoleate, tocopherol ferulate and tocopherol phosphate, as well as tocotrienol and various other vitamin derivatives.

Preferred examples of antiphlogistics and anti-inflammatory agents include glycyrrhizic acid and derivatives thereof, glycyrrhetic acid derivatives, salicylic acid derivatives, hinokitiol, guaiazulene, allantoin, indomethacin, zinc oxide, hydrocortisone acetate, prednisone, diphenhydramine hydrochloride, and chlorpheniramine maleate; and plant extracts such as peach leaf extract and *Artemisia princeps* leaf extract.

Preferred examples of hair growth-promoting agents, blood circulation promoters and stimulants include plant extracts and tinctures such as *Swertia japonica* extract, capsicum tincture, ginger tincture, ginger extract, and cantharis tincture; capsaicin, nonanoic acid vanillylamide, zingerone, ichthammol, tannic acid, borneol, cyclandelate, cinnarizine, tolazoline, acetylcholine, verapamil, cepharanthine, γ-oryzanol, cepharanthine, vitamin E and derivatives thereof such as tocopherol nicotinate and tocopherol acetate, γ-oryzanol, nicotinic acid and derivatives thereof such as nicotinamide, benzyl nicotinate, inositol hexanicotinate and nicotinyl alcohol, allantoin, Photosensitizer 301, Photosensitizer 401, carpronium chloride, pentadecanoic acid monoglyceride, flavanonol derivatives, stigmasterol or stigmastanol and glycosides thereof, and minoxidil.

Preferred examples of hormones include estradiol, estrone, ethinylestradiol, cortisone, hydrocortisone and prednisone.

Preferred examples of other medicinal agents such as anti-wrinkle agents, anti-aging agents, skin-firming agents, cooling agents, warming agents, wound-healing promoters, irritation relievers, analgesics and cell activators include retinols, retinoic acids and tocopheryl retinoate; lactic acid, glycolic acid, gluconic acid, fruit acids, salicylic acid and derivatives thereof such as glycosides and esters, and α- or β-hydroxy acids and derivatives thereof, such as hydroxycapric acid, long-chain α-hydroxy fatty acids and cholesteryl esters of long-chain α-hydroxy fatty acids; γ-aminobutyric acid and γ-amino-β-hydroxybutyric acid; carnitine; creatine; ceramides and sphingosines; caffeine, xanthine and derivatives thereof; antioxidants and active oxygen scavengers such as coenzyme Q10 (ubiquinone), carotene, lycopene and astaxanthin; catechins; flavones such as quercetin; isoflavones; gallic acid and sugar ester derivatives thereof; polyphenols such as tannin, sesamin, protoanthocyanidin, chlorogenic acid and apple polyphenols; rutin and derivatives thereof such as glycosides; hesperidin and derivatives thereof such as glycosides; lignan glycosides; licorice extract-related substances such as glabridin, glabrene, liquiritin, and isoliquiritin; lactoferrin; shogaol and gingerol; perfume substances such as menthol, camphor and cedrol, as well as derivatives thereof; capsaicin, vanillin, and derivatives thereof; insect repellents such as diethyltoluamide; and complexes of physiologically active substances and cyclodextrins.

Examples of antipruritics include diphenhydramine hydrochloride, chlorpheniramine maleate, camphor and substance P inhibitors. Examples of exfoliates/keratolytic agents include salicylic acid, sulfur, resorcin, selenium sulfide and pyridoxine. Preferred examples of antiperspirants include aluminum chlorohydrate, aluminum chloride, zinc oxide and zinc p-phenolsulfonate.

Examples of algefacients include menthol and methyl salicylate.

Examples of astringents include citric acid, tartaric acid, lactic acid, aluminum potassium sulfate and tannic acid. Preferred examples of enzymes include superoxide dismutase, catalase, lysozyme chloride, lipase, papain, pancreatin and protease.

Preferred examples of nucleic acids include ribonucleic acid and salts thereof, deoxyribonucleic acid and salts thereof, and adenosine triphosphate disodium.

Preferred examples of water include, aside from ordinary water and purified water: hard water, soft water, spring water, deep ocean water, ionized alkaline water, ionized acid water, ionized water and cluster water.

In addition, the skin cosmetic may contain known ingredients, including ingredients mentioned in, for example, the INCI dictionary (The International Cosmetic Ingredient Dictionary and Handbook), the Japanese Standards of Quasi-drug Ingredients (including the former Japanese Standards of Cosmetic Ingredients and the former Japanese Standards of Cosmetic Ingredients by Type of Cosmetic), Japanese Pharmacopoeia, Japanese Pharmaceutical Excipients and Japan's Specifications and Standards for Food Additives, and ingredients mentioned in Japanese and foreign patent publications and patent application publications (including Japanese translations of PCT international application publications and re-publications of PCT international publications) belonging to International Patent Classification (IPC) classes A61K8 and C11D, in known combinations, proportions and amounts.

The skin cosmetic of the invention includes, for example, skin care products, hair care products, antiperspirants, makeup products, UV protection products and scented products. Specific examples include base cosmetics such as milky emulsions, creams, lotions, calamine lotions, sunscreens, makeup bases, suntan lotions, aftershave lotions, preshave lotions, packs, cleansing materials, facial cleansers, cosmetics for acne, and essences; makeup cosmetics such as foundation, face powder, mascara, concealer, eye shadow, eyeliner, eyebrow, cheek, nail color, lip cream and lipstick; and also shampoos, rinses, conditioners, hair colors, hair tonics, setting agents, body powders, hair growth promoters, deodorants, depilatories, soaps, body shampoos, bath preparations, hand soaps and perfumes. The form of the product is not particularly limited and includes, for example, liquids, emulsions, creams, solids, pastes, gels, powders, multilayer preparations, mousses and sprays.

## EXAMPLES

Examples and Comparative Examples are given below by way of illustration, although the invention is not limited to these Examples. Evaluations in the production Examples and Comparative Production Examples below were carried out by the following methods.

(1) Sphere-Equivalent Volume Mean Particle Size (MV) of Polymer Particles

The volume mean particle size was measured using the MICROTRACK MT3000 (Nikkiso Co., Ltd.).

(2) Aspect Ratio of Polymer Particles

A scanning electron microscope (S-4800, from Hitachi High Technologies Corporation; abbreviated below as "SEM") was used to capture photographs at a magnification at which particle measurement is possible (300 to 30,000×), thereby rendering the oblate ellipsoidal polymer particles obtained into two-dimensional images. In the images, 100 particles were randomly sampled and the length (L) of the flat region, breadth (D) of the flat region and the lateral thickness (T) for each particle were measured. Based on these measurements, the following were determined:

average value $P^1_{AV}$ of the aspect ratio L/D;
average value $P^2_{AV}$ of the aspect ratio D/T; and
average value $P^3_{AV}$ of the aspect ratio L/T.

The average length $L_{AV}$ of the flat region, average breadth $D_{AV}$ of the flat region and average thickness $T_{AV}$ for the particles were similarly calculated after measuring the length L, breadth D and lateral thickness T of 100 randomly sampled particles. FIG. 1 shows a diagram illustrating the length (L), breadth (D) and thickness (T) of oblate ellipsoidal polymer particle A.

(3) Specific Surface Area (SB)

The specific surface area (SB) was measured by the nitrogen gas adsorption method using an automatic specific surface area/pore size distribution measuring instrument (BELSORP-max, from Bel Japan, Inc.).

(4) Theoretical Specific Surface Area (SD)

Letting 2r (m) be the volume mean particle size, r (m) be the radius and G (g/$m^3$) be the density of a polymer particle, the surface area S' ($m^2$) and volume V ($m^3$) of a spherical particle of radius r (m) are expressed as follows.
Surface area of spherical particle of radius r: $S' = 4\pi r^2$
Volume of spherical particle of radius r: $V = 4\pi r^3/3$
In this case, the number N of particles contained in one gram of particles is expressed as follows.
Number of particles contained in one gram of particles: N = 1/VG
Therefore, the theoretical specific surface area SD ($m^2$/g) of spherical particles calculated from the polymer particles is expressed as follows.

$$SD = S'N = S'/VG = 3/rG$$

(5) Bulk Density of Polymer Particles

Calculated as the loose bulk density using Method 1 ("Measurement in a graduated cylinder") of the test methods specified in the Japanese, European and U.S. Pharmacopoeias. Units are in g/mL.

(6) Measurement of Water Absorption

A dried polymer particle powder was dispersed in water to a concentration of about 2 wt% and left at rest for one day, following which it was re-dispersed and then filtered under reduced pressure using a glass filter. The glass filter was then subjected to 30 minutes of centrifugation at 3,000 rpm using a centrifuge (CR-20GII, from Hitachi High Technologies Corporation), following which the resulting polymer particle powder was dried. The weight of the powder before and after drying was measured, and the difference in weight was treated as the water absorption.

(7) Measurement of Oil Absorption

Oil absorption was measured in accordance with the boiled linseed oil method described in JIS K 5101.

(8) pH Measurement

The pH was determined by immersing pH test paper (from Whatman) in the reaction mixture under stirring, and visually judging the change in color.

[1] Production of Oblate Ellipsoidal Polymer Particles

[Production Example 1]

The ingredients shown below were charged all at once into a 2,000 mL flask and stirred at room temperature (here and below, 25°C) for one hour.

| | |
|---|---|
| Water | 912.0 g |
| Methanol | 45.0 g |
| Polypropylene glycol (#3000) | 88.0 g |
| Polyvinyl pyrrolidone (K-15) | 56.5 g |
| Polyethylene oxide (M.W., 300,000) | 22.5 g |
| Benzoyl peroxide (oil-soluble) | 1.6 g |
| Sodium persulfate (water-soluble) | 3.7 g |
| Methyl methacrylate | 524.0 g |
| Ethylene glycol dimethacrylate | 6.3 g |

At this time, the liquid phase was in a state where an aqueous phase portion, an emulsified phase portion and an oil phase portion are intermingled. The pH of the system was measured and found to be 7.

Next, the solution was set to an oil bath temperature of 85°C and, under a stream of nitrogen, heating and stirring (400 rpm) were begun. The polymerization reaction was carried out for 8 hours, giving a methyl methacrylate-ethylene glycol dimethacrylate copolymer particle dispersion. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle A1.

One hundred of the resulting particles were randomly sampled and their shapes examined with the SEM. As shown in FIG. 2, the particles were oblate ellipsoidal polymer particles having fine particles with a mean particle size of 0.3 μm attached to the surface layer portion and surface thereof.

$L_{AV}$ was 9 μm, $D_{AV}$ was 3 μm, and $T_{AV}$ was 1 μm. The particles were checked for extraneous matter, but substantially no deformed matter such as agglomerates or mutually sticking particles was observed.

[Production Example 2]

The ingredients shown below were charged all at once into a 2,000 mL flask and stirred at room temperature for one hour.

| | |
|---|---|
| Water | 912.0 g |
| Ethanol | 27.0 g |
| Polypropylene glycol (#3000) | 96.0 g |
| Polyvinyl pyrrolidone (K-15) | 48.0 g |
| Sucrose laurate | 9.6 g |
| Azobisisobutyronitrile (AIBN) (oil-soluble) | 2.8 g |
| Ammonium persulfate (water-soluble) | 3.2 g |
| Methyl methacrylate | 580.0 g |
| Ethylene glycol dimethacrylate | 5.8 g |

At this time, the liquid phase was in a state where an aqueous phase portion, an emulsified phase portion and an oil phase portion are intermingled. The pH of the system was measured and found to be 7.

Next, the solution was set to an oil bath temperature of 75°C and, under a stream of nitrogen, heating and stirring (250 rpm) were begun. The polymerization reaction was carried out for 8 hours, giving a methyl methacrylate-ethylene glycol dimethacrylate copolymer particle dispersion. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle A2.

One hundred of the resulting particles were randomly sampled and their shapes examined with the SEM. As shown in FIG. 3, the particles were oblate ellipsoidal polymer particles having fine particles with a mean particle size of 1.2 μm attached to the surface layer portion and surface thereof.

$L_{AV}$ was 35 μm, $D_{AV}$ was 8 μm, and $T_{AV}$ was 2 μm. The particles were checked for extraneous matter, but substantially no deformed matter such as agglomerates or mutually sticking particles was observed.

[Production Example 3]

The ingredients shown below were charged all at once into a 2,000 mL flask and stirred at room temperature for one hour.

| | |
|---|---|
| Water | 930.0 g |
| Ethanol | 45.5 g |
| Polypropylene glycol (#3000) | 68.0 g |
| Polyvinyl pyrrolidone (K-15) | 53.5 g |
| Sucrose laurate | 10.5 g |
| 2,2'-Dimethyl azobis(isobutyrate) (oil-soluble) | 2.2 g |
| Ammonium persulfate (water-soluble) | 3.6 g |
| Methyl methacrylate | 555.0 g |
| Ethylene glycol dimethacrylate | 5.6 g |

At this time, the liquid phase was in a state where an aqueous phase portion, an emulsified phase portion and an oil phase portion are intermingled. The pH of the system was measured and found to be 7.

Next, the solution was set to an oil bath temperature of 75°C and, under a stream of nitrogen, heating and stirring (400 rpm) were begun. The polymerization reaction was carried out for 8 hours, giving a methyl methacrylate-ethylene glycol dimethacrylate copolymer particle dispersion. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle A3.

One hundred of the resulting particles were randomly sampled and their shapes examined with the SEM. As shown in FIG. 4, the particles were oblate ellipsoidal polymer particles having fine particles with a mean particle size of 0.6 μm attached to the surface layer portion and surface thereof.

$L_{AV}$ was 43 μm, $D_{AV}$ was 4 μm, and $T_{AV}$ was 2 μm. The particles were checked for extraneous matter, but substantially no deformed matter such as agglomerates or mutually sticking particles was observed.

[Production Example 4]

The ingredients shown below were charged all at once into a 2,000 mL flask and stirred at room temperature for one hour.

| | |
|---|---|
| Water | 760.0 g |
| Ethanol | 44.6 g |
| Polypropylene glycol (#3000) | 88.0 g |

(continued)

| | |
|---|---|
| Polyvinyl pyrrolidone (K-15) | 49.5 g |
| Sucrose laurate | 8.6 g |
| AIBN (oil-soluble) | 2.4 g |
| Ammonium persulfate (water-soluble) | 1.9 g |
| Methyl methacrylate | 480.0 g |

At this time, the liquid phase was in a state where an aqueous phase portion, an emulsified phase portion and an oil phase portion are intermingled. The pH of the system was measured and found to be 7.

Next, the solution was set to an oil bath temperature of 75°C and, under a stream of nitrogen, heating and stirring (300 rpm) were begun. The polymerization reaction was carried out for 8 hours, giving a polymethyl methacrylate particle dispersion. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle A4.

One hundred of the resulting particles were randomly sampled and their shapes examined with the SEM. The particles were oblate ellipsoidal polymer particles having fine particles with a mean particle size of 0.4 μm attached to the surface layer portion and surface thereof.

$L_{AV}$ was 16 μm, $D_{AV}$ was 8 μm, and $T_{AV}$ was 2 μm. The particles were checked for extraneous matter, but substantially no deformed matter such as agglomerates or mutually sticking particles was observed.

[Production Example 5]

The ingredients shown below were charged all at once into a 2,000 mL flask and stirred at room temperature for one hour.

| | |
|---|---|
| Water | 880.0 g |
| Ethanol | 52.0 g |
| Polypropylene glycol (#2000) | 98.0 g |
| Polyvinyl pyrrolidone (K-15) | 46.0 g |
| Sucrose laurate | 11.0 g |
| 2,2'-Dimethyl azobis(isobutyrate) (oil-soluble) | 2.5 g |
| Ammonium persulfate (water-soluble) | 3.5 g |
| Methyl methacrylate | 684.0 g |
| Ethylene glycol dimethacrylate | 10.3 g |

At this time, the liquid phase was in a state where an aqueous phase portion, an emulsified phase portion and an oil phase portion are intermingled. The pH of the system was measured and found to be 7.

Next, the solution was set to an oil bath temperature of 73°C and, under a stream of nitrogen, heating and stirring (300 rpm) were begun. The polymerization reaction was carried out for 8 hours, giving a methyl methacrylate-ethylene glycol dimethacrylate copolymer particle dispersion. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle A5.

One hundred of the resulting particles were randomly sampled and their shapes examined with the SEM. The particles were oblate ellipsoidal polymer particles having fine particles with a mean particle size of 0.6 μm attached to the surface layer portion and surface thereof.

$L_{AV}$ was 24 μm, $D_{AV}$ was 10 μm, and $T_{AV}$ was 4 μm. The particles were checked for extraneous matter, but substantially no deformed matter such as agglomerates or mutually sticking particles was observed.

[Production Example 6]

The ingredients shown below were charged all at once into a 2,000 mL flask and stirred at room temperature for one hour.

| | |
|---|---|
| Water | 865.0 g |
| Ethanol | 42.5 g |
| Polypropylene glycol (#2000) | 102.0 g |
| Polyvinyl pyrrolidone (K-30) | 22.5 g |
| Fatty acid glycerides | 13.0 g |
| Azobisisovaleronitrile (oil-soluble) | 2.0 g |
| 2,2'-Azobis(2-methyl-N-phenylpropionamidine)dihydrochloride (water-soluble) | 2.5 g |
| Methyl methacrylate | 580.0 g |
| Polyethylene glycol dimethacrylate (n=4) | 17.4 g |

At this time, the liquid phase was in a state where an aqueous phase portion, an emulsified phase portion and an oil phase portion are intermingled. The pH of the system was measured and found to be 7.

Next, the solution was set to an oil bath temperature of 76°C and, under a stream of nitrogen, heating and stirring (350 rpm) were begun. The polymerization reaction was carried out for 8 hours, giving a methyl methacrylate-polyethylene glycol dimethacrylate (n=4) copolymer particle dispersion. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle A6.

One hundred of the resulting particles were randomly sampled and their shapes examined with the SEM. The particles were oblate ellipsoidal polymer particles having fine particles with a mean particle size of 0.8 μm attached to the surface layer portion and surface thereof.

$L_{AV}$ was 29 μm, $D_{AV}$ was 7 μm, and $T_{AV}$ was 3 μm. The particles were checked for extraneous matter, but substantially no deformed matter such as agglomerates or mutually sticking particles was observed.

[Production Example 7]

The ingredients shown below were charged all at once into a 2,000 mL flask and stirred at room temperature for one hour.

| | |
|---|---|
| Water | 712.5 g |
| Methanol | 37.5 g |
| Polypropylene glycol (#3000) | 75.0 g |
| Polyvinyl pyrrolidone (K-30) | 43.2 g |
| Sucrose palmitate | 10.8 g |
| AIBN (oil-soluble) | 3.2 g |
| Ammonium persulfate (water-soluble) | 4.3 g |
| Methyl methacrylate | 486.0 g |
| Methacrylic acid | 54.0 g |
| Ethylene glycol dimethacrylate | 10.8 g |

At this time, the liquid phase was in a state where an aqueous phase portion, an emulsified phase portion and an oil phase portion are intermingled. The pH of the system was measured and found to be 7.

Next, the solution was set to an oil bath temperature of 72°C and, under a stream of nitrogen, heating and stirring (250 rpm) were begun. The polymerization reaction was carried out for 8 hours, giving a methyl methacrylate-methacrylic acid-ethylene glycol dimethacrylate copolymer particle dispersion. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle A7.

One hundred of the resulting particles were randomly sampled and their shapes examined with the SEM. The particles were oblate ellipsoidal polymer particles having fine particles with a mean particle size of 1.2 μm attached to the surface layer portion and surface thereof.

$L_{AV}$ was 49 μm, $D_{AV}$ was 19 μm, and $T_{AV}$ was 6 μm. The particles were checked for extraneous matter, but substantially no deformed matter such as agglomerates or mutually sticking particles was observed.

[Production Example 8]

The ingredients shown below were charged all at once into a 2,000 mL flask and stirred at room temperature for one hour.

| | |
|---|---|
| Water | 912.0 g |
| Isopropyl alcohol | 27.4 g |
| Polypropylene glycol (#4000) | 69.5 g |
| Polyvinyl pyrrolidone (K-15) | 52.6 g |
| Polyoxyethylene lauryl ether | 15.5 g |
| Azobismethylbutyronitrile (oil-soluble) | 2.6 g |
| Potassium persulfate (water-soluble) | 3.0 g |
| Styrene | 652.0 g |
| Divinylbenzene | 9.8 g |

At this time, the liquid phase was in a state where an aqueous phase portion, an emulsified phase portion and an oil phase portion are intermingled. The pH of the system was measured and found to be 7.

Next, the solution was set to an oil bath temperature of 78°C and, under a stream of nitrogen, heating and stirring (400 rpm) were begun. The polymerization reaction was carried out for 8 hours, giving a styrene-divinylbenzene copolymer particle dispersion. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle A8.

One hundred of the resulting particles were randomly sampled and their shapes examined with the SEM. The particles were oblate ellipsoidal polymer particles having fine particles with a mean particle size of 0.5 μm attached to the surface layer portion and surface thereof.

$L_{AV}$ was 19 μm, $D_{AV}$ was 6 μm, and $T_{AV}$ was 2 μm. The particles were checked for extraneous matter, but substantially no deformed matter such as agglomerates or mutually sticking particles was observed.

[Comparative Production Example 1]

The ingredients shown below were charged all at once into a 2,000 mL flask and a suspension was prepared using the dispersing element in a dispersion mixer at 1,000 rpm. The suspension was heated and stirred for 8 hours under a stream of nitrogen and at an oil bath temperature of 80°C, giving a particle dispersion. Next, centrifugal separation was repeated five times and classification and washing operations were carried out, thereby producing spherical polymer particles B1 of polymethyl methacrylate alone having a mean particle size of 5 μm.

| | |
|---|---|
| Water | 1,386.5 g |
| Methyl methacrylate | 173.4 g |
| Lauryl peroxide | 8.6 g |
| Polyvinyl pyrrolidone (K-30) | 17.3 g |

[Comparative Production Example 2]

Aside from not using AIBN and changing the amount of ammonium persulfate used to 14.0 g, a methyl methacrylate-ethylene glycol dimethacrylate copolymer particle dispersion was obtained in the same way as in Production Example 2. The pH of the reaction system before the start of heating was 7, the pH of the reaction system two hours after the start of heating was 2, and the pH of the reaction system at the time of reaction completion was 1.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the particle dispersion was filtered and the resulting filtered matter was repeatedly (5 times) washed with methanol and filtered, and then vacuum dried, giving Polymer Particle B2.

One hundred of the resulting particles were randomly sampled and their shapes examined with the SEM. As shown in FIG. 5, the particles were ellipsoidal (prolate spheroidal) polymer particles without a flat region. The average length ($L_{AV}$) was 28 μm, the average breach ($D_{AV}$) was 7 μm, and the average aspect ratio ($P^1_{AV}$) was 4. The particles were checked for extraneous matter, but substantially no deformed matter such as agglomerates or mutually sticking particles was observed.

Table 1 below shows the shape, particle ingredients, volume mean particle size (MV), average length of the flat region ($L_{AV}$), average breadth of the flat region ($D_{AV}$), average thickness ($T_{AV}$), average value ($P^1_{AV}$) of the aspect ratio L/D, average value ($P^2_{AV}$) of the aspect ratio D/T and average value ($P^3_{AV}$) of the aspect ratio L/T for polymer particles A1 to A8 and B1 and B2.

[Table 1]

| Polymer particle | Ingredients | Main shape | MV (μm) | $L_{AV}$ (μm) | $D_{AV}$ (μm) | $T_{AV}$ (μm) | $P^1_{AV}$ | $P^2_{AV}$ | $P^3_{AV}$ |
|---|---|---|---|---|---|---|---|---|---|
| A1 | methyl methacrylate<br>ethylene glycol dimethacrylate | oblate ellipsoidal | 6 | 9 | 3 | 1 | 3 | 3 | 9 |
| A2 | methyl methacrylate<br>ethylene glycol dimethacrylate | oblate ellipsoidal | 14 | 35 | 8 | 2 | 4.4 | 4 | 17.5 |
| A3 | methyl methacrylate<br>ethylene glycol dimethacrylate | oblate ellipsoidal | 19 | 43 | 4 | 2 | 10.8 | 2 | 21.5 |
| A4 | methyl methacrylate | oblate ellipsoidal | 11 | 16 | 8 | 2 | 2 | 4 | 8 |
| | methyl methacrylate<br>ethylene glycol dimethacrylate | oblate ellipsoidal | 13 | 24 | 10 | 4 | 2.4 | 2.5 | 6 |
| A6 | methyl methacrylate<br>polyethylene glycol dimethacrylate (n=4) | oblate ellipsoidal | 12 | 29 | 7 | 3 | 4.1 | 2.3 | 9.7 |
| A7 | methyl methacrylate<br>methacrylic acid<br>ethylene glycol dimethacrylate | oblate ellipsoidal | 23 | 49 | 19 | 6 | 2.6 | 3.2 | 8.2 |
| A8 | styrene<br>divinylbenzene | oblate ellipsoidal | 10 | 19 | 6 | 2 | 3.2 | 3 | 9.5 |
| B1 | methyl methacrylate | spherical | 5 | 5 | 5 | 5 | 1 | 1 | 1 |

(continued)

| Polymer particle | Ingredients | Main shape | MV (μm) | $L_{AV}$ (μm) | $D_{AV}$ (μm) | $T_{AV}$ (μm) | $P^1_{AV}$ | $P^2_{AV}$ | $P^3_{AV}$ |
|---|---|---|---|---|---|---|---|---|---|
| B2 | methyl methacrylate<br>ethylene glycol dimethacrylate | oblate spheroidal | 13 | 28 | 7 | 7 | 4 | 1 | 4 |

Table 2 below shows the specific surface area (SB), theoretical specific surface area (SD) and ratio (SB/SD) therebetween, and also the bulk density, water absorption and oil absorption for polymer particles A1 to A8 and B1 and B2.

[Table 2]

| Polymer particle | SB ($m^2/g$) | SD ($m^2/g$) | SB/SD | Bulk density (g/mL) | Water absorption (g/100 g) | Oil absorption (g/ 100 g) |
|---|---|---|---|---|---|---|
| A1 | 4.3156 | 0.833 | 5.18 | 0.18 | 94.2 | 121.4 |
| A2 | 3.1547 | 0.357 | 8.84 | 0.28 | 92.8 | 98.4 |
| A3 | 2.7416 | 0.263 | 10.42 | 0.30 | 89.9 | 105.5 |
| A4 | 3.7211 | 0.455 | 8.19 | 0.21 | 96.8 | 102.5 |
| A5 | 2.2205 | 0.385 | 5.77 | 0.41 | 86.9 | 100.2 |
| A6 | 2.1416 | 0.417 | 5.14 | 0.44 | 106.5 | 100.5 |
| A7 | 1.8324 | 0.217 | 8.43 | 0.51 | 120.1 | 94.6 |
| A8 | 3.3145 | 0.500 | 6.63 | 0.23 | 81.8 | 120.8 |
| B1 | 1.0374 | 1.000 | 1.04 | 0.72 | 57.9 | 53.3 |
| B2 | 2.1234 | 0.385 | 5.52 | 0.34 | 91.4 | 94.4 |

[2] Evaluation of Oblate Ellipsoidal Polymer Particles

[Evaluation Test 1] Sensory Tests and Evaluation of Adhesion

[Reference Examples 1-1 to 1-7, Comparative Reference Examples 1-1 and 1-2]

Evaluations of feel, slip characteristics and particle adhesion were carried out by the methods described below on Polymer Particles A1 to A5 and B1 and B2. The results are shown in Table 3.

(1) Feel

The feel of each type of particle when spread over the skin was rated according to the criteria shown below.

(2) Slip Characteristics

One gram of each type of particle was placed on black synthetic leather and the length when spread with a finger was rated according to the criteria shown below.

(3) Particle Adhesion

One gram of each type of particle was placed on black synthetic leather and uniformly spread with a powder puff, following which the leather was struck three times and the amount of particles remaining was examined with a digital microscope (VHX200, from Keyence Corporation) and rated according to the criteria shown below.

◎: excellent
○: good

Δ: standard
×: unacceptable

[Table 3]

| | | Polymer particle | Feel | Slip | Particle adhesion |
|---|---|---|---|---|---|
| Reference Example | 1-1 | A1 | ○ | ○ | ◎ |
| | 1-2 | A2 | ◎ | ○ | ◎ |
| | 1-3 | A3 | ◎ | ○ | ◎ |
| | 1-4 | A4 | ◎ | ○ | ◎ |
| | 1-5 | A5 | ○ | ○ | ○ |
| | 1-6 | A1 + A2 (weight ratio: 5:5) | ◎ | ◎ | ◎ |
| | 1-7 | A3 + A5 (weight ratio: 5:5) | ◎ | ○ | ◎ |
| Comparative Reference Example | 1-1 | B1 | Δ | ○ | × |
| | 1-2 | B2 | Δ | ○ | ○ |

As shown in Table 3, with regard to feel and slip characteristics, the oblate ellipsoidal polymer particles were at least comparable to polymer particles lacking a flat region. With regard to particle adhesion, the oblate ellipsoidal polymer particles were far superior to polymer particles lacking a flat region.

[Evaluation Test 2] Transmitted Light Analysis

[Reference Examples 2-1 to 2-14, Comparative Reference Examples 2-1 and 2-2]

Polymer particles A1 to A5, polymer particles B1 to B2 and purified water were mixed together to give the compositions shown in Table 4 below, thereby preparing 0.1 wt% Polymer Particle Dispersions 1 to 16.

[Table 4]

| | | Polymer Particle Dispersion | Polymer particles (g) | | | | | | | Purified water (g) |
|---|---|---|---|---|---|---|---|---|---|---|
| | | | A1 | A2 | A3 | A4 | A5 | B1 | B2 | |
| Reference Example | 2-1 | 1 | 0.015 | - | - | - | - | - | - | 14.985 |
| | 2-2 | 2 | - | 0.015 | - | - | - | - | - | 14.985 |
| | 2-3 | 3 | - | - | 0.015 | - | - | - | - | 14.985 |
| | 2-4 | 4 | - | - | - | 0.015 | - | - | - | 14.985 |
| | 2-5 | 5 | - | - | - | - | 0.015 | - | - | 14.985 |
| | 2-6 | 6 | 0.01 | 0.005 | - | - | - | - | - | 14.985 |
| | 2-7 | 7 | 0.005 | - | 0.01 | - | - | - | - | 14.985 |
| | 2-8 | 8 | 0.005 | - | - | - | 0.01 | - | - | 14.985 |
| | 2-9 | 9 | 0.012 | - | - | - | - | 0.003 | - | 14.985 |
| | 2-10 | 10 | - | 0.012 | - | - | - | 0.003 | - | 14.985 |
| | 2-11 | 11 | - | - | 0.012 | - | - | 0.003 | - | 14.985 |
| | 2-12 | 12 | - | 0.012 | - | - | - | - | 0.003 | 14.985 |
| | 2-13 | 13 | - | - | 0.012 | - | - | - | 0.003 | 14.985 |
| | 2-14 | 14 | - | 0.009 | - | - | - | 0.003 | 0.003 | 14.985 |

(continued)

| | | Polymer Particle Dispersion | Polymer particles (g) | | | | | | | Purified water (g) |
|---|---|---|---|---|---|---|---|---|---|---|
| | | | A1 | A2 | A3 | A4 | A5 | B1 | B2 | |
| Comparative Reference Example | 2-1 | 15 | - | - | - | - | - | 0.015 | - | 14.985 |
| | 2-2 | 16 | - | - | - | - | - | - | 0.015 | 14.985 |

Polymer Particle Dispersions 1 to 16 were each poured into separate quartz cells (supplied with the following instrument) and, using a UV-visible spectrophotometer (UV-2450, from JASCO Corporation), transmitted light analysis during particle dispersion was carried out at wavelengths of 320 nm, 360 nm, 500 nm, 600 nm and 700 nm. The results are shown in Table 5.

[Table 5]

| | | Polymer particle dispersion | Transmittance (%) | | | | |
|---|---|---|---|---|---|---|---|
| | | | 320 nm | 360 nm | 500 nm | 600 nm | 700 nm |
| Reference Example | 2-1 | 1 | 0.1 | 0.2 | 0.3 | 0.4 | 0.6 |
| | 2-2 | 2 | 0.3 | 0.3 | 0.4 | 0.5 | 0.7 |
| | 2-3 | 3 | 0.4 | 0.5 | 0.6 | 0.7 | 0.9 |
| | 2-4 | 4 | 0.2 | 0.2 | 0.3 | 0.5 | 0.6 |
| | 2-5 | 5 | 0.2 | 0.3 | 0.4 | 0.6 | 0.8 |
| | 2-6 | 6 | 0.1 | 0.2 | 0.3 | 0.4 | 0.7 |
| | 2-7 | 7 | 0.2 | 0.3 | 0.4 | 0.6 | 0.8 |
| | 2-8 | 8 | 0.2 | 0.3 | 0.4 | 0.5 | 0.7 |
| | 2-9 | 9 | 1.1 | 1.3 | 1.6 | 2.3 | 2.7 |
| | 2-10 | 10 | 1.5 | 1.8 | 2.2 | 2.6 | 3.2 |
| | 2-11 | 11 | 1.8 | 2.0 | 2.3 | 3.0 | 3.5 |
| | 2-12 | 12 | 1.3 | 1.6 | 2.0 | 2.3 | 2.9 |
| | 2-13 | 13 | 1.6 | 1.8 | 2.5 | 2.8 | 3.3 |
| | 2-14 | 14 | 2.0 | 2.2 | 2.7 | 3.2 | 3.6 |
| Comparative Reference Example | 2-1 | 15 | 10.5 | 10.4 | 9.1 | 8.5 | 8.3 |
| | 2-2 | 16 | 2.9 | 3.2 | 4.4 | 5.8 | 7.2 |

As shown in Table 5, Dispersions 1 to 14 containing oblate ellipsoidal polymer particles demonstrated better light-diffusing effects than Dispersions 15 and 16 containing polymer particles without a flat region. Also, Dispersions 1 to 14 had a high light scattering effect from the UV region to the visible light region, and thus also had an excellent hiding ability.

[Evaluation Test 3] Evaluation of Reflected Light Scattering Properties

[Reference Examples 3-1 and 3-2, Comparative Reference Examples 3-1 and 3-2]

Test Sheets 1 to 4 were prepared by patting, and thereby uniformly applying (0.24 mg/cm$^2$), the polymer particles shown in Table 6 below onto black synthetic leather (5 cm × 8 cm) with a cosmetic powder puff. Next, using an automated goniophotometer (GP-200, from Murakami Color Research Laboratory Co., Ltd.), a fixed amount of light was irradiated onto the test sheet at an incident angle of 45° and the light scattering distribution of the reflected light was measured.

The results are shown in FIG. 6.

[Table 6]

| | | Test sheet | Polymer particles (wt%) | | |
|---|---|---|---|---|---|
| | | | A2 | B1 | B2 |
| Reference Example | 3-1 | 1 | 100 | - | - |
| | 3-2 | 2 | 90 | - | 10 |
| Comparative Reference Example | 3-1 | 3 | - | 100 | - |
| | 3-2 | 4 | - | - | 100 |

It was confirmed from FIG. 6 that oblate ellipsoidal polymer particles have a high light scattering effect. It was also confirmed that these particles are effective in applications where a UV-cutting ability is required in cosmetic preparations. When a plurality (n=5) of each type of test sheet were prepared and similar tests carried out, Test Sheet 1 showed somewhat more of a variability in properties among the individual sheets than did Test Sheet 2 which has a high scattering effect. The reason is thought to be that the flow properties of the oblate ellipsoidal polymer particles and the orienting properties of the flat sides of the particles give rise to variability in the light scattering effect. In other words, by mixing together a second type of polymer particle having a shape differing from that of the oblate ellipsoidal polymer particle (Reference Example 3-2), the second type of polymer particle has a sterically hindering effect, thus enabling a light scattering effect to be stably maintained without compromising the characteristics of the oblate ellipsoidal polymer particles.

[3] Production and Evaluation of Skin Cosmetics

[Evaluation Test 4] Powdery Foundations

[Examples 1 to 7, Comparative Examples 1 and 2]

Makeup compositions (Foundations 1 to 9) constituted as shown in Table 7 below were produced using Polymer Particles A1 to A4 and Polymer Particles B1 and B2.

[Table 7]

| | | Example | | | | | | | Comparative Example | |
|---|---|---|---|---|---|---|---|---|---|---|
| | | 1 | 2 | 3 | 4 | 5 | 6 | 7 | 1 | 2 |
| | | Foundation 1 | Foundation 2 | Foundation 3 | Foundation 4 | Foundation 5 | Foundation 6 | Foundation 7 | Foundation 8 | Foundation 9 |
| Ingredients (g) | Red iron oxide | 0.4 | 0.4 | 0.4 | 0.4 | 0.4 | 0.4 | 0.4 | 0.4 | 0.4 |
| | Yellow iron oxide | 1.0 | 1.0 | 1.0 | 1.0 | 1.0 | 1.0 | 1.0 | 1.0 | 1.0 |
| | Black iron oxide | 0.2 | 0.2 | 0.2 | 0.2 | 0.2 | 0.2 | 0.2 | 0.2 | 0.2 |
| | Titanium oxide | 7.0 | 7.0 | 7.0 | 7.0 | 7.0 | 7.0 | 7.0 | 7.0 | 7.0 |
| | Zinc oxide | 3.0 | 3.0 | 3.0 | 3.0 | 3.0 | 3.0 | 3.0 | 3.0 | 3.0 |
| | Silicone-treated large particle size titanium oxide | 3.0 | 3.0 | 3.0 | 3.0 | 3.0 | 3.0 | 3.0 | 3.0 | 3.0 |
| | Lauroyl lysine powder | 14.0 | 14.0 | 14.0 | 14.0 | 14.0 | 14.0 | 14.0 | 14.0 | 14.0 |
| | Titanium mica | 4.0 | 4.0 | 4.0 | 4.0 | 4.0 | 4.0 | 4.0 | 4.0 | 4.0 |
| | Talc | 35.97 | 35.97 | 35.97 | 35.97 | 35.97 | 35.97 | 35.97 | 35.97 | 35.97 |
| | Methyl phenyl polysiloxane | 2.0 | 2.0 | 2.0 | 2.0 | 2.0 | 2.0 | 2.0 | 2.0 | 2.0 |
| | Crystalline cellulose | 5.0 | 5.0 | 5.0 | 5.0 | 5.0 | 5.0 | 5.0 | 5.0 | 5.0 |
| | Cornstarch | 10.0 | 10.0 | 10.0 | 10.0 | 10.0 | 10.0 | 10.0 | 10.0 | 10.0 |
| | Methylparaben | 0.1 | 0.1 | 0.1 | 0.1 | 0.1 | 0.1 | 0.1 | 0.1 | 0.1 |
| | Sodium dehydroacetate | 0.1 | 0.1 | 0.1 | 0.1 | 0.1 | 0.1 | 0.1 | 0.1 | 0.1 |
| | Liquid paraffin | 1.5 | 1.5 | 1.5 | 1.5 | 1.5 | 1.5 | 1.5 | 1.5 | 1.5 |
| | Butylene glycol | 0.5 | 0.5 | 0.5 | 0.5 | 0.5 | 0.5 | 0.5 | 0.5 | 0.5 |
| | Coix seed extract | 0.1 | 0.1 | 0.1 | 0.1 | 0.1 | 0.1 | 0.1 | 0.1 | 0.1 |
| | Carrot extract | 0.1 | 0.1 | 0.1 | 0.1 | 0.1 | 0.1 | 0.1 | 0.1 | 0.1 |
| | Ubiquinone | 0.03 | 0.03 | 0.03 | 0.03 | 0.03 | 0.03 | 0.03 | 0.03 | 0.03 |
| | Polymer particle A1 | 12.0 | - | - | - | - | - | 6.0 | - | - |
| | Polymer particle A2 | - | 12.0 | - | - | 10.0 | - | 3.0 | - | - |
| | Polymer particle A3 | - | - | 12.0 | - | - | - | 3.0 | - | - |
| | Polymer particle A4 | - | - | - | 12.0 | - | 9.0 | - | - | - |
| | Polymer particle B1 | - | - | - | - | 2.0 | - | - | 12.0 | - |
| | Polymer particle B2 | - | - | - | - | - | 3.0 | - | - | 12.0 |

Fifteen people were selected as panelists, and the feel during use and difference before and after use for Foundations 1 to 9 were evaluated overall in terms of the following five qualities: "adherence to the skin," "sense of fit when applied," "sensation during use," "soft focus effect" and "durability of cosmetic effect (4 hours)," based on which the acceptability of the cosmetic formulations was assessed.

A: Foundation 1 was best
B: Foundation 2 was best
C: Foundation 3 was best
D: Foundation 4 was best
E: Foundation 5 was best
F: Foundation 6 was best
G: Foundation 7 was best
H: Foundation 8 was best
I: Foundation 9 was best
J: They were all the same

As a result, the assessments by the panelists were as follows:

| | |
|---|---|
| A: | 3 panelists |
| B: | 1 panelist |
| C: | 2 panelists |
| D: | 2 panelists |
| E: | 2 panelists |
| F: | 2 panelists |
| G: | 3 panelists |
| H: | 0 panelists |
| I: | 0 panelists |
| J: | 0 panelists |

Many of the panelists thought that Foundations 1, 2, 3, 4, 6 and 7 were particularly outstanding with respect to "adherence to the skin" and "durability of cosmetic effect (4 hours)." Many of the panelists thought that Foundations 1, 2, 3, 4, 5 and 6 were good in terms of the "soft focus effect." As for the "sense of fit when applied" and "sensation during use," Foundations 1, 2, 3, 4, 5 and 6 all had a good sensation, but panelist preferences led to differing views. What this appears to signify is that these are useful particles which, based on the oblate spheroidal shape and particle size, can be selected as appropriate for the product concept. Many of the panelists thought that Foundation 8 lacked "adherence to the skin," "durability of cosmetic effect (4 hours)" and a "soft focus effect." Finally, many of the panelists thought that Foundation 9 was better than Foundation 8, yet was inferior overall compared with Foundations 1 to 7.

[Evaluation Test 5] Liquid Foundations

[Examples 8 to 13, Comparative Examples 3 and 4]

Makeup compositions (Foundations 10 to 17) constituted as shown in Table 8 below were produced using Polymer Particles A1 to A3 and Polymer Particles B1 and B2.

[Table 8]

| | | Example | | | | | | Comparative Example | |
|---|---|---|---|---|---|---|---|---|---|
| | | 8 | 9 | 10 | 11 | 12 | 13 | 3 | 4 |
| | | Foundation 10 | Foundation 11 | Foundation 12 | Foundation 13 | Foundation 14 | Foundation 15 | Foundation 16 | Foundation 17 |
| Ingredients (g) | 2% Acrylic acid-alkyl methacrylate copolymer dispersion | 15 | 15 | 15 | 15 | 15 | 15 | 15 | 15 |
| | 2% Carboxyvinyl polymer dispersion | 15 | 15 | 15 | 15 | 15 | 15 | 15 | 15 |
| | Dipropylene glycol | 5 | 5 | 5 | 5 | 5 | 5 | 5 | 5 |
| | Disodium edetate | 0.05 | 0.05 | 0.05 | 0.05 | 0.05 | 0.05 | 0.05 | 0.05 |
| | Purified water | 2.7 | 2.7 | 2.7 | 2.7 | 2.7 | 2.7 | 2.7 | 2.7 |
| | Ethanol | 15 | 15 | 15 | 15 | 15 | 15 | 15 | 15 |
| | Sorbitan monoisostearate | 2 | 2 | 2 | 2 | 2 | 2 | 2 | 2 |
| | Polyoxyethylene (2) $C_{12-16}$ alkyl ether phosphate | 0.5 | 0.5 | 0.5 | 0.5 | 0.5 | 0.5 | 0.5 | 0.5 |
| | 2-Ethylhexyl hydroxystearate | 5 | 5 | 5 | 5 | 5 | 5 | 5 | 5 |
| | Methylcyclopolysiloxane | 5 | 5 | 5 | 5 | 5 | 5 | 5 | 5 |
| | 2-Amino-2-methyl-1-propanol | 0.5 | 0.5 | 0.5 | 0.5 | 0.5 | 0.5 | 0.5 | 0.5 |
| | Titanium oxide | 10 | 10 | 10 | 10 | 10 | 10 | 10 | 10 |
| | Red iron oxide | 0.2 | 0.2 | 0.2 | 0.2 | 0.2 | 0.2 | 0.2 | 0.2 |
| | Yellow iron oxide | 1 | 1 | 1 | 1 | 1 | 1 | 1 | 1 |
| | Black iron oxide | 0.1 | 0.1 | 0.1 | 0.1 | 0.1 | 0.1 | 0.1 | 0.1 |
| | Talc | 3.7 | 3.7 | 3.7 | 3.7 | 3.7 | 3.7 | 3.7 | 3.7 |
| | Crosslinked silicone powder | 1.5 | 1.5 | 1.5 | 1.5 | 1.5 | 1.5 | 1.5 | 1.5 |
| | 2% Xanthan gum dispersion | 15 | 15 | 15 | 15 | 15 | 15 | 15 | 15 |
| | Phenoxyethanol | 0.2 | 0.2 | 0.2 | 0.2 | 0.2 | 0.2 | 0.2 | 0.2 |
| | Sodium dehydroacetate | 0.05 | 0.05 | 0.05 | 0.05 | 0.05 | 0.05 | 0.05 | 0.05 |
| | Polymer particle A1 | 2.5 | - | - | - | 1.5 | - | - | - |
| | Polymer particle A2 | - | 2.5 | - | 1.5 | - | 2 | - | - |
| | Polymer particle A3 | - | - | 2.5 | 1 | 1 | - | - | - |
| | Polymer particle B1 | - | - | - | - | - | - | 2.5 | - |
| | Polymer particle B2 | - | - | - | - | - | 0.5 | - | 2.5 |

Fifteen people were selected as panelists, and the feel during use and difference before and after use for Foundations 10 to 17 were evaluated overall in terms of the following five qualities: "adherence to the skin," "sense of fit when applied," "sensation during use," "soft focus effect" and "durability of cosmetic effect (4 hours)," based on which the acceptability of the cosmetic formulations was assessed.

A: Foundation 10 was best
B: Foundation 11 was best
C: Foundation 12 was best
D: Foundation 13 was best
E: Foundation 14 was best
F: Foundation 15 was best
G: Foundation 16 was best
H: Foundation 17 was best

(continued)

| | |
|---|---|
| I: | They were all the same |

As a result, the assessments by the panelists were as follows:

| | |
|---|---|
| A: | 3 panelists |
| B: | 3 panelists |
| C: | 3 panelists |
| D: | 2 panelists |
| E: | 2 panelists |
| F: | 2 panelists |
| G: | 0 panelists |
| H: | 0 panelists |
| I: | 0 panelists |

Many of the panelists thought that Foundations 10 to 15 were particularly outstanding with respect to the "adherence to the skin" and "durability of cosmetic effect (4 hours)," and were good also with respect to the "soft focus effect." With regard to "sense of fit when applied" and "sensation during use," panelist preferences led to differing views for Foundations 10 to 15, but particles with a relatively high aspect ratio were thought to have a stronger impact in terms of sensation. Many of the panelists thought that Foundation 16 lacked "adherence to the skin," "durability of cosmetic effect (4 hours)" and a "soft focus effect." Finally, many of the panelists thought that Foundation 17 was better than Foundation 16, yet was inferior overall compared with Foundations 10 to 15.

The above results demonstrate that oblate ellipsoidal polymer particle A, while fully exploiting the weight-reducing effect of the polymer ingredients, has distinctive properties differing from conventional polymer particles in terms of, for example, their high adhesive properties and their hiding power, light-diffusing properties, UV-cutting properties, tactile qualities and flow properties, enabling them to be effectively used in skin cosmetics.

[4] Production of Various Skin Cosmetics

[Formulation Examples 1 to 120]

Given the large number of applications and products in cosmetics overall, particularly skin cosmetics, tests based on generally disclosed cosmetic formulations were conducted to determine whether it is possible to use oblate ellipsoidal polymer particle A in skin cosmetics. As a result, it was confirmed that oblate ellipsoidal polymer particle A can be handled in the same way as existing polymer particles and inorganic particles, and that the intrinsic properties of oblate ellipsoidal polymer particle A can be conferred to skin cosmetics.

Example formulations are presented below of skin cosmetics on which studies were carried out based on Polymer Particles A1 to A5, all of which are oblate ellipsoidal polymer particles. Using Polymer Particles A1 to A5, various skin cosmetics were produced having the compositions shown in Tables 9 to 33 below. Numbers in the tables indicate the content (wt%) of the ingredients.

(1) Powder Cosmetics

[Table 9]

| Powder Foundations | | Formulation Example 1 | Formulation Example 2 | Formulation Example 3 | Formulation Example 4 | Formulation Example 5 | Formulation Example 6 |
|---|---|---|---|---|---|---|---|
| Ingredients (wt%) | Silicone-treated red iron oxide | 1.0 | 1.0 | 1.0 | 1.0 | 1.0 | 1.0 |
| | Silicone-treated yellow iron oxide | 4.0 | 4.0 | 4.0 | 4.0 | 4.0 | 4.0 |
| | Silicone-treated black iron oxide | 0.5 | 0.5 | 0.5 | 0.5 | 0.5 | 0.5 |
| | Silicone-treated titanium oxide | 15.0 | 15.0 | 15.0 | 15.0 | 15.0 | 15.0 |
| | Silicone-treated talc | 27.97 | 27.97 | 27.97 | 27.97 | 27.97 | 27.97 |
| | Silicone-treated sericite | 5.0 | 5.0 | 5.0 | 5.0 | 5.0 | 5.0 |
| | Silicone-treated synthetic phlogopite | 5.0 | 5.0 | 5.0 | 5.0 | 5.0 | 5.0 |
| | Zinc myristate-treated zinc oxide/titanium oxide-coated mica | 18.0 | 18.0 | 18.0 | 18.0 | 18.0 | 18.0 |
| | Nylon powder | 8.0 | 8.0 | 8.0 | 8.0 | 8.0 | 8.0 |
| | Composite silicone resin powder | 2.0 | 2.0 | 2.0 | 2.0 | 2.0 | 2.0 |
| | Methylparaben | 0.1 | 0.1 | 0.1 | 0.1 | 0.1 | 0.1 |
| | Ethylparaben | 0.1 | 0.1 | 0.1 | 0.1 | 0.1 | 0.1 |
| | Sodium dehydroacetate | 0.1 | 0.1 | 0.1 | 0.1 | 0.1 | 0.1 |
| | Vaseline | 1.5 | 1.5 | 1.5 | 1.5 | 1.5 | 1.5 |
| | Methyl polysiloxane | 3.0 | 3.0 | 3.0 | 3.0 | 3.0 | 3.0 |
| | Isocetyl myristate | 1.5 | 1.5 | 1.5 | 1.5 | 1.5 | 1.5 |
| | Liquid paraffin | 2.0 | 2.0 | 2.0 | 2.0 | 2.0 | 2.0 |
| | 2-Ethylhexyl p-methoxycinnamate | 2.0 | 2.0 | 2.0 | 2.0 | 2.0 | 2.0 |
| | *Sambucus nigra* flower extract | 0.1 | 0.1 | 0.1 | 0.1 | 0.1 | 0.1 |
| | Bitter orange peel extract | 0.01 | 0.01 | 0.01 | 0.01 | 0.01 | 0.01 |
| | Olive extract | 0.01 | 0.01 | 0.01 | 0.01 | 0.01 | 0.01 |
| | St. John’s wort extract | 0.01 | 0.01 | 0.01 | 0.01 | 0.01 | 0.01 |
| | N-Methyl-L-serine | 0.1 | 0.1 | 0.1 | 0.1 | 0.1 | 0.1 |
| | Polymer Particle A1 | 3.0 | - | - | - | - | 2.0 |
| | Polymer Particle A2 | - | 3.0 | - | - | - | - |
| | Polymer Particle A3 | - | - | 3.0 | - | - | 1.0 |
| | Polymer Particle A4 | - | - | - | 3.0 | - | - |
| | Polymer Particle A5 | - | - | - | - | 3.0 | - |

[Table 10]

| Powder Foundations | | Formulation Example 7 | Formulation Example 8 | Formulation Example 9 | Formulation Example 10 | Formulation Example 11 | Formulation Example 12 |
|---|---|---|---|---|---|---|---|
| Ingredients (wt%) | Perfluorooctyltriethoxysilane-treated red iron oxide | 1.0 | 1.0 | 1.0 | 1.0 | 1.0 | 1.0 |
| | Perfluorooctyltriethoxysilane-treated yellow iron oxide | 4.0 | 4.0 | 4.0 | 4.0 | 4.0 | 4.0 |
| | Perfluorooctyltriethoxysilane-treated black iron oxide | 0.5 | 0.5 | 0.5 | 0.5 | 0.5 | 0.5 |
| | Perfluorooctyltriethoxysilane-treated titanium oxide | 10.0 | 10.0 | 10.0 | 10.0 | 10.0 | 10.0 |
| | Silicone-treated strongly aggregated titanium oxide | 8.0 | 8.0 | 8.0 | 8.0 | 8.0 | 8.0 |
| | Aluminum hydroxide/N-lauroyl lysine-treated acicular titanium oxide | 3.0 | 3.0 | 3.0 | 3.0 | 3.0 | 3.0 |
| | Silicone-treated talc | 21.3 | 21.3 | 21.3 | 21.3 | 21.3 | 21.3 |
| | Silicone-treated calcined talc/potassium fluorosilicate | 5.0 | 5.0 | 5.0 | 5.0 | 5.0 | 5.0 |
| | Boron nitride | 10.0 | 10.0 | 10.0 | 10.0 | 10.0 | 10.0 |
| | Perfluoroalkyl phosphate diethanolamine-treated mica | 5.0 | 5.0 | 5.0 | 5.0 | 5.0 | 5.0 |
| | Zinc myristate-treated zinc oxide/titanium oxide-coated mica | 10.0 | 10.0 | 10.0 | 10.0 | 10.0 | 10.0 |
| | Crosslinked silicone powder | 0.6 | 0.6 | 0.6 | 0.6 | 0.6 | 0.6 |
| | Sodium hyaluronate-treated silk powder | 1.0 | 1.0 | 1.0 | 1.0 | 1.0 | 1.0 |
| | Chlorphenesin | 0.2 | 0.2 | 0.2 | 0.2 | 0.2 | 0.2 |
| | Sodium dehydroacetate | 0.2 | 0.2 | 0.2 | 0.2 | 0.2 | 0.2 |
| | Hydrogenated castor oil isostearate | 1.0 | 1.0 | 1.0 | 1.0 | 1.0 | 1.0 |
| | Methyl polysiloxane | 4.0 | 4.0 | 4.0 | 4.0 | 4.0 | 4.0 |
| | Isocetyl myristate | 1.0 | 1.0 | 1.0 | 1.0 | 1.0 | 1.0 |
| | 2-Ethylhexyl p-methoxycinnamate | 2.0 | 2.0 | 2.0 | 2.0 | 2.0 | 2.0 |
| | Glyceryl tri(caprylate/caprate) | 2.0 | 2.0 | 2.0 | 2.0 | 2.0 | 2.0 |
| | Water-soluble collagen solution | 0.1 | 0.1 | 0.1 | 0.1 | 0.1 | 0.1 |
| | *Citrus unshiu* peel extract | 0.1 | 0.1 | 0.1 | 0.1 | 0.1 | 0.1 |
| | Spherical polymethyl methacrylate | 5.0 | 5.0 | 5.0 | 5.0 | 5.0 | 5.0 |
| | Polymer Particle A1 | 5.0 | - | - | - | - | 3.0 |
| | Polymer Particle A2 | - | 5.0 | - | - | - | - |
| | Polymer Particle A3 | - | - | 5.0 | - | - | 2.0 |
| | Polymer Particle A4 | - | - | - | 5.0 | - | - |
| | Polymer Particle A5 | - | - | - | - | 5.0 | - |

[Table 11]

| Powder Foundations | | Formulation Example 13 | Formulation Example 14 | Formulation Example 15 | Formulation Example 16 | Formulation Example 17 | Formulation Example 18 |
|---|---|---|---|---|---|---|---|
| Ingredients (wt%) | N-Lauroyl lysine-treated red iron oxide | 1.0 | 1.0 | 1.0 | 1.0 | 1.0 | 1.0 |
| | N-Lauroyl lysine-treated yellow iron oxide | 4.0 | 4.0 | 4.0 | 4.0 | 4.0 | 4.0 |
| | N-Lauroyl lysine-treated black iron oxide | 0.5 | 0.5 | 0.5 | 0.5 | 0.5 | 0.5 |
| | N-Lauroyl lysine-treated titanium oxide | 7.0 | 7.0 | 7.0 | 7.0 | 7.0 | 7.0 |
| | N-Lauroyl lysine-treated strongly aggregated titanium oxide | 7.0 | 7.0 | 7.0 | 7.0 | 7.0 | 7.0 |
| | Hydrated silica/aluminum oxide silicone-treated finely divided titanium oxide | 2.0 | 2.0 | 2.0 | 2.0 | 2.0 | 2.0 |
| | N-Lauroyl lysine-treated sericite | 30.06 | 30.06 | 30.06 | 30.06 | 30.06 | 30.06 |
| | Zinc myristate-treated synthetic phlogopite | 15.0 | 15.0 | 15.0 | 15.0 | 15.0 | 15.0 |
| | Boron nitride | 5.0 | 5.0 | 5.0 | 5.0 | 5.0 | 5.0 |
| | Silicone-treated hemp cellulose powder | 10.0 | 10.0 | 10.0 | 10.0 | 10.0 | 10.0 |
| | Chlorphenesin | 0.1 | 0.1 | 0.1 | 0.1 | 0.1 | 0.1 |
| | Sodium dehydroacetate | 0.1 | 0.1 | 0.1 | 0.1 | 0.1 | 0.1 |
| | Methyl polysiloxane | 4.0 | 4.0 | 4.0 | 4.0 | 4.0 | 4.0 |
| | Diisostearyl malate | 1.5 | 1.5 | 1.5 | 1.5 | 1.5 | 1.5 |
| | Glyceryl tri(2-ethylhexanoate) | 2.0 | 2.0 | 2.0 | 2.0 | 2.0 | 2.0 |
| | Glyceryl tri(caprylate caprate myristate stearate) | 0.5 | 0.5 | 0.5 | 0.5 | 0.5 | 0.5 |
| | 2-Ethylhexyl p-methoxycinnamate | 3.0 | 3.0 | 3.0 | 3.0 | 3.0 | 3.0 |
| | Water-soluble collagen solution | 0.1 | 0.1 | 0.1 | 0.1 | 0.1 | 0.1 |
| | Orchid extract | 0.01 | 0.01 | 0.01 | 0.01 | 0.01 | 0.01 |
| | Royal jelly extract | 0.01 | 0.01 | 0.01 | 0.01 | 0.01 | 0.01 |
| | Hydrolyzed conchiolin solution | 0.01 | 0.01 | 0.01 | 0.01 | 0.01 | 0.01 |
| | *Rubus suavissimus* extract | 0.01 | 0.01 | 0.01 | 0.01 | 0.01 | 0.01 |
| | Silk extract | 0.1 | 0.1 | 0.1 | 0.1 | 0.1 | 0.1 |
| | Urethane powder | 2.0 | 2.0 | 2.0 | 2.0 | 2.0 | 2.0 |
| | Polymer Particle A1 | 5.0 | - | - | - | - | - |
| | Polymer Particle A2 | - | 5.0 | - | - | - | 2.5 |
| | Polymer Particle A3 | - | - | 5.0 | - | - | 2.5 |
| | Polymer Particle A4 | - | - | - | 5.0 | - | - |
| | Polymer Particle A5 | - | - | - | - | 5.0 | - |

[Table 12]

| | Cover-Type Solid Face Powders | Formulation Example 19 | Formulation Example 20 | Formulation Example 21 | Formulation Example 22 | Formulation Example 23 | Formulation Example 24 |
|---|---|---|---|---|---|---|---|
| Ingredients (wt%) | Silicone-treated red iron oxide | 0.3 | 0.3 | 0.3 | 0.3 | 0.3 | 0.3 |
| | Silicone-treated yellow iron oxide | 0.5 | 0.5 | 0.5 | 0.5 | 0.5 | 0.5 |
| | Silicone-treated black iron oxide | 0.05 | 0.05 | 0.05 | 0.05 | 0.05 | 0.05 |
| | Silicone-treated titanium oxide | 2.0 | 2.0 | 2.0 | 2.0 | 2.0 | 2.0 |
| | Silicone-treated zinc oxide | 2.0 | 2.0 | 2.0 | 2.0 | 2.0 | 2.0 |
| | Silicone-treated strongly aggregated titanium oxide | 3.0 | 3.0 | 3.0 | 3.0 | 3.0 | 3.0 |
| | Sintered (iron oxide/titanium oxide) | 1.0 | 1.0 | 1.0 | 1.0 | 1.0 | 1.0 |
| | Fluorine-treated zinc oxide/titanium oxide-coated mica | 5.0 | 5.0 | 5.0 | 5.0 | 5.0 | 5.0 |
| | Silica/titanium oxide-coated sericite | 5.0 | 5.0 | 5.0 | 5.0 | 5.0 | 5.0 |
| | Silk powder | 1.0 | 1.0 | 1.0 | 1.0 | 1.0 | 1.0 |
| | N-Lauroyl lysine-treated barium sulfate | 35.0 | 35.0 | 35.0 | 35.0 | 35.0 | 35.0 |
| | Silicone-treated talc | 34.75 | 34.75 | 34.75 | 34.75 | 34.75 | 34.75 |
| | Methylparaben | 0.1 | 0.1 | 0.1 | 0.1 | 0.1 | 0.1 |
| | Sodium dehydroacetate | 0.1 | 0.1 | 0.1 | 0.1 | 0.1 | 0.1 |
| | Vaseline | 1.0 | 1.0 | 1.0 | 1.0 | 1.0 | 1.0 |
| | Octyl dodecyl lactate | 1.0 | 1.0 | 1.0 | 1.0 | 1.0 | 1.0 |
| | Glyceryl tri(2-ethylhexanoate) | 2.0 | 2.0 | 2.0 | 2.0 | 2.0 | 2.0 |
| | Isononyl isononanoate | 2.0 | 2.0 | 2.0 | 2.0 | 2.0 | 2.0 |
| | Octyl dodecanol | 1.0 | 1.0 | 1.0 | 1.0 | 1.0 | 1.0 |
| | Licorice flavonoids | 0.1 | 0.1 | 0.1 | 0.1 | 0.1 | 0.1 |
| | Disodium L-ascorbyl sulfate | 0.1 | 0.1 | 0.1 | 0.1 | 0.1 | 0.1 |
| | Polymer Particle A1 | 3.0 | - | - | - | - | - |
| | Polymer Particle A2 | - | 3.0 | - | - | - | 2.0 |
| | Polymer Particle A3 | - | - | 3.0 | - | - | - |
| | Polymer Particle A4 | - | - | - | 3.0 | - | - |
| | Polymer Particle A5 | - | - | - | - | 3.0 | 1.0 |

[Table 13]

| | Powder Foundations | Formulation Example 25 | Formulation Example 26 | Formulation Example 27 | Formulation Example 28 | Formulation Example 29 | Formulation Example 30 |
|---|---|---|---|---|---|---|---|
| Ingredients (wt%) | Organotitanate-treated red iron oxide | 0.4 | 0.4 | 0.4 | 0.4 | 0.4 | 0.4 |
| | Organotitanate-treated yellow iron oxide | 1.0 | 1.0 | 1.0 | 1.0 | 1.0 | 1.0 |
| | Organotitanate-treated black iron oxide | 0.2 | 0.2 | 0.2 | 0.2 | 0.2 | 0.2 |
| | Organotitanate-treated titanium oxide | 5.0 | 5.0 | 5.0 | 5.0 | 5.0 | 5.0 |
| | Organotitanate-treated zinc oxide | 3.0 | 3.0 | 3.0 | 3.0 | 3.0 | 3.0 |
| | Aluminum oxide/silicone-treated titanium oxide | 2.0 | 2.0 | 2.0 | 2.0 | 2.0 | 2.0 |
| | Silicone-treated large particle-size titanium oxide | 4.0 | 4.0 | 4.0 | 4.0 | 4.0 | 4.0 |
| | N-Lauroyl lysine powder | 15.0 | 15.0 | 15.0 | 15.0 | 15.0 | 15.0 |
| | Titanium mica | 4.0 | 4.0 | 4.0 | 4.0 | 4.0 | 4.0 |
| | Talc | 35.97 | 35.97 | 35.97 | 35.97 | 35.97 | 35.97 |
| | Microcrystalline cellulose | 5.0 | 5.0 | 5.0 | 5.0 | 5.0 | 5.0 |
| | Cornstarch | 10.0 | 10.0 | 10.0 | 10.0 | 10.0 | 10.0 |
| | Methylparaben | 0.1 | 0.1 | 0.1 | 0.1 | 0.1 | 0.1 |
| | Sodium dehydroacetate | 0.1 | 0.1 | 0.1 | 0.1 | 0.1 | 0.1 |
| | Liquid paraffin | 1.5 | 1.5 | 1.5 | 1.5 | 1.5 | 1.5 |
| | Methyl phenyl polysiloxane | 2.0 | 2.0 | 2.0 | 2.0 | 2.0 | 2.0 |
| | 1,3-Butylene glycol | 0.5 | 0.5 | 0.5 | 0.5 | 0.5 | 0.5 |
| | Coix seed extract | 0.1 | 0.1 | 0.1 | 0.1 | 0.1 | 0.1 |
| | Carrot extract | 0.1 | 0.1 | 0.1 | 0.1 | 0.1 | 0.1 |
| | Ubiquinone | 0.03 | 0.03 | 0.03 | 0.03 | 0.03 | 0.03 |
| | Polymer Particle A1 | 10.0 | - | - | - | - | - |
| | Polymer Particle A2 | - | 10.0 | - | - | - | 3.0 |
| | Polymer Particle A3 | - | - | 10.0 | - | - | 2.0 |
| | Polymer Particle A4 | - | - | - | 10.0 | - | - |
| | Polymer Particle A5 | - | - | - | - | 10.0 | 5.0 |

[Table 14]

| Loose Face Powders | | Formulation Example 31 | Formulation Example 32 | Formulation Example 33 | Formulation Example 34 | Formulation Example 35 | Formulation Example 36 |
|---|---|---|---|---|---|---|---|
| Ingredients (wt%) | Red iron oxide | 0.2 | 0.2 | 0.2 | 0.2 | 0.2 | 0.2 |
| | Yellow iron oxide | 0.1 | 0.1 | 0.1 | 0.1 | 0.1 | 0.1 |
| | Ultramarine | 0.05 | 0.05 | 0.05 | 0.05 | 0.05 | 0.05 |
| | Branched silicone/methyl hydrogen polysiloxane-treated titanium oxide | 2.0 | 2.0 | 2.0 | 2.0 | 2.0 | 2.0 |
| | Acicular titanium oxide | 3.0 | 3.0 | 3.0 | 3.0 | 3.0 | 3.0 |
| | Silicone-treated finely divided zinc oxide | 1.0 | 1.0 | 1.0 | 1.0 | 1.0 | 1.0 |
| | Fluorine compound-treated synthetic phlogopite | 5.0 | 5.0 | 5.0 | 5.0 | 5.0 | 5.0 |
| | Silicone-treated mica | 10.0 | 10.0 | 10.0 | 10.0 | 10.0 | 10.0 |
| | Talc | 67.65 | 67.65 | 67.65 | 67.65 | 67.65 | 67.65 |
| | Silica | 0.3 | 0.3 | 0.3 | 0.3 | 0.3 | 0.3 |
| | Methylparaben | 0.1 | 0.1 | 0.1 | 0.1 | 0.1 | 0.1 |
| | Ethylparaben | 0.1 | 0.1 | 0.1 | 0.1 | 0.1 | 0.1 |
| | Liquid paraffin | 1.5 | 1.5 | 1.5 | 1.5 | 1.5 | 1.5 |
| | 2-Ethylhexyl p-methoxycinnamate | 2.0 | 2.0 | 2.0 | 2.0 | 2.0 | 2.0 |
| | Urethane powder | 5.0 | 5.0 | 5.0 | 5.0 | 5.0 | 5.0 |
| | Polymer Particle A1 | 2.0 | - | - | - | - | 1.0 |
| | Polymer Particle A2 | - | 2.0 | - | - | - | - |
| | Polymer Particle A3 | - | - | 2.0 | - | - | 1.0 |
| | Polymer Particle A4 | - | - | - | 2.0 | - | - |
| | Polymer Particle A5 | - | - | - | - | 2.0 | - |

(2) Oil-Based Cosmetics, Cosmetic Gels

[Table 15]

| Foundations | | Formulation Example 37 | Formulation Example 38 | Formulation Example 39 | Formulation Example 40 | Formulation Example 41 |
|---|---|---|---|---|---|---|
| Ingredients (wt%) | Red iron oxide | 1.5 | 1.5 | 1.5 | 1.5 | 1.5 |
| | Yellow iron oxide | 2.0 | 2.0 | 2.0 | 2.0 | 2.0 |
| | Black iron oxide | 0.5 | 0.5 | 0.5 | 0.5 | 0.5 |
| | Titanium oxide | 8.0 | 8.0 | 8.0 | 8.0 | 8.0 |
| | Silicone-treated strongly aggregated titanium oxide | 3.0 | 3.0 | 3.0 | 3.0 | 3.0 |
| | Mica | 3.3 | 3.3 | 3.3 | 3.3 | 3.3 |
| | 1,3-Butylene glycol | 3.0 | 3.0 | 3.0 | 3.0 | 3.0 |
| | Octyl glycoside | 1.0 | 1.0 | 1.0 | 1.0 | 1.0 |
| | Candelilla wax | 4.0 | 4.0 | 4.0 | 4.0 | 4.0 |
| | Paraffin wax | 8.0 | 8.0 | 8.0 | 8.0 | 8.0 |
| | Vaseline | 10.0 | 10.0 | 10.0 | 10.0 | 10.0 |
| | Cetanol | 2.0 | 2.0 | 2.0 | 2.0 | 2.0 |
| | 2-Hexyldecanol | 5.0 | 5.0 | 5.0 | 5.0 | 5.0 |
| | Glyceryl trioctanoate | 20.0 | 20.0 | 20.0 | 20.0 | 20.0 |
| | Isononyl isononanoate | 5.0 | 5.0 | 5.0 | 5.0 | 5.0 |
| | Liquid paraffin | 10.0 | 10.0 | 10.0 | 10.0 | 10.0 |
| | Methyl phenyl polysiloxane | 3.0 | 3.0 | 3.0 | 3.0 | 3.0 |
| | Stearyl glycyrrhetinate | 0.1 | 0.1 | 0.1 | 0.1 | 0.1 |
| | Tocopherol acetate | 0.1 | 0.1 | 0.1 | 0.1 | 0.1 |
| | Perfume | suitable amount | suitable amount | suitable amount | suitable amount | suitable amount |
| | Nylon powder | 5.0 | 5.0 | 5.0 | 5.0 | 5.0 |
| | Polymethylsilsesquioxane | 3.0 | 3.0 | 3.0 | 3.0 | 3.0 |
| | Urethane powder | 0.5 | 0.5 | 0.5 | 0.5 | 0.5 |
| | Polymer Particle A1 | 2.0 | - | - | - | 1.5 |
| | Polymer Particle A2 | - | 2.0 | - | - | 0.5 |
| | Polymer Particle A3 | - | - | 2.0 | - | - |
| | Polymer Particle A5 | - | - | - | 2.0 | - |

[Table 16]

| | Foundations | Formulation Example 42 | Formulation Example 43 | Formulation Example 44 | Formulation Example 45 | Formulation Example 46 |
|---|---|---|---|---|---|---|
| Ingredients (wt%) | Organotitanate-treated red iron oxide | 0.2 | 0.2 | 0.2 | 0.2 | 0.2 |
| | Organotitanate-treated yellow iron oxide | 0.5 | 0.5 | 0.5 | 0.5 | 0.5 |
| | Organotitanate-treated black iron oxide | 0.05 | 0.05 | 0.05 | 0.05 | 0.05 |
| | Organotitanate-treated titanium oxide | 10.0 | 10.0 | 10.0 | 10.0 | 10.0 |
| | Silicic acid | 5.0 | 5.0 | 5.0 | 5.0 | 5.0 |
| | Silicone-treated finely divided zinc oxide | 2.0 | 2.0 | 2.0 | 2.0 | 2.0 |
| | Zinc oxide/Titanium oxide-coated mica | 5.0 | 5.0 | 5.0 | 5.0 | 5.0 |
| | Sorbitan monoisostearate | 1.0 | 1.0 | 1.0 | 1.0 | 1.0 |
| | Purified water | 15.0 | 15.0 | 15.0 | 15.0 | 15.0 |
| | Low-melting paraffin | 20.0 | 20.0 | 20.0 | 20.0 | 20.0 |
| | Isocetyl myristate | 34.85 | 34.85 | 34.85 | 34.85 | 34.85 |
| | 2-Ethylhexyl p-methoxycinnamate | 1.0 | 1.0 | 1.0 | 1.0 | 1.0 |
| | Water-soluble collagen | 0.1 | 0.1 | 0.1 | 0.1 | 0.1 |
| | Methylparaben | 0.2 | 0.2 | 0.2 | 0.2 | 0.2 |
| | Phenoxyethanol | 0.1 | 0.1 | 0.1 | 0.1 | 0.1 |
| | Polymer Particle A1 | 5.0 | - | - | - | 3.0 |
| | Polymer Particle A2 | - | 5.0 | - | - | - |
| | Polymer Particle A3 | - | - | 5.0 | - | 2.0 |
| | Polymer Particle A5 | - | - | - | 5.0 | - |

[Table 17]

| Concealers | | Formulation Example 47 | Formulation Example 48 | Formulation Example 49 | Formulation Example 50 | Formulation Example 51 |
|---|---|---|---|---|---|---|
| Ingredients (wt%) | Octyltriethoxysilane-treated red iron oxide | 1.5 | 1.5 | 1.5 | 1.5 | 1.5 |
| | Octyltriethoxysilane-treated yellow iron oxide | 2.0 | 2.0 | 2.0 | 2.0 | 2.0 |
| | Octyltriethoxysilane-treated black iron oxide | 0.5 | 0.5 | 0.5 | 0.5 | 0.5 |
| | Octyltriethoxysilane-treated titanium oxide | 10.0 | 10.0 | 10.0 | 10.0 | 10.0 |
| | Strongly aggregated titanium oxide | 6.0 | 6.0 | 6.0 | 6.0 | 6.0 |
| | Hydrated silica/aluminum hydroxide-treated finely divided titanium oxide | 2.0 | 2.0 | 2.0 | 2.0 | 2.0 |
| | (Dimethicone/vinyl dimethicone) crosspolymer dimethicone paste | 1.5 | 1.5 | 1.5 | 1.5 | 1.5 |
| | Octyltriethoxysilane/N-lauroyl lysine-treated platy barium sulfate | 4.0 | 4.0 | 4.0 | 4.0 | 4.0 |
| | Polyamide-modified silicone | 16.0 | 16.0 | 16.0 | 16.0 | 16.0 |
| | Trimethylsiloxysilicic acid/ methyl trimethicone solution | 1.0 | 1.0 | 1.0 | 1.0 | 1.0 |
| | Branched silicone-type polyglyceryl-modified silicone | 0.2 | 0.2 | 0.2 | 0.2 | 0.2 |
| | Cyclomethicone | 7.0 | 7.0 | 7.0 | 7.0 | 7.0 |
| | Methyl trimethicone | 2.0 | 2.0 | 2.0 | 2.0 | 2.0 |
| | Methyl phenyl polysiloxane | 20.0 | 20.0 | 20.0 | 20.0 | 20.0 |
| | 2-Ethylhexyl p-methoxycinnamate | 2.0 | 2.0 | 2.0 | 2.0 | 2.0 |
| | Isononyl Isononanoate | 15.4 | 15.4 | 15.4 | 15.4 | 15.4 |
| | Dipropylene glycol | 0.4 | 0.4 | 0.4 | 0.4 | 0.4 |
| | Composite silicone resin powder | 0.5 | 0.5 | 0.5 | 0.5 | 0.5 |
| | Polymer Particle A1 | 8.0 | - | - | - | 2.0 |
| | Polymer Particle A2 | - | 8.0 | - | - | 1.0 |
| | Polymer Particle A3 | - | - | 8.0 | - | 1.0 |
| | Polymer Particle A5 | - | - | - | 8.0 | 4.0 |

[Table 18]

| Lipsticks | | Formulation Example 52 | Formulation Example 53 | Formulation Example 54 | Formulation Example 55 | Formulation Example 56 |
|---|---|---|---|---|---|---|
| Ingredients (wt%) | Paraffin wax | 3.0 | 3.0 | 3.0 | 3.0 | 3.0 |
| | Synthetic hydrocarbon wax | 3.0 | 3.0 | 3.0 | 3.0 | 3.0 |
| | Ethylene-propylene copolymer | 3.0 | 3.0 | 3.0 | 3.0 | 3.0 |
| | Ceresin | 3.0 | 3.0 | 3.0 | 3.0 | 3.0 |
| | Microcrystalline wax | 3.0 | 3.0 | 3.0 | 3.0 | 3.0 |
| | Vaseline | 5.0 | 5.0 | 5.0 | 5.0 | 5.0 |
| | Heavy liquid isoparaffin | 25.0 | 25.0 | 25.0 | 25.0 | 25.0 |
| | Di(octyldodecyl/phytosteryl/behenyl) lauroyl glutamate | 10.0 | 10.0 | 10.0 | 10.0 | 10.0 |
| | Polyglyceryl triisostearate | 5.0 | 5.0 | 5.0 | 5.0 | 5.0 |
| | Octyl dodecanol | 5.0 | 5.0 | 5.0 | 5.0 | 5.0 |
| | Squalane | 5.0 | 5.0 | 5.0 | 5.0 | 5.0 |
| | Hydrogenated polydecene | 5.0 | 5.0 | 5.0 | 5.0 | 5.0 |
| | Glyceryl Tri(caprylate/caprate) | 13.6 | 13.6 | 13.6 | 13.6 | 13.6 |
| | Tocopherol | 0.1 | 0.1 | 0.1 | 0.1 | 0.1 |
| | Red No. 201 | 0.1 | 0.1 | 0.1 | 0.1 | 0.1 |
| | Red No. 202 | 0.1 | 0.1 | 0.1 | 0.1 | 0.1 |
| | Acicular titanium oxide | 1.0 | 1.0 | 1.0 | 1.0 | 1.0 |
| | Iron oxide | 0.5 | 0.5 | 0.5 | 0.5 | 0.5 |
| | Silica | 3.0 | 3.0 | 3.0 | 3.0 | 3.0 |
| | Mica | 2.0 | 2.0 | 2.0 | 2.0 | 2.0 |
| | Titanium mica | 1.5 | 1.5 | 1.5 | 1.5 | 1.5 |
| | Titanium oxide-coated glass flakes | 2.0 | 2.0 | 2.0 | 2.0 | 2.0 |
| | Raspberry extract | 0.1 | 0.1 | 0.1 | 0.1 | 0.1 |
| | Polymer Particle A1 | 1.0 | - | - | - | 0.5 |
| | Polymer Particle A2 | - | 1.0 | - | - | 0.5 |
| | Polymer Particle A3 | - | - | 1.0 | - | - |
| | Polymer Particle A5 | - | - | - | 1.0 | - |

[Table 19]

| | Lip coats | Formulation Example 57 | Formulation Example 58 | Formulation Example 59 |
|---|---|---|---|---|
| Ingredients (wt%) | Dextrin palmitate | 10.0 | 10.0 | 10.0 |
| | Heavy liquid isoparaffin | 50.0 | 50.0 | 50.0 |
| | Di(phytosteryl 2-octyl dodecyl) lauroyl glutamate | 10.0 | 10.0 | 10.0 |
| | Polyglyceryl diisostearate | 10.0 | 10.0 | 10.0 |
| | Diisostearyl malate | 14.3 | 14.3 | 14.3 |
| | Tocopherol | 0.1 | 0.1 | 0.1 |
| | Red No. 218 | 0.1 | 0.1 | 0.1 |
| | Yellow No. 4 aluminum lake | 0.5 | 0.5 | 0.5 |
| | Synthetic phlogopite | 1.0 | 1.0 | 1.0 |
| | Acicular titanium oxide | 0.5 | 0.5 | 0.5 |
| | Titanium oxide-coated glass flakes | 3.0 | 3.0 | 3.0 |
| | Polymer Particle A1 | 0.5 | - | - |
| | Polymer Particle A2 | - | 0.5 | - |
| | Polymer Particle A3 | - | - | 0.5 |

[Table 20]

| | Mascaras | Formulation Example 60 | Formulation Example 61 | Formulation Example 62 | Formulation Example 63 | Formulation Example 64 |
|---|---|---|---|---|---|---|
| Ingredients (wt%) | Light isoparaffin | 31.8 | 31.8 | 31.8 | 31.8 | 31.8 |
| | Dimethyl polysiloxane | 3.0 | 3.0 | 3.0 | 3.0 | 3.0 |
| | Decamethyl cyclopentane siloxane | 5.0 | 5.0 | 5.0 | 5.0 | 5.0 |
| | Octyl palmitate | 1.0 | 1.0 | 1.0 | 1.0 | 1.0 |
| | Isostearic acid | 1.0 | 1.0 | 1.0 | 1.0 | 1.0 |
| | Microcrystalline wax | 1.0 | 1.0 | 1.0 | 1.0 | 1.0 |
| | Carnauba wax | 2.0 | 2.0 | 2.0 | 2.0 | 2.0 |
| | Beeswax | 2.0 | 2.0 | 2.0 | 2.0 | 2.0 |
| | Dextrin (palmitate/octanoate) | 15.0 | 15.0 | 15.0 | 15.0 | 15.0 |
| | Sucrose mixed fatty acid esters | 20.0 | 20.0 | 20.0 | 20.0 | 20.0 |
| | Sucrose tetraisostearate | 10.0 | 10.0 | 10.0 | 10.0 | 10.0 |
| | Trimethylsiloxysilicic acid | 5.0 | 5.0 | 5.0 | 5.0 | 5.0 |
| | Aluminum stearate | 1.0 | 1.0 | 1.0 | 1.0 | 1.0 |
| | Silicone-coated pigment (iron oxide) | 0.5 | 0.5 | 0.5 | 0.5 | 0.5 |
| | Silicone-coated pigment (titanium oxide) | 0.3 | 0.3 | 0.3 | 0.3 | 0.3 |
| | Silicone-coated pigment (red iron oxide) | 0.2 | 0.2 | 0.2 | 0.2 | 0.2 |
| | Barium sulfate | 0.1 | 0.1 | 0.1 | 0.1 | 0.1 |
| | Red iron oxide-coated mica | suitable amount | suitable amount | suitable amount | suitable amount | suitable amount |
| | Tocopherol | 0.1 | 0.1 | 0.1 | 0.1 | 0.1 |
| | Polymer Particle A1 | 1.0 | - | - | - | 0.5 |
| | Polymer Particle A2 | - | 1.0 | - | - | - |
| | Polymer Particle A3 | - | - | 1.0 | - | 0.5 |
| | Polymer Particle A5 | - | - | - | 1.0 | - |

(3) Oil-in-Water Emulsified Cosmetics

[Table 21]

| Foundations | | Formulation Example 65 | Formulation Example 66 | Formulation Example 67 | Formulation Example 68 |
|---|---|---|---|---|---|
| Ingredients (wt%) | 2% Acrylic acid-alkyl methacrylate copolymer dispersion | 15.0 | 15.0 | 15.0 | 15.0 |
| | 2% Carboxyvinyl polymer dispersion | 15.0 | 15.0 | 15.0 | 15.0 |
| | Dipropylene glycol | 5.0 | 5.0 | 5.0 | 5.0 |
| | Disodium edetate | 0.05 | 0.05 | 0.05 | 0.05 |
| | Purified water | 2.7 | 2.7 | 2.7 | 2.7 |
| | Ethanol | 15.0 | 15.0 | 15.0 | 15.0 |
| | Sorbitan monoisostearate | 2.0 | 2.0 | 2.0 | 2.0 |
| | Polyoxyethylene (2) $C_{12-16}$ alkyl ether phosphate | 0.5 | 0.5 | 0.5 | 0.5 |
| | 2-Ethylhexyl hydroxystearate | 5.0 | 5.0 | 5.0 | 5.0 |
| | Methyl cyclopolysiloxane | 5.0 | 5.0 | 5.0 | 5.0 |
| | 2-Amino-2-methyl-1-propanol | 0.5 | 0.5 | 0.5 | 0.5 |
| | Titanium oxide | 10.0 | 10.0 | 10.0 | 10.0 |
| | Red iron oxide | 0.2 | 0.2 | 0.2 | 0.2 |
| | Yellow iron oxide | 1.0 | 1.0 | 1.0 | 1.0 |
| | Black iron oxide | 0.1 | 0.1 | 0.1 | 0.1 |
| | Talc | 3.7 | 3.7 | 3.7 | 3.7 |
| | 2% Xanthan gum dispersion | 15.0 | 15.0 | 15.0 | 15.0 |
| | Phenoxyethanol | 0.2 | 0.2 | 0.2 | 0.2 |
| | Sodium dehydroacetate | 0.05 | 0.05 | 0.05 | 0.05 |
| | Crosslinked silicone powder | 2.0 | 2.0 | 2.0 | 2.0 |
| | Polymer Particle A1 | 2.0 | - | - | 1.0 |
| | Polymer Particle A2 | - | 2.0 | - | 1.0 |
| | Polymer Particle A3 | - | - | 2.0 | - |

[Table 22]

| Foundations | | Formulation Example 69 | Formulation Example 70 | Formulation Example 71 | Formulation Example 72 |
|---|---|---|---|---|---|
| Ingredients (wt%) | 2% Carboxyvinyl polymer solution (aq.) | 30.0 | 30.0 | 30.0 | 30.0 |
| | Concentrated glycerin | 5.0 | 5.0 | 5.0 | 5.0 |
| | Maltitol solution | 2.0 | 2.0 | 2.0 | 2.0 |
| | Disodium edetate | 0.05 | 0.05 | 0.05 | 0.05 |
| | Olive leaf extract | 0.1 | 0.1 | 0.1 | 0.1 |
| | Peony root extract | 0.1 | 0.1 | 0.1 | 0.1 |
| | St. John's wort extract | 0.1 | 0.1 | 0.1 | 0.1 |
| | Purified water | 8.0 | 8.0 | 8.0 | 8.0 |
| | Ethanol | 15.0 | 15.0 | 15.0 | 15.0 |
| | Sorbitan monoisostearate | 1.5 | 1.5 | 1.5 | 1.5 |
| | Sorbitan diisostearate | 1.0 | 1.0 | 1.0 | 1.0 |
| | 2-Ethylhexyl hydroxystearate | 5.0 | 5.0 | 5.0 | 5.0 |
| | Propylene glycol dicaprylate | 3.0 | 3.0 | 3.0 | 3.0 |
| | 2-Amino-2-methyl-1-propanol | 0.5 | 0.5 | 0.5 | 0.5 |
| | 10% Silicic anhydride-coated titanium oxide | 10.0 | 10.0 | 10.0 | 10.0 |
| | 10% Silicic anhydride-coated red iron oxide | 0.3 | 0.3 | 0.3 | 0.3 |
| | 10% Silicic anhydride-coated yellow iron oxide | 1.0 | 1.0 | 1.0 | 1.0 |
| | 10% Silicic anhydride-coated black iron oxide | 0.1 | 0.1 | 0.1 | 0.1 |
| | 2% Xanthan gum solution (aq.) | 10.0 | 10.0 | 10.0 | 10.0 |
| | Phenoxyethanol | 0.2 | 0.2 | 0.2 | 0.2 |
| | Sodium dehydroacetate | 0.05 | 0.05 | 0.05 | 0.05 |
| | Crosslinked silicone powder dispersion (aq.) | 5.0 | 5.0 | 5.0 | 5.0 |
| | Polymer Particle A1 | 2.0 | - | - | - |
| | Polymer Particle A2 | - | 2.0 | - | 1.5 |
| | Polymer Particle A3 | - | - | 2.0 | 0.5 |

[Table 23]

| | Makeup Bases | Formulation Example 73 | Formulation Example 74 | Formulation Example 75 | Formulation Example 76 | Formulation Example 77 |
|---|---|---|---|---|---|---|
| Ingredients (wt%) | 2% Acrylic acid-alkyl methacrylate copolymer dispersion | 5.0 | 5.0 | 5.0 | 5.0 | 5.0 |
| | Glycerin | 5.0 | 5.0 | 5.0 | 5.0 | 5.0 |
| | Disodium edetate | 0.05 | 0.05 | 0.05 | 0.05 | 0.05 |
| | Purified water | 74.0 | 74.0 | 74.0 | 74.0 | 74.0 |
| | Ethanol | 5.0 | 5.0 | 5.0 | 5.0 | 5.0 |
| | Sorbitan monoisostearate | 0.5 | 0.5 | 0.5 | 0.5 | 0.5 |
| | 2-Ethylhexyl p-methoxycinnamate | 3.0 | 3.0 | 3.0 | 3.0 | 3.0 |
| | Diethylaminohydroxybenzoyl hexyl benzoate | 0.5 | 0.5 | 0.5 | 0.5 | 0.5 |
| | Silicone-treated finely divided titanium oxide | 0.5 | 0.5 | 0.5 | 0.5 | 0.5 |
| | Silicone-treated finely divided zinc oxide | 0.5 | 0.5 | 0.5 | 0.5 | 0.5 |
| | Triethanolamine | 0.1 | 0.1 | 0.1 | 0.1 | 0.1 |
| | 2% Xanthan gum solution (aq.) | 5.0 | 5.0 | 5.0 | 5.0 | 5.0 |
| | Water-soluble collagen | 0.1 | 0.1 | 0.1 | 0.1 | 0.1 |
| | Phenoxyethanol | 0.2 | 0.2 | 0.2 | 0.2 | 0.2 |
| | Sodium dehydroacetate | 0.05 | 0.05 | 0.05 | 0.05 | 0.05 |
| | Polymer Particle A1 | 0.5 | - | - | - | 0.3 |
| | Polymer Particle A2 | - | 0.5 | - | - | - |
| | Polymer Particle A3 | - | - | 0.5 | - | 0.2 |
| | Polymer Particle A4 | - | - | - | 0.5 | - |

[Table 24]

| Pore-Correcting Makeup Bases | | Formulation Example 78 | Formulation Example 79 | Formulation Example 80 | Formulation Example 81 |
|---|---|---|---|---|---|
| Ingredients (wt%) | 2% Carboxyvinyl polymer solution (aq.) | 3.0 | 3.0 | 3.0 | 3.0 |
| | 2% Alkyl-modified carboxyvinyl polymer solution (aq.) | 3.0 | 3.0 | 3.0 | 3.0 |
| | Dipropylene glycol | 3.0 | 3.0 | 3.0 | 3.0 |
| | Disodium edetate | 0.05 | 0.05 | 0.05 | 0.05 |
| | *Camellia sinensis* extract | 0.1 | 0.1 | 0.1 | 0.1 |
| | Elderberry extract | 0.1 | 0.1 | 0.1 | 0.1 |
| | Purified water | 14.6 | 14.6 | 14.6 | 14.6 |
| | Ethanol | 10.0 | 10.0 | 10.0 | 10.0 |
| | 2-Ethylhexyl p-methoxycinnamate | 7.0 | 7.0 | 7.0 | 7.0 |
| | Diethylaminohydroxybenzoyl hexyl benzoate | 1.0 | 1.0 | 1.0 | 1.0 |
| | Methyl polysiloxane | 4.0 | 4.0 | 4.0 | 4.0 |
| | Sorbitan monoisostearate | 1.0 | 1.0 | 1.0 | 1.0 |
| | 2-Amino-2-methyl-1-propanol | 0.15 | 0.15 | 0.15 | 0.15 |
| | Methyl siloxane network polymer | 8.0 | 8.0 | 8.0 | 8.0 |
| | 2% Xanthan gum solution (aq.) | 15.0 | 15.0 | 15.0 | 15.0 |
| | Crosslinked silicone powder dispersion (aq.) | 25.0 | 25.0 | 25.0 | 25.0 |
| | Polymer Particle A1 | 5.0 | - | - | 2.0 |
| | Polymer Particle A2 | - | 5.0 | - | 2.0 |
| | Polymer Particle A3 | - | - | 5.0 | 1.0 |

[Table 25]

| | Mascaras | Formulation Example 82 | Formulation Example 83 | Formulation Example 84 |
|---|---|---|---|---|
| Ingredients (wt%) | Polyacrylic acid ester emulsion | 50.0 | 50.0 | 50.0 |
| | Polymethacrylic acid | 2.0 | 2.0 | 2.0 |
| | Triethanolamine | 2.5 | 2.5 | 2.5 |
| | Butylene glycol | 3.0 | 3.0 | 3.0 |
| | Sodium chloride | 0.5 | 0.5 | 0.5 |
| | Preservative | suitable amount | suitable amount | suitable amount |
| | Silica | 2.0 | 2.0 | 2.0 |
| | Pigment | 1.0 | 1.0 | 1.0 |
| | Perfume | 0.1 | 0.1 | 0.1 |
| | Purified water | 33.9 | 33.9 | 33.9 |
| | Nylon powder | 4.0 | 4.0 | 4.0 |
| | Polymer Particle A1 | 1.0 | - | - |
| | Polymer Particle A2 | - | 1.0 | - |
| | Polymer Particle A3 | - | - | 1.0 |

[Table 26]

| Mascaras | | Formulation Example 85 | Formulation Example 86 | Formulation Example 87 | Formulation Example 88 |
|---|---|---|---|---|---|
| Ingredients (wt%) | Purified water | 43.9 | 43.9 | 43.9 | 43.9 |
| | Bentonite | 1.5 | 1.5 | 1.5 | 1.5 |
| | Phenoxyethanol | 0.5 | 0.5 | 0.5 | 0.5 |
| | Hydroxyethylcellulose | 2.0 | 2.0 | 2.0 | 2.0 |
| | Sericite | 0.5 | 0.5 | 0.5 | 0.5 |
| | PEG-20 glyceryl stearate | 5.0 | 5.0 | 5.0 | 5.0 |
| | Butylene glycol | 6.0 | 6.0 | 6.0 | 6.0 |
| | Boron nitride | 12.0 | 12.0 | 12.0 | 12.0 |
| | Iron oxide | 2.0 | 2.0 | 2.0 | 2.0 |
| | Titanium oxide | 1.0 | 1.0 | 1.0 | 1.0 |
| | Triethanolamine | 1.0 | 1.0 | 1.0 | 1.0 |
| | Carnauba wax | 1.0 | 1.0 | 1.0 | 1.0 |
| | Beeswax | 6.5 | 6.5 | 6.5 | 6.5 |
| | Cetanol | 2.0 | 2.0 | 2.0 | 2.0 |
| | Stearic acid | 2.0 | 2.0 | 2.0 | 2.0 |
| | Polyisobutene | 1.0 | 1.0 | 1.0 | 1.0 |
| | Tocopherol acetate | 0.1 | 0.1 | 0.1 | 0.1 |
| | Acrylates copolymer | 9.0 | 9.0 | 9.0 | 9.0 |
| | Polymer Particle A1 | 3.0 | - | - | 1.0 |
| | Polymer Particle A2 | - | 3.0 | - | 1.0 |
| | Polymer Particle A3 | - | - | 3.0 | 1.0 |

(4) Water-in-Oil Emulsified Cosmetics

[Table 27]

| | Water-in-Oil Foundations | Formulation Example 89 | Formulation Example 90 | Formulation Example 91 | Formulation Example 92 | Formulation Example 93 |
|---|---|---|---|---|---|---|
| Ingredients (wt%) | Diglyceryl monoisostearate | 1.0 | 1.0 | 1.0 | 1.0 | 1.0 |
| | Polyether-modified silicone mixture | 5.0 | 5.0 | 5.0 | 5.0 | 5.0 |
| | Volatile silicone | 15.0 | 15.0 | 15.0 | 15.0 | 15.0 |
| | Isododecane | 5.0 | 5.0 | 5.0 | 5.0 | 5.0 |
| | Dipentaerythrityl tripolyhydroxystearate | 3.0 | 3.0 | 3.0 | 3.0 | 3.0 |
| | Liquid paraffin | 6.0 | 6.0 | 6.0 | 6.0 | 6.0 |
| | Soybean lecithin | 0.1 | 0.1 | 0.1 | 0.1 | 0.1 |
| | Propylene glycol dicaprylate | 4.0 | 4.0 | 4.0 | 4.0 | 4.0 |
| | Glycerin | 3.0 | 3.0 | 3.0 | 3.0 | 3.0 |
| | Maltitol | 3.0 | 3.0 | 3.0 | 3.0 | 3.0 |
| | Carrot extract | 0.1 | 0.1 | 0.1 | 0.1 | 0.1 |
| | *Sambucus nigra* flower extract | 0.1 | 0.1 | 0.1 | 0.1 | 0.1 |
| | Octyltriethoxysilane-treated titanium oxide | 10.0 | 10.0 | 10.0 | 10.0 | 10.0 |
| | Octyltriethoxysilane-treated red iron oxide | 0.2 | 0.2 | 0.2 | 0.2 | 0.2 |
| | Octyltriethoxysilane-treated yellow iron oxide | 0.8 | 0.8 | 0.8 | 0.8 | 0.8 |
| | Octyltriethoxysilane-treated black iron oxide | 0.1 | 0.1 | 0.1 | 0.1 | 0.1 |
| | Talc | 0.9 | 0.9 | 0.9 | 0.9 | 0.9 |
| | Purified water | 40.85 | 40.85 | 40.85 | 40.85 | 40.85 |
| | Sodium dehydroacetate | 0.05 | 0.05 | 0.05 | 0.05 | 0.05 |
| | Phenoxyethanol | 0.3 | 0.3 | 0.3 | 0.3 | 0.3 |
| | Crosslinked silicone powder | 0.5 | 0.5 | 0.5 | 0.5 | 0.5 |
| | Polymer Particle A1 | 1.0 | - | - | - | 0.8 |
| | Polymer Particle A2 | - | 1.0 | - | - | 0.2 |
| | Polymer Particle A3 | - | - | 1.0 | - | - |
| | Polymer Particle A5 | - | - | - | 1.0 | - |

[Table 28]

| Foundations | | Formulation Example 94 | Formulation Example 95 | Formulation Example 96 | Formulation Example 97 |
|---|---|---|---|---|---|
| Ingredients (wt%) | Crosslinked polyether-modified silicone mixture | 10.0 | 10.0 | 10.0 | 10.0 |
| | Branched polyether-modified silicone | 1.0 | 1.0 | 1.0 | 1.0 |
| | Volatile silicone (cyclopentasiloxane) | 10.0 | 10.0 | 10.0 | 10.0 |
| | Dimethyl silicone | 2.0 | 2.0 | 2.0 | 2.0 |
| | Lauroyl lysine-treated titanium oxide | 8.0 | 8.0 | 8.0 | 8.0 |
| | Lauroyl lysine-treated red iron oxide | 0.2 | 0.2 | 0.2 | 0.2 |
| | Lauroyl lysine-treated yellow iron oxide | 0.7 | 0.7 | 0.7 | 0.7 |
| | Lauroyl lysine-treated black iron oxide | 0.1 | 0.1 | 0.1 | 0.1 |
| | Lauroyl lysine-treated talc | 1.0 | 1.0 | 1.0 | 1.0 |
| | Dipropylene glycol | 2.0 | 2.0 | 2.0 | 2.0 |
| | Maltitol | 2.0 | 2.0 | 2.0 | 2.0 |
| | Honeysuckle extract | 0.1 | 0.1 | 0.1 | 0.1 |
| | Sodium citrate | 0.5 | 0.5 | 0.5 | 0.5 |
| | Methylparaben | 0.2 | 0.2 | 0.2 | 0.2 |
| | Phenoxyethanol | 0.2 | 0.2 | 0.2 | 0.2 |
| | Purified water | 60.0 | 60.0 | 60.0 | 60.0 |
| | Polymer Particle A1 | 2.0 | - | - | 1.5 |
| | Polymer Particle A2 | - | 2.0 | - | - |
| | Polymer Particle A3 | - | - | 2.0 | 0.5 |

[Table 29]

| | Makeup Bases | Formulation Example 98 | Formulation Example 99 | Formulation Example 100 | Formulation Example 101 |
|---|---|---|---|---|---|
| Ingredients (wt%) | Branched polyether-modified silicone | 2.0 | 2.0 | 2.0 | 2.0 |
| | Dimethyl silicone | 5.0 | 5.0 | 5.0 | 5.0 |
| | Volatile silicone | 11.0 | 11.0 | 11.0 | 11.0 |
| | Polypropylene silsesquioxane/cyclopentasiloxane mixture | 3.0 | 3.0 | 3.0 | 3.0 |
| | Octyl p-methoxycinnamate | 2.0 | 2.0 | 2.0 | 2.0 |
| | Dipropylene glycol | 2.0 | 2.0 | 2.0 | 2.0 |
| | Glycerin | 1.0 | 1.0 | 1.0 | 1.0 |
| | Sodium citrate | 0.5 | 0.5 | 0.5 | 0.5 |
| | Olive extract | 0.1 | 0.1 | 0.1 | 0.1 |
| | Purified water | 50.7 | 50.7 | 50.7 | 50.7 |
| | Silicone-treated titanium oxide | 2.0 | 2.0 | 2.0 | 2.0 |
| | Silicone-treated zinc oxide | 2.0 | 2.0 | 2.0 | 2.0 |
| | Silicone-treated red iron oxide | 0.4 | 0.4 | 0.4 | 0.4 |
| | Silicone-treated yellow iron oxide | 1.5 | 1.5 | 1.5 | 1.5 |
| | Silicone-treated black iron oxide | 0.2 | 0.2 | 0.2 | 0.2 |
| | Silicone-treated talc | 5.9 | 5.9 | 5.9 | 5.9 |
| | Ethanol | 10.0 | 10.0 | 10.0 | 10.0 |
| | Phenoxyethanol | 0.2 | 0.2 | 0.2 | 0.2 |
| | Polymer particle A1 | 0.5 | - | - | 0.2 |
| | Polymer particle A2 | - | 0.5 | - | 0.3 |
| | Polymer particle A3 | - | - | 0.5 | - |

[Table 30]

| Solid Foundations | | Formulation Example 102 | Formulation Example 103 | Formulation Example 104 | Formulation Example 105 |
|---|---|---|---|---|---|
| Ingredients (wt%) | Sorbitan monoisostearate | 2.0 | 2.0 | 2.0 | 2.0 |
| | Polyether-modified silicone | 1.0 | 1.0 | 1.0 | 1.0 |
| | Volatile silicone (cyclopentasiloxane) | 10.0 | 10.0 | 10.0 | 10.0 |
| | Volatile silicone (cyclohexasiloxane) | 15.0 | 15.0 | 15.0 | 15.0 |
| | Dimethyl silicone | 3.0 | 3.0 | 3.0 | 3.0 |
| | Propylene glycol dicaprylate | 2.0 | 2.0 | 2.0 | 2.0 |
| | Plant-derived squalane | 1.0 | 1.0 | 1.0 | 1.0 |
| | Paraffin | 5.0 | 5.0 | 5.0 | 5.0 |
| | Ceresin | 2.0 | 2.0 | 2.0 | 2.0 |
| | Vaseline | 1.0 | 1.0 | 1.0 | 1.0 |
| | Dipropylene glycol | 3.0 | 3.0 | 3.0 | 3.0 |
| | Maltitol solution | 3.0 | 3.0 | 3.0 | 3.0 |
| | Orange fruit juice | 1.0 | 1.0 | 1.0 | 1.0 |
| | Phellodendron bark extract | 1.0 | 1.0 | 1.0 | 1.0 |
| | Methylparaben | 0.2 | 0.2 | 0.2 | 0.2 |
| | Purified water | 22.8 | 22.8 | 22.8 | 22.8 |
| | Titanium oxide | 15.0 | 15.0 | 15.0 | 15.0 |
| | Red iron oxide | 0.4 | 0.4 | 0.4 | 0.4 |
| | Yellow iron oxide | 1.4 | 1.4 | 1.4 | 1.4 |
| | Black iron oxide | 1.2 | 1.2 | 1.2 | 1.2 |
| | Talc | 5.0 | 5.0 | 5.0 | 5.0 |
| | Polyethylene powder | 1.0 | 1.0 | 1.0 | 1.0 |
| | Polymer particle A1 | 3.0 | - | - | 1.0 |
| | Polymer particle A2 | - | 3.0 | - | 1.0 |
| | Polymer particle A3 | - | - | 3.0 | 1.0 |

[Table 31]

| Sunscreens | | Formulation Example 106 | Formulation Example 107 | Formulation Example 108 | Formulation Example 109 | Formulation Example 110 |
|---|---|---|---|---|---|---|
| Ingredients (wt%) | Sorbitan monoisostearate | 1.0 | 1.0 | 1.0 | 1.0 | 1.0 |
| | Polyether-modified silicone mixture | 5.0 | 5.0 | 5.0 | 5.0 | 5.0 |
| | Volatile silicone | 15.0 | 15.0 | 15.0 | 15.0 | 15.0 |
| | Isohexadecane | 5.0 | 5.0 | 5.0 | 5.0 | 5.0 |
| | Di(phytosteryl isostearyl cetyl stearyl behenyl) dimer dilinoleate | 0.2 | 0.2 | 0.2 | 0.2 | 0.2 |
| | Octyl p-methoxycinnamate | 5.0 | 5.0 | 5.0 | 5.0 | 5.0 |
| | Diethylaminohydroxybenzoyl hexyl benzoate | 1.0 | 1.0 | 1.0 | 1.0 | 1.0 |
| | Squalane | 3.0 | 3.0 | 3.0 | 3.0 | 3.0 |
| | Purified water | 43.6 | 43.6 | 43.6 | 43.6 | 43.6 |
| | Ethanol | 10.0 | 10.0 | 10.0 | 10.0 | 10.0 |
| | Finely divided titanium oxide | 3.0 | 3.0 | 3.0 | 3.0 | 3.0 |
| | Finely divided zinc oxide | 3.0 | 3.0 | 3.0 | 3.0 | 3.0 |
| | Titanium oxide | 1.0 | 1.0 | 1.0 | 1.0 | 1.0 |
| | Trimethylsiloxysilicic acid | 3.0 | 3.0 | 3.0 | 3.0 | 3.0 |
| | Phenoxyethanol | 0.2 | 0.2 | 0.2 | 0.2 | 0.2 |
| | Polymer Particle A1 | 1.0 | - | - | - | - |
| | Polymer Particle A2 | - | 1.0 | - | - | - |
| | Polymer Particle A3 | - | - | 1.0 | - | 0.5 |
| | Polymer Particle A5 | - | - | - | 1.0 | 0.5 |

[Table 32]

| Solid Wrinkle Concealers | | Formulation Example 111 | Formulation Example 112 | Formulation Example 113 | Formulation Example 114 | Formulation Example 115 |
|---|---|---|---|---|---|---|
| Ingredients (wt%) | Sorbitan monoisostearate | 1.5 | 1.5 | 1.5 | 1.5 | 1.5 |
| | Polyether-modified silicone mixture | 0.5 | 0.5 | 0.5 | 0.5 | 0.5 |
| | Volatile silicone | 40.0 | 40.0 | 40.0 | 40.0 | 40.0 |
| | Dipentaerythrityl tripolyhydroxystearate | 5.0 | 5.0 | 5.0 | 5.0 | 5.0 |
| | Dimethylpolysiloxane | 7.0 | 7.0 | 7.0 | 7.0 | 7.0 |
| | Liquid paraffin | 5.0 | 5.0 | 5.0 | 5.0 | 5.0 |
| | Ceresin | 2.0 | 2.0 | 2.0 | 2.0 | 2.0 |
| | Rosehip oil | 0.1 | 0.1 | 0.1 | 0.1 | 0.1 |
| | Olive squalane | 0.1 | 0.1 | 0.1 | 0.1 | 0.1 |
| | Titanium oxide | 0.5 | 0.5 | 0.5 | 0.5 | 0.5 |
| | Glycerin | 4.0 | 4.0 | 4.0 | 4.0 | 4.0 |
| | Maltitol | 3.0 | 3.0 | 3.0 | 3.0 | 3.0 |
| | Methylparaben | 0.05 | 0.05 | 0.05 | 0.05 | 0.05 |
| | Sorbic acid | 0.05 | 0.05 | 0.05 | 0.05 | 0.05 |
| | Purified water | 16.2 | 16.2 | 16.2 | 16.2 | 16.2 |
| | Polymethylsilsesquioxane | 5.0 | 5.0 | 5.0 | 5.0 | 5.0 |
| | Crosslinked silicone powder | 5.0 | 5.0 | 5.0 | 5.0 | 5.0 |
| | Polymer Particle A1 | 5.0 | - | - | - | - |
| | Polymer Particle A2 | - | 5.0 | - | - | 2.0 |
| | Polymer Particle A3 | - | - | 5.0 | - | 1.0 |
| | Polymer Particle A5 | - | - | - | 5.0 | 2.0 |

[Table 33]

| | Mascaras | Formulation Example 116 | Formulation Example 117 | Formulation Example 118 | Formulation Example 119 | Formulation Example 120 |
|---|---|---|---|---|---|---|
| Ingredients (wt%) | Hydrogenated polyisobutene | 47.0 | 47.0 | 47.0 | 47.0 | 47.0 |
| | Cyclomethicone | 10.5 | 10.5 | 10.5 | 10.5 | 10.5 |
| | Dextrin palmitate | 7.0 | 7.0 | 7.0 | 7.0 | 7.0 |
| | Sucrose acetate stearate | 5.0 | 5.0 | 5.0 | 5.0 | 5.0 |
| | Disteardimonium hectorite | 5.0 | 5.0 | 5.0 | 5.0 | 5.0 |
| | Tri(trimethylsiloxy)silylpropylcarbamic acid pullulan | 4.5 | 4.5 | 4.5 | 4.5 | 4.5 |
| | Candelilla wax | 4.0 | 4.0 | 4.0 | 4.0 | 4.0 |
| | PEG-10 dimethicone | 3.0 | 3.0 | 3.0 | 3.0 | 3.0 |
| | Butylene glycol | 1.5 | 1.5 | 1.5 | 1.5 | 1.5 |
| | Purified water | 1.5 | 1.5 | 1.5 | 1.5 | 1.5 |
| | PEG-20 hydrogenated castor oil triisostearate | 1.0 | 1.0 | 1.0 | 1.0 | 1.0 |
| | Hydrophobized pigment | 5.0 | 5.0 | 5.0 | 5.0 | 5.0 |
| | Nylon fibers (1 to 3 mm) | 3.0 | 3.0 | 3.0 | 3.0 | 3.0 |
| | Polymer Particle A1 | 2.0 | - | - | - | - |
| | Polymer Particle A2 | - | 2.0 | - | - | - |
| | Polymer Particle A3 | - | - | 2.0 | - | 1.0 |
| | Polymer Particle A5 | - | - | - | 2.0 | 1.0 |

## Claims

1. A skin cosmetic comprising from 0.1 to 50 wt% of polymer particles that include an oblate ellipsoidal polymer particle A which has, in projections based on the third-angle projection method, a front view, a plan view and a side view that are all elliptical, and which satisfies conditions (1) to (3) below pertaining to a flat region of the particle:

   (1) the flat region has a length L whose average $L_{AV}$ is such that 0.13 μm ≤ $L_{AV}$ ≤ 500 μm,
   (2) the flat region has a breadth D whose average $D_{AV}$ is such that 0.1 μm ≤ $D_{AV}$ ≤ 250 μm, and
   (3) the aspect ratio L/D calculated from the length L and breadth D has an average value $P^1_{AV}$ such that 1.3 < $P^1_{AV}$ ≤ 50.

2. The skin cosmetic of claim 1, wherein the oblate ellipsoidal polymer particle A further satisfies at least one of conditions (4) and (5) below:

   (4) the aspect ratio D/T calculated from the breadth D and a lateral thickness T of the particle has an average value $P^2_{AV}$ such that 1.2 < $P^2_{AV}$ ≤ 100, and
   (5) the aspect ratio L/T calculated from the length L and the lateral thickness T of the particle has an average value $P^3_{AV}$ such that 1.56 < $P^3_{AV}$ ≤ 150.

3. The skin cosmetic of claim 1 or 2 which includes two or more types of oblate ellipsoidal polymer particle A.

4. The skin cosmetic of any one of claims 1 to 3, wherein the polymer particles further include at least one type of polymer particle B having a shape different from oblate ellipsoidal polymer particle A.

**5.** The skin cosmetic of claim 4, wherein polymer particle B has a volume mean particle size of $L_{AV}$ or less.

**6.** The skin cosmetic of claim 4 or 5, wherein polymer particle B is spherical, spheroidal or ellipsoidal.

**7.** The skin cosmetic of any one of claims 4 to 6, wherein the weight ratio of oblate ellipsoidal polymer particle A to polymer particle B is from 99:1 to 10:90.

**8.** The skin cosmetic of any one of claims 1 to 7, further comprising inorganic particles.

**9.** The skin cosmetic of claim 8, wherein the inorganic particles have a shape that is plate-like or flake-like.

**10.** The skin cosmetic of any one of claims 1 to 9, further comprising an oil-based ingredient.

FIG.1

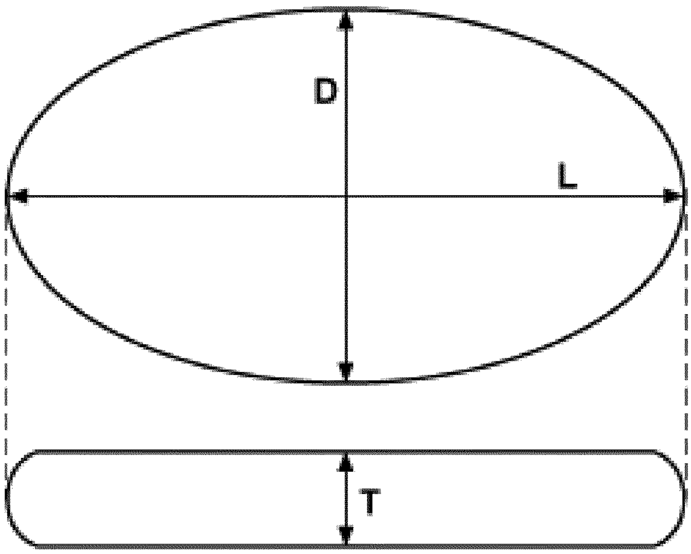


FIG.2

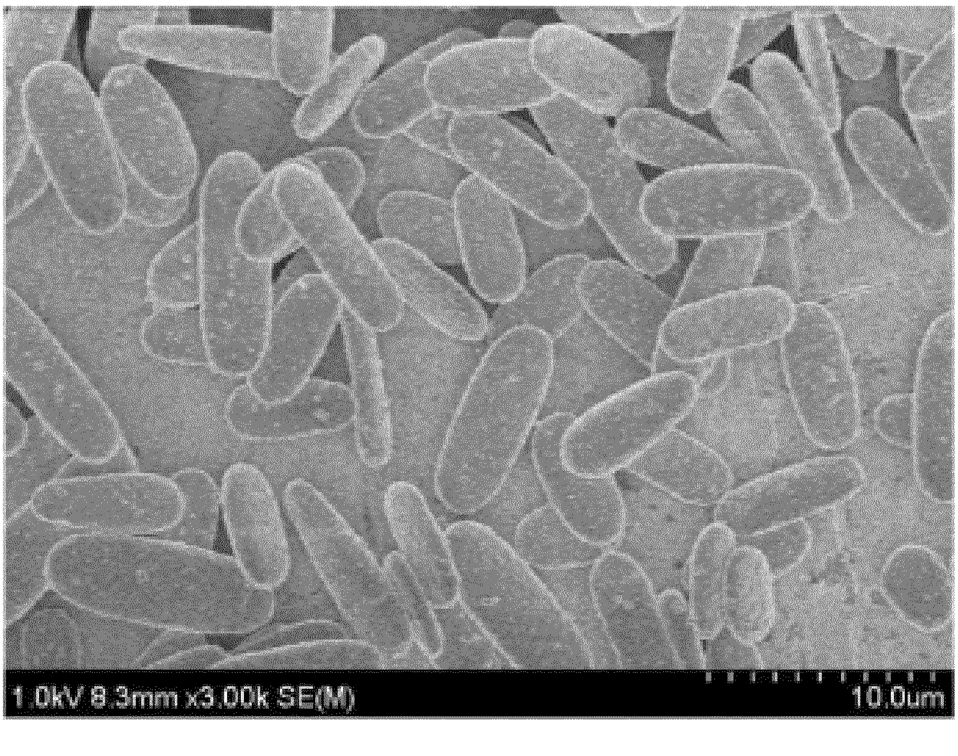

FIG.3

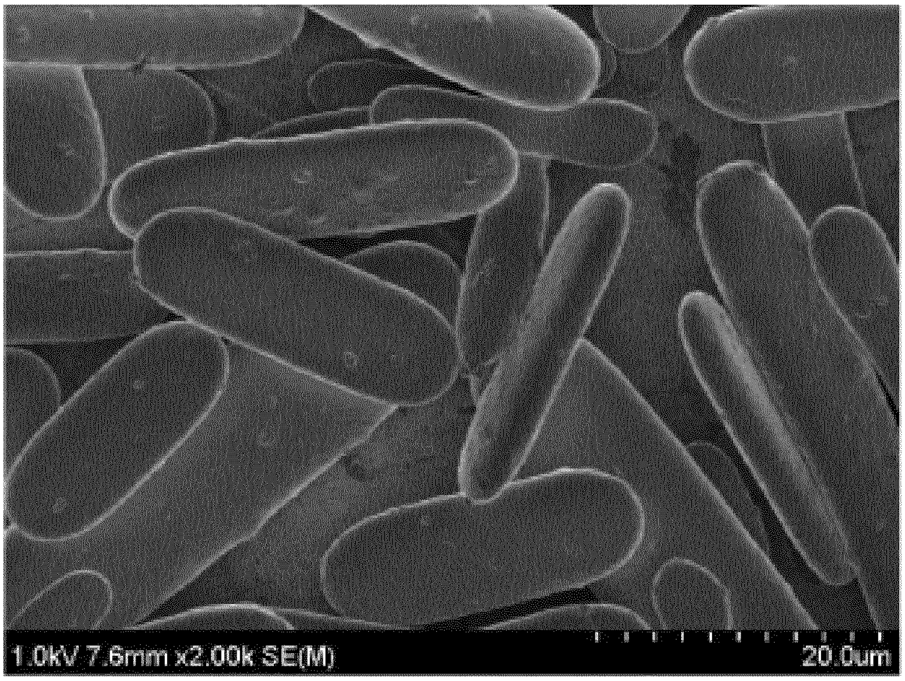


FIG.4

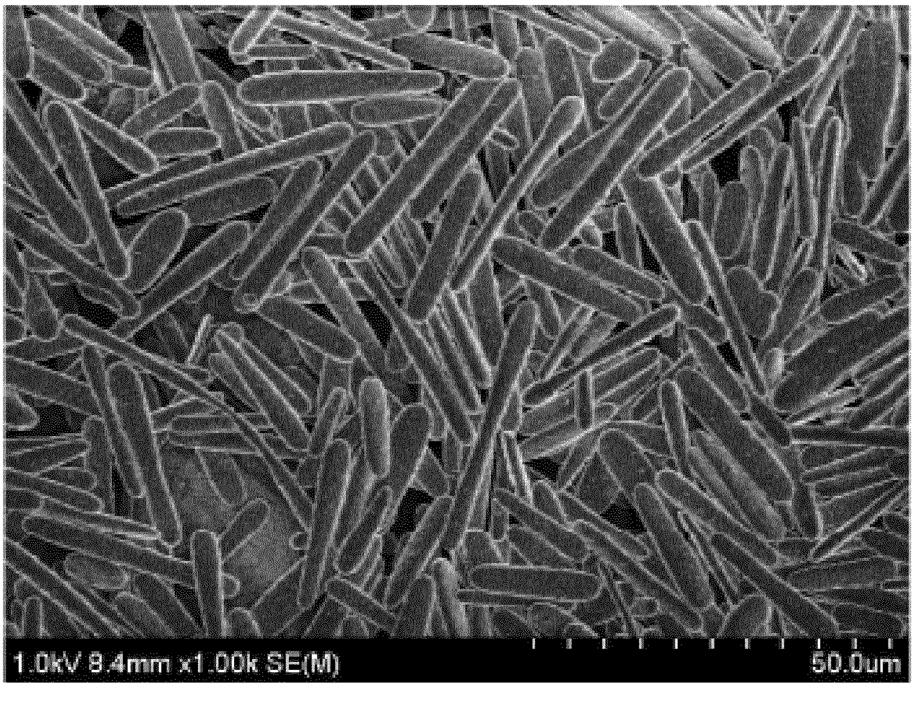

FIG.5

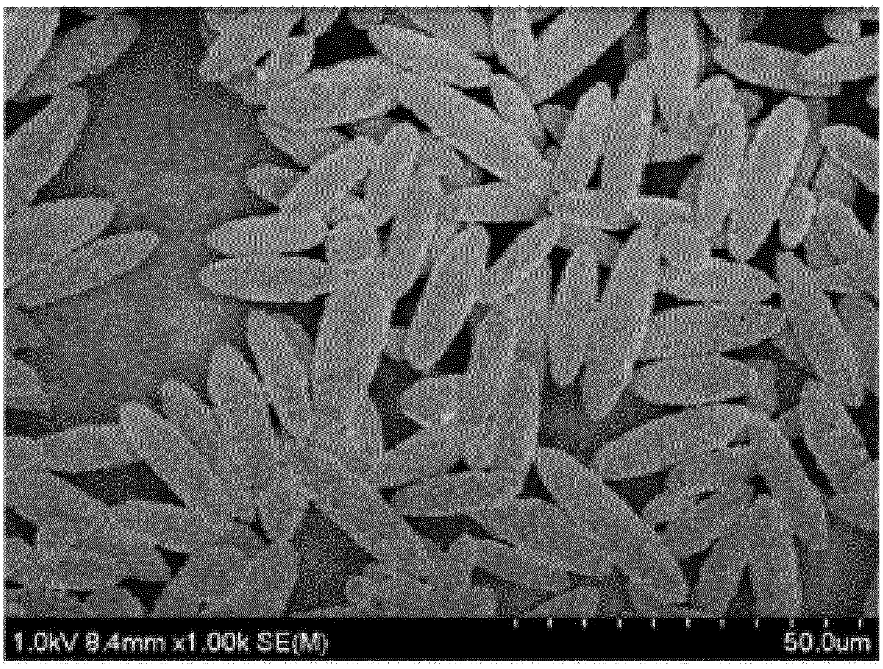
1.0kV 8.4mm x1.00k SE(M)
50.0um

FIG.6

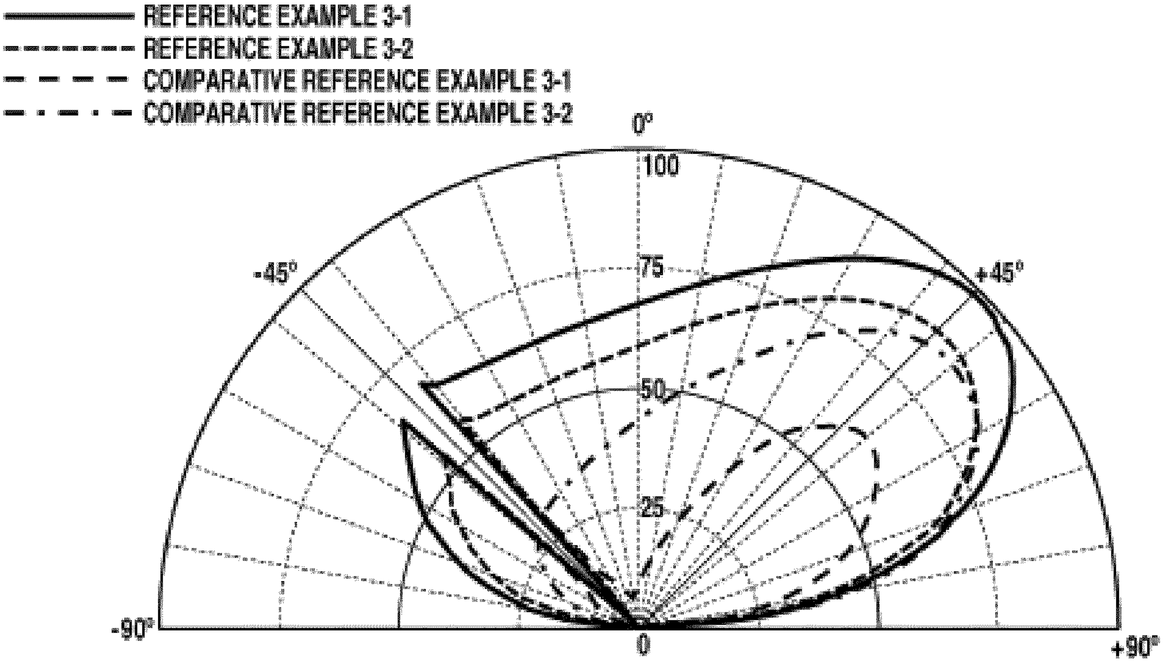
REFERENCE EXAMPLE 3-1
REFERENCE EXAMPLE 3-2
COMPARATIVE REFERENCE EXAMPLE 3-1
COMPARATIVE REFERENCE EXAMPLE 3-2
0°
100
-45°
+45°
75
50
25
-90°
0
+90°

**INTERNATIONAL SEARCH REPORT**

International application No.
PCT/JP2017/039523

A. CLASSIFICATION OF SUBJECT MATTER

Int.Cl. A61K8/81(2006.01)i, A61K8/02(2006.01)i, A61K8/19(2006.01)i, A61Q1/02(2006.01)i, A61Q1/10(2006.01)i, A61Q1/12(2006.01)i, A61Q17/04(2006.01)i, A61Q19/00(2006.01)i

According to International Patent Classification (IPC) or to both national classification and IPC

B. FIELDS SEARCHED

Minimum documentation searched (classification system followed by classification symbols)

Int.Cl. A61K8/81, A61K8/02, A61K8/19, A61Q1/02, A61Q1/10, A61Q1/12, A61Q17/04, A61Q19/00, C08C19/00-19/44, C08F6/00-246/00, C08K3/00-13/08, C08L1/00-101/14

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)

JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| Y | JP 2015-93973 A (NISSHINBO HOLDINGS INC.) 18 May 2015, claims, paragraphs [0008], [0011], [0049], [0051], tables 8-10 & US 2016/0262988 A1, claims, paragraphs [0012], [0019], [0058], [0060], tables 8-10 & WO 2015/072443 A1 & EP 3070138 A1 | 1-10 |
| Y | JP 2012-1440 A (OHKEN CO., LTD.) 05 January 2012, claims, paragraphs [0009]-[0010] (Family: none) | 1-10 |
| A | JP 2016-17048 A (NISSHINBO HOLDINGS INC.) 01 February 2016, claims (Family: none) | 1-10 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>21 December 2017 (21.12.2017) | Date of mailing of the international search report<br>09 January 2018 (09.01.2018) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009235353 A
- JP 2009235355 A